# EUROPÄISCHE PATENTSCHRIFT

(11) **EP 0 759 907 B1**
(45) Veröffentlichungstag und Bekanntmachung des Hinweises auf die Patenterteilung: **10.10.2001**
(21) Anmeldenummer: 95920017.1
(22) Anmeldetag: 10.05.1995
(51) Int. Cl.: C07D 231/20, A01N 43/50, C07D 401/12, C07D 403/12, C07D 405/10, C07D 405/12, C07D 409/10, C07D 413/12

(54) **SUBSTITUIERTE 3-PHENYLPYRAZOLE ALS HERBIZIDE**
SUBSTITUTED 3-PHENYLPYRAZOLES FOR USE AS HERBICIDES
3-PHENYLPYRAZOLS SUBSTITUES UTILISES COMME HERBICIDES

(30) Priorität: 20.05.1994 DE 4417837
(43) Veröffentlichungstag der Anmeldung: 05.03.1997
(60) Teilanmeldung: 01107592.6
(73) Patentinhaber: BASF AKTIENGESELLSCHAFT, 67056 Ludwigshafen (DE)
(72) Erfinder: VON DEM BUSSCHE-HÜNNEFELD, Christoph-Sweder, D-68199 Mannheim (DE); KLINTZ, Ralf, D-67269 Gruenstadt (DE); HAMPRECHT, Gerhard, D-69469 Weinheim (DE); HEISTRACHER, Elisabeth, D-67061 Ludwigshafen (DE); SCHÄFER, Peter, D-67308 Ottersheim (DE); MÜNSTER, Peter, D-67354 Römerberg (DE); DITRICH, Klaus, D-67161 Gönnheim (DE); KÖNIG, Hartmann, D-69115 Heidelberg (DE); WESTPHALEN, Karl-Otto, D-67346 Speyer (DE); GERBER, Matthias, D-67117 Limburgerhof (DE); WALTER, Helmut, D-67283 Obrigheim (DE)
(86) Internationale Anmeldenummer: EP9501772
(87) Internationale Veröffentlichungsnummer: WO9532188

(56) Entgegenhaltungen:
- WO-A-92/02509
- WO-A-93/25535
- FR-A- 2 262 663
- GB-A- 2 073 172
- CHEMICAL ABSTRACTS, vol. 122, no. 7, 13. Februar 1995, Columbus, Ohio, US; abstract no. 81124f, G.R. BROWN ET AL. 'Quinuclidine derivatives useful as squalene synthase inhibitors and their preparation.' Seite 1066 ;Spalte 2 ; & CA,A,2 104 981 (ZENECA LTD.) 1. März 1994
- CHEMICAL ABSTRACTS, vol. 115, no. 5, 5. August 1991, Columbus, Ohio, US; abstract no. 49684h, J. MIURA ET AL. 'Preparation of 3-phenylpyrazole derivatives as herbicides.' Seite 849 ;Spalte 1 ; in der Anmeldung erwähnt & JP,A,03 072 460 (NIHON NOHYAKU CO., LTD.) 27. März 1991
- CHEMICAL ABSTRACTS, vol. 121, no. 25, 19. Dezember 1994, Columbus, Ohio, US; abstract no. 300886r, T. KODAIRA ET AL. 'Preparation of difluoromethoxypyrazoles as herbicides.' Seite 1014 ;Spalte 2 ; & JP,A,06 199 806 (NIHON NOHAYAKU CO., LTD.) 19. Juli 1994

## Beschreibung

Die vorliegende Erfindung betrifft neue substituierte 3-Phenylpyrazole der Formel I 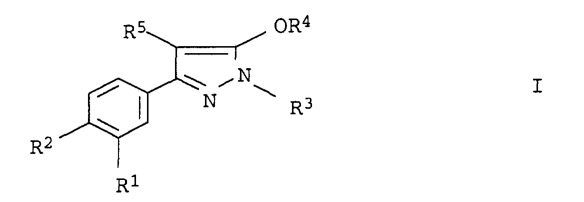 in der die Variablen folgende Bedeutung haben:
- R¹: Wasserstoff, Nitro, C₁-C₈-Alkyl, C₁-C₈-Halogenalkyl, C₁-C₈-Alkyl-O-R⁶, C₁-C₈-Alkyl-O-CO-R⁶, C₁-C₈-Alkyl-S-R⁶, C₁-C₈-Alkyl-SO-R⁶, C₁-C₈-Alkyl-SO₂-R⁶, -SO₂-R⁶, -SO₂-O-R⁶, -SO₂-N(R⁷,R⁸), -N(R⁷,R⁸), -N(R⁷)-N(R⁸,R³²), -N=N-CO-R⁹, -N(R⁷)-N(R⁸)-CO-R⁹, -N(R¹⁰)-CO-R⁹, -N(R¹⁰)-SO₂-R¹¹, -N(SO₂-R¹¹)(SO₂-R¹²), -A-CO-O-R⁶, -A-CO-N(R⁷,R⁸), -A-CO-R²⁰, -A-CH (XR²¹, YR²²), -A-CO-N(R⁷)-C (R⁸,R¹⁸)-COOR⁶, -SO₂-N(R⁷)-C(R⁸,R¹⁸)-COOR⁶, -SO₂-N(R⁷)-C(R⁸,R¹⁸)-CO-N(R³²,R³³), 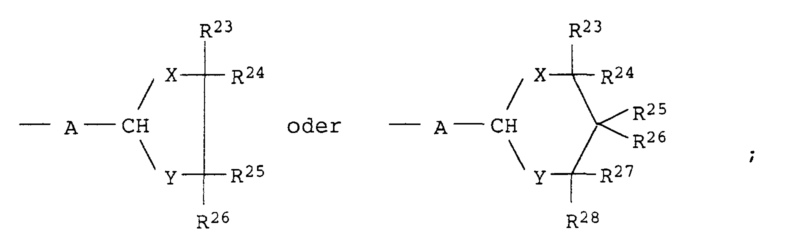
- R²: Cyano, Trifluormethyl oder Halogen;
- R³: Wasserstoff, C₁-C₄-Alkyl oder C₁-C₄-Halogenalkyl;
- R⁴: C₁-C₄-Alkyl oder C₁-C₄-Halogenalkyl;
- R⁵: Halogen;
- X,Y: unabhängig voneinander Sauerstoff oder Schwefel;
- A: eine chemische Bindung, Methylen, Ethylen, 1,3-Propylen, 1,4-Butylen, Vinylen oder 1,4-Butadienylen;
- R⁶: Wasserstoff, C₁-C₈-Alkyl, C₁-C₈-Halogenalkyl, C₃-C₇-Cycloalkyl, das seinerseits ein bis drei C₁-C₃-Alkylreste tragen kann, C₃-C₆-Alkenyl, C₅-C₇-Cycloalkenyl, das seinerseits ein bis drei C₁-C₃-Alkylreste tragen kann, C₃-C₆-Halogenalkenyl, Cyano-C₁-C₈-alkyl, C₃-C₆-Alkinyl, C₁-C₄-Alkoxy-C₁-C₄-alkyl, 2-Tetrahydrofuranyl-C₁-C₈-alkyl, 3-Oxetanyl, 3-Thiethanyl, Carboxyl-C₁-C₆-alkyl, (C₁-C₈-Alkoxy)carbonyl-C₁-C₆-alkyl, C₁-C₄-Alkoxy-(C₁-C₄-alkoxy)carbonyl-C₁-C₆-alkyl, Cyclopropylmethyl, (1-Methylthiocycloprop-1-yl)methyl, C₃-C₉-(α-Alkylalkyliden)iminooxy-C₁-C₆-alkyl, (C₁-C₄-Alkyl)carbonyl, C₁-C₄-Alkyl, das durch -C(R¹⁹)=N-O-(C₁-C₄-Alkyl), -C(R¹⁹)=N-O-(C₁-C₄-Halogenalkyl), -C(R¹⁹)=N-O-(C₃-C₆-Alkenyl), -C(R¹⁹)=N-O-(C₃-C₆-Halogenalkenyl) oder -C(R¹⁹)=N-O-(C₁-C₄-Alkyl)-R³⁴ substituiert ist, Phenyl, Phenyl-C₁-C₆-alkyl, Phenyl-C₂-C₆-alkenyl, Phenyl-C_{*3*}-C₆-alkinyl oder Phenoxy-C₁-C₆-alkyl, wobei der Phenylring jeweils unsubstituiert sein oder seinerseits ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl, Heteroaryl-C₁-C₆-alkyl, Heteroaryl-C₃-C₆-alkenyl, Heteroaryl-C₃-C₆-alkinyl oder Heteroaryloxy-C₁-C₆-alkyl, wobei der Heteroaromat jeweils ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ringglied einen Rest tragen kann, jeweils ausgewählt aus der Gruppe bestehend aus Hydroxyl, Halogen, C₁-C₄-Halogenalkyl, C₁-C₄-Alkoxy, C₁-C₄-Alkylthio und C₁-C₄-Alkyl;
- R⁷, R⁸, R³², R³³: unabhängig voneinander
Wasserstoff, C₁-C₈-Alkyl, C₁-C₈-Halogenalkyl, C₂-C₈-Alkenyl, C₂-C₈-Alkinyl, C₁-C₄-Alkoxy-C₁-C₄-alkyl, C₁-C₄-Alkylthio-C₁-C₄-alkyl, Cyano-C₁-C₈-alkyl, Carboxyl-C₁-C₄-alkyl, (C₁-C₄-Alkoxy)carbonyl-C₁-C₄-alkyl, C₁-C₄-Alkylsulfonyl-C₁-C₄-alkyl, C₃-C₈-Cycloalkyl, C₁-C₆-Alkoxy, (C₃-C₆-Cycloalkoxy)carbonyl-C₁-C₄-alkyl, C₁-C₄-Alkoxy-(C₁-C₄-alkoxy)carbonyl-C₁-C₄-alkyl, (C₁-C₄-Alkyl)carbonyl, (C₁-C₄-Halogenalkyl)carbonyl, Tetrahydrofuran-2-on-3-yl, Phenyl, Phenyl-C₁-C₄-alkyl, wobei der Phenylring jeweils unsubstituiert sein oder ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl oder Heteroaryl-C₁-C₄-alkyl, wobei der Heteroaromat ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
oder
- R⁷ und R⁸: zusammen eine Tetramethylen-, Pentamethylen- oder Ethylenoxyethylenkette, die gewünschtenfalls ein bis drei C₁-C₄-Alkylreste und/ oder einen Rest -COOR⁶ tragen kann;
- R⁹: Wasserstoff, C₁-C₆-Alkyl, C₁-C₆-Halogenalkyl, C₁-C₄-Alkoxy-C₁-C₄-alkyl, C₃-C₇-Cycloalkyl, das seinerseits ein bis drei Reste tragen kann, ausgewählt aus der Gruppe bestehend aus Halogen, C₁-C₄-Alkyl, C₁-C₄-Alkoxy und C₁-C₄-Alkylthio, Phenyl oder Phenyl-C₁-C₆-alkyl, wobei der Phenylring jeweils unsubstituiert sein oder ein bis drei Reste tragen kann, ausgewählt aus der Gruppe bestehend aus Halogen, Nitro, C₁-C₄-Alkyl, C₁-C₄-Alkoxy, C₁-C₄-Alkylthio und C₁-C₄-Halogenalkyl;
- R¹⁰: Wasserstoff, C₁-C₉-Alkyl, C₃-C₆-Alkenyl, C₃-C₆-Alkinyl, C₁-C₄-Alkoxy-C₁-C₄-alkyl oder das Äquivalent eines landwirtschaftlich brauchbaren Kations;
- R¹¹, R¹²: unabhängig voneinander
C₁-C₄-Alkyl, C₁-C₄-Halogenalkyl, Phenyl, das unsubstituiert sein oder ein bis drei Substituenten tragen kann, jeweils ausgewählt aus der Gruppe bestehend aus Halogen, Nitro, C₁-C₄-Alkyl, C₁-C₄-Alkoxy, C₁-C₄-Alkylthio und C₁-C₄-Halogenalkyl, oder 5- oder 6-gliedriges Heteroaryl mit ein bis drei Heteroatomen, ausgewählt aus der Gruppe bestehend aus 2 Stickstoffatomen und einem Sauerstoff- oder Schwefelatom, wobei der Heteroaromat unsubstituiert sein oder an jedem substituierbaren Ringglied gewünschtenfalls einen Substituenten tragen kann, jeweils ausgewählt aus der Gruppe bestehend aus Hydroxy, Halogen, C₁-C₄-Alkyl, C₁-C₄-Halogenalkyl, C₁-C₄-Alkoxy und C₁-C₄-Alkylthio;
- R¹⁸: Wasserstoff oder C₁-C₄-Alkyl;
- R¹⁹: Wasserstoff, C₁-C₄-Alkyl, Phenyl oder Benzyl;
- R²⁰: Wasserstoff, Cyano, Halogen, C₁-C₄-Alkyl, C₁-C₄-Alkylthio, C₂-C₄-Alkenyl, C₁-C₄-Halogenalkyl, C₁-C₄-Alkoxy-C₁-C₄-alkyl, Di-(C₁-C₄-alkoxy)-C₁-C₄-alkyl, C₁-C₄-Alkylthio-C₁-C₄-alkyl, (1,3-Dioxolan-2-yl)-C₁-C₄-alkyl oder (1,3-Dioxan-2-yl)-C₁-C₄-alkyl;
- R²¹, R²²: unabhängig voneinander C₁-C₈-Alkyl, C₁-C₈-Halogenalkyl oder C₁-C₄-Alkoxy-C₁-C₄-alkyl;
- R²³, R²⁴, R²⁵, R²⁶, R²⁷, R²⁸: unabhängig voneinander Wasserstoff, Cyano, C₁-C₈-Alkyl, C₁-C₈-Halogenalkyl, C₁-C₄-Alkoxy-C₁-C₄-alkyl, C₁-C₈-Alkoxy, C₁-C₄-Alkoxy-C₁-C₄-alkoxy, -CO-O-R⁶, -CO-N(R⁷, R⁸), -CO-R²⁰, -S-R⁶, -SO₂-R⁶, -O-CO-R⁹ oder C₃-C₇-Cycloalkyl, das seinerseits ein bis drei Reste tragen kann, ausgewählt aus der Gruppe bestehend aus Halogen, C₁-C₄-Alkyl, C₁-C₄-Alkoxy und C₁-C₄-Alkylthio;
- R³⁴: Phenyl oder 5- oder 6-gliedriges Heteroaryl mit ein bis drei Heteroatomen, ausgewählt aus der Gruppe bestehend aus 2 Stickstoffatomen und einem Sauerstoff- oder Schwefelatom, wobei jeder Phenyl- oder Heteroarylring unsubstituiert sein oder an jedem substituierbaren Ringglied gewünschtenfalls einen Substituenten tragen kann, jeweils ausgewählt aus der Gruppe bestehend aus Hydroxy, Nitro, Cyano, Halogen, C₁-C₄-Alkyl, C₁-C₄-Halogenalkyl, C₁-C₄-Alkoxy und C₁-C₄-Alkylthio,
sowie die landwirtschaftlich brauchbaren Salze von I.

Außerdem betrifft die Erfindung die Verwendung dieser Verbindungen als Herbizide, herbizide Mittel, welche die Verbindungen I als wirksame Substanzen enthalten, Verfahren zur Herstellung dieser herbiziden Mittel sowie Verfahren zur Bekämpfung von unerwünschtem Pflanzenwuchs mit den Verbindungen I.

In der JP 03/151 367 werden herbizid wirksame 1-(1-Alkyl-4-halogen-5-halogenalkoxy-1H-pyrazol-3-yl)-4,6-dihalogenphenyl-Derivate mit verschiedenen Substituenten in 3-Position des Phenylrings beschrieben, insbesondere Verbindungen mit dem folgenden Substitutionsmuster IIa:

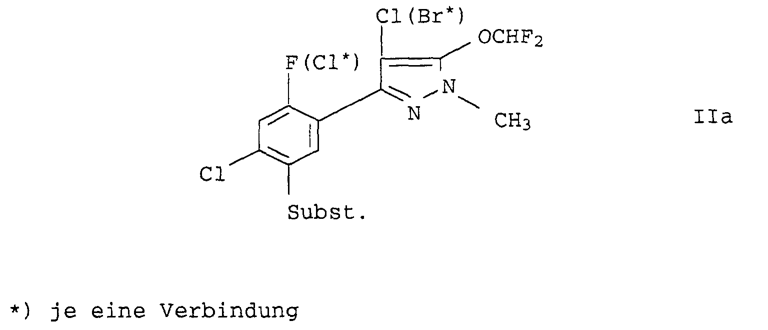

Außerdem wird in der EP-A 443 059 gelehrt, daß sich 1-Alkyl- und 1-Halogenalkyl-3-(4-chlor-6-halogenphenyl)-pyrazole bzw. -4-halogenpyrazole, die in 3-Position des Phenylrings bestimmte Substituenten tragen und die in 5-Position des Pyrazolrings durch Hydroxyl, Mercapto, niederes Alkoxy, Alkylthio, Halogenalkoxy oder Halogenalkylthio substituiert sind, zur Bekämpfung unerwünschter Pflanzen eignen.

Ferner ist der JP-A 03/072 460 zu entnehmen, daß 3-substituierte Phenylpyrazole der Formel IIb 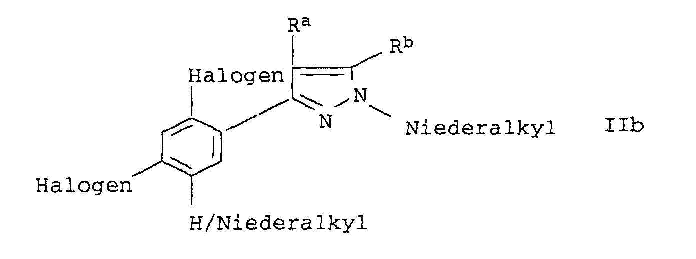 in der R^{a} für Wasserstoff, Halogen oder Cyano und R^{b} für niederes Alkoxy, Halogenalkoxy, Alkylthio, Halogenalkylthio, Alkylsulfinyl, Halognalkylsulfinyl, Alkylsulfonyl oder Halogenalkylsulfonyl stehen, herbizid wirksam sind.

Aus der EP-A 447 055 ist bekannt, daß 1-(Niederalkyl)-3-(4-Chlor-6-halogenphenyl)-4-Halogen-5-difluormethoxypyrazole, die in 3-Position des Phenylrings eine Alkylthiocarbonyl-, Alkenylthiocarbonyl- oder Benzylthiocarbonyl-methoxycarbonylgruppe tragen, herbizide Wirkung zeigen.

Gemäß den Lehren der JP-A 03/047 180 und der JP-A 03/081 275 sind u.a. Pyrazol-Derivate der Formeln IIc und IId 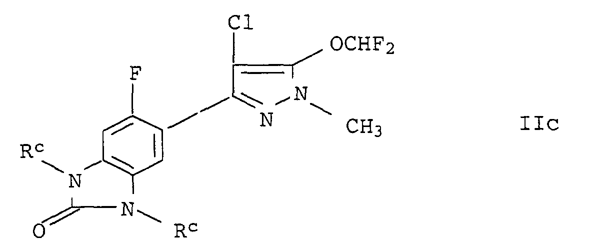 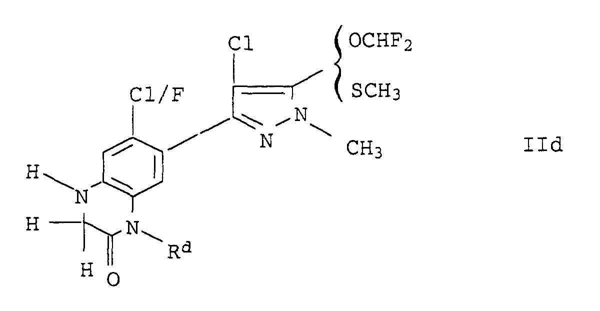 wobei
- R^{c}: Wasserstoff, Methyl oder Allyl und
- R^{d}: Wasserstoff, Ethyl, Allyl oder Propargyl bedeuten,
als Herbizide geeignet.

Gemäß der JP-A 02/300 173 und der JP-A 03/093 774 weisen bestimmte 1-Alkyl-3-phenylpyrazole, die am Phenylring ein bis vier Halogenatome tragen können, ebenfalls herbizide Aktivität auf. Besonders genannt sind 1-Methyl-3-(2,4-dichlorphenyl)-pyrazole und drei 1-Methyl-5-chlor-3-(2-fluor-4-chlorphenyl)-pyrazole.

Ferner werden in der WO 92/06962 herbizide 4-Halogen-5-halogenalkyl-3-phenylpyrazole mit verschiedenen Substituenten am Phenylring beschrieben.

Weiterhin lehrt WO 93/25535 unter anderem 1-Alkyl-5-halogenalkoxy-3-phenylpyrazole, wobei der in 3-Position gebundene Phenylrest substituiert sein kann und in 4-Position des Pyrazols eine Carboxamid bzw. Thiocarboxamidgruppe gebunden ist (Formel IIe).

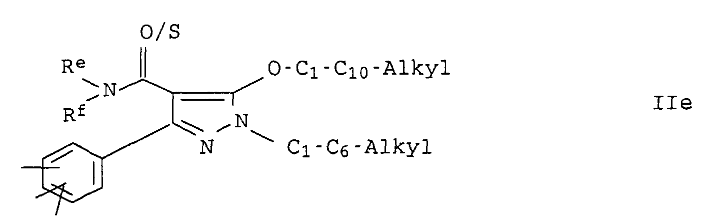

Diese Verbindungen werden als Herbizide verwendet.

Schließlich sind aus FR-A-22 62 663 3-Phenylpyrazole der Formel IIf bekannt, wobei der Phenylring gegebenenfalls substituiert ist und der Pyrazolrest in 1-Position durch Alkyl, in 4-Position durch Chlor oder Brom und in 5-Position durch einen Schwefelrest substituiert ist. Auch diese Verbindungen werden als Herbizide verwendet.

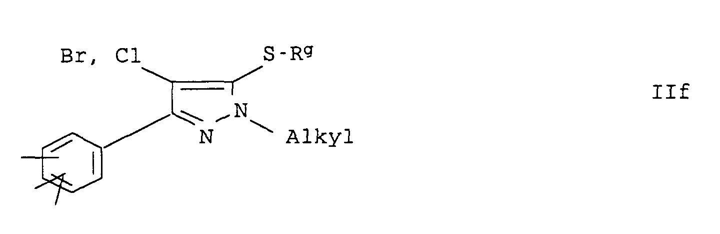

Die herbiziden Eigenschaften der bekannten Herbizide bezüglich der Schadpflanzen vermögen jedoch nur bedingt zu befriedigen.

Aufgabe der vorliegenden Erfindung war es deshalb, neue herbizid wirksame Verbindungen bereitzustellen, mit denen sich unerwünschte Pflanzen besser als bisher gezielt bekämpfen lassen.

Demgemäß wurden die vorliegenden substituierten 3-Phenylpyrazole der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Im Hinblick auf die Verwendung der substituierten 3-Phenylpyrazole I als Herbizide sind Verbindungen I bevorzugt, in denen die Variablen folgende Bedeutungen haben, und zwar jeweils für sich allein oder in Kombination:
- R¹: Wasserstoff, Nitro, C₁-C₈-Alkyl, C₁-C₈-Halogenalkyl, C₁-C₈-Alkyl-O-R⁶, C₁-C₈-Alkyl-O-CO-R⁶, C₁-C₈-Alkyl-S-R⁶, C₁-C₈-Alkyl-SO-R⁶, C₁-C₈-Alkyl-SO₂-R⁶, -SO₂-R⁶, -SO₂-O-R⁶, -SO₂-N(R⁷,R⁸), -N(R⁷,R⁸), -N(R⁷)-N(R⁸,R³²), -N=N-CO-R⁹, -N(R⁷)-N(R⁸)-CO-R⁹, -N(R¹⁰)-CO-R⁹, -N(R¹⁰)-SO₂-R¹¹, -N(SO₂-R¹¹)(SO₂-R¹²), -A-CO-O-R⁶, -A-CO-N(R⁷,R⁸), -A-CO-R²⁰, -A-CH(XR²¹, YR²²), -A-CO-N(R⁷)-C(R⁸,R¹⁸)-COOR⁶, -SO₂-N(R⁷)-C(R⁸,R¹⁸)-COOR⁶, -SO₂-N(R⁷) -C(R⁸,R¹⁸) -CO-N(R³²,R³³), 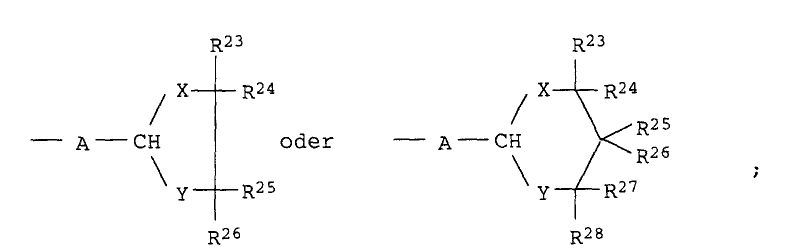
- R²: Cyano, Trifluormethyl oder Halogen;
- R³: C₁-C₄-Alkyl oder C₁-C₄-Halogenalkyl;
- R⁴: C₁-C₄-Alkyl oder C₁-C₄-Halogenalkyl;
- R⁵: Halogen;
- X,Y: Sauerstoff oder Schwefel;
- A: eine chemische Bindung, Methylen, Ethylen oder Vinylen;
- R⁶: Wasserstoff, C₁-C₈-Alkyl, C₁-C₈-Halogenalkyl, C₃-C₆-Cycloalkyl, das seinerseits ein oder zwei C₁-C₃-Alkylreste tragen kann, C₃-C₆-Alkenyl, C₃-C₆-Halogenalkenyl, Cyano-C₁-C₄-alkyl, C₃-C₆-Alkinyl, C₁-C₂-Alkoxy-C₁-C₂-alkyl, Carboxyl-C₁-C₆-alkyl, (C₁-C₄-Alkoxy)carbonyl-C₁-C₄-alkyl, Cyclopropylmethyl, C₁-C₄-Alkyl, das durch -C(R¹⁹)=N-O-(C₁-C₄-Alkyl), -C(R¹⁹)=N-O-(C₁-C₄-Halogenalkyl), -C(R¹⁹)=N-O-(C₃-C₆-Alkenyl), -C(R¹⁹)=N-O-(C₃-C₆-Halogenalkenyl) oder -C(R¹⁹)=N-O-(C₁-C₄-Alkyl)-phenyl substituiert ist, Phenyl, Phenyl-C₁-C₄-alkyl, Phenyl-C₂-C₄-alkenyl oder Phenoxy-C₁-C₄-alkyl, wobei der Phenylring jeweils unsubstituiert sein oder seinerseits ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl oder Heteroaryl-C₁-C₆-alkyl, wobei der Heteroaromat jeweils ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxyl, Halogen, C₁-C₄-Halogenalkyl, C₁-C₄-Alkoxy, C₁-C₄-Alkylthio und C₁-C₄-Alkyl;
- R⁷, R⁸, R³², R³³: unabhängig voneinander Wasserstoff, C₁-C₄-Alkyl, C₁-C₄-Halogenalkyl, C₂-C₄-Alkenyl, C₂-C₄-Alkinyl, C₁-C₄-Alkoxy-C₁-C₄-alkyl, Cyano-C₁-C₄-alkyl, Carboxyl-C₁-C₄-alkyl, (C₁-C₄-Alkoxy)carbonyl-C₁-C₄-alkyl, C₃-C₆-Cycloalkyl, C₁-C₄-Alkoxy, (C₃-C₆-Cycloalkoxy)carbonyl-C₁-C₄-alkyl, Tetrahydrofuran-2-on-3-yl, Phenyl, Phenyl-C₁-C₄-alkyl, wobei der Phenylring jeweils unsubstituiert sein oder ein bis drei Reste tragen kann, ausgewählt aus der Gruppe bestehend aus Halogen, Nitro, Cyano, C₁-C₄-Alkyl, C₁-C₄-Alkoxy und C₁-C₄-Halogenalkyl, 5- oder 6-gliedriges Heteroaryl oder Heteroaryl-C₁-C₄-alkyl, wobei der Heteroaromat ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxyl, Halogen, C₁-C₄-Alkyl, C₁-C₄-Alkoxy und C₁-C₄-Halogenalkyl;
oder
- R⁷ und R⁸: zusammen eine Tetramethylen-, Pentamethylen- oder Ethylenoxyethylenkette, die gewünschtenfalls ein bis drei C₁-C₄-Alkylreste und/oder einen Rest -COOR⁶ tragen kann;
- R⁹: Wasserstoff, C₁-C₄-Alkyl, C₁-C₄-Halogenalkyl, C₁-C₄-Alkoxy-C₁-C₄-alkyl, C₃-C₇-Cycloalkyl, das seinerseits ein bis drei Reste tragen kann, ausgewählt aus der Gruppe bestehend aus Halogen, C₁-C₄-Alkyl und C₁-C₄-Alkoxy,
Phenyl oder Phenyl-C₁-C₆-alkyl, wobei der Phenylring jeweils unsubstituiert sein oder ein bis drei Reste tragen kann, ausgewählt aus der Gruppe bestehend aus Halogen, Nitro, C₁-C₄-Alkyl, C₁-C₄-Alkoxy und C₁-C₄-Halogenalkyl;
- R¹⁰: Wasserstoff, C₁-C₄-Alkyl, C₃-C₆-Alkenyl, C₃-C₆-Alkinyl, C₁-C₄-Alkoxy-C₁-C₄-alkyl oder das Äquivalent eines landwirtschaftlich brauchbaren Kations;
- R¹¹, R¹²: unabhängig voneinander
C₁-C₄-Alkyl, C₁-C₄-Halogenalkyl, Phenyl oder Thienyl, wobei der Phenyl- und Thienylring unsubstituiert sein oder ein bis drei Reste tragen kann, ausgewählt aus der Gruppe bestehend aus Halogen, Nitro, C₁-C₄-Alkyl, C₁-C₄-Alkoxy, C₁-C₄-Alkylthio und C₁-C₄-Halogenalkyl,
oder 5- oder 6-gliedriges Heteroaryl mit ein bis drei Heteroatomen, ausgewählt aus der Gruppe bestehend aus 2 Stickstoffatomen und einem Sauerstoffatom, wobei der Heteroaromat unsubstituiert sein oder an jedem substituierbaren Ringglied gewünschtenfalls einen Substituenten tragen kann, jeweils ausgewählt aus der Gruppe bestehend aus Hydroxyl, Halogen, C₁-C₄-Alkyl und C₁-C₄-Alkoxy;
- R¹⁸: Wasserstoff oder C₁-C₄-Alkyl;
- R¹⁹: Wasserstoff oder C₁-C₄-Alkyl;
- R²⁰: Wasserstoff, C₁-C₄-Alkyl, C₂-C₄-Alkenyl, C₁-C₄-Halogenalkyl, C₁-C₄-Alkoxy-C₁-C₄-alkyl oder Di-(C₁-C₄-alkoxy)-C₁-C₄-alkyl;
- R²¹, R²²: unabhängig voneinander C₁-C₄-Alkyl, C₁-C₄-Halogenalkyl oder C₁-C₄-Alkoxy-C₁-C₄-alkyl;
- R²³, R²⁴, R²⁵, R²⁶, R²⁷, R²⁸: unabhängig voneinander Wasserstoff, Cyano, C₁-C₄-Alkyl, C₁-C₄-Alkoxy oder -CO-O-R²⁰.

Die für die Substituenten R¹ bis R³⁴ oder als Reste an (Hetero)aromaten genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Alkylcarbonyl-, Halogenalkylcarbonyl-, Alkenyl-, Alkinyl-, Halogenalkyl-, Halogenalkenyl-, Halogenalkinyl-, Cyanoalkyl-, Phenylalkyl, Carboxylalkyl-, Alkoxy-, Alkylthio-, Alkylcarbonyl-, Alkoxycarbonyl- und Alkylsulfonyl-Teile sowie der α-Alkylalkyliden-Teil können geradkettig oder verzweigt sein. Halogenierte Substituenten tragen vorzugsweise ein bis fünf gleiche oder verschiedene Halogenatome.

Im einzelnen stehen beispielsweise:
- Halogen für: Fluor, Chlor, Brom und Jod, vorzugsweise für Fluor und Chlor;
- C₁-C₆-Alkyl und die C₁-C₆-Alkyl-Teile von Carboxyl-C₁-C₆-alkyl, (C₁-C₈-Alkoxy)carbonyl-C₁-C₆-alkyl, C₁-C₄-Alkoxy-(C₁-C₄-alkoxy)carbonyl-C₁-C₆-alkyl, (C₁-C₈-Alkoxy)carbonyl-C₁-C₆-alkyl, C₃-C₉-(α-Alkylalkyliden)iminooxy-C₁-C₆-alkyl, Phenyl-C₁-C₆-alkyl, Phenoxy-C₁-C₆-alkyl, Heteroaryl-C₁-C₆-alkyl und Heteroaryloxy-C₁-C₆-alkyl für: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise für C₁-C₄-Alkyl, insbesondere für Methyl und Ethyl;
- C₁-C₈-Alkyl und der Alkylteil von Cyano-C₁-C₈-alkyl für: C₁-C₆-Alkyl wie vorstehend genannt, sowie z.B. für n-Heptyl, und n-Octyl, vorzugsweise für C₁-C₆-Alkyl, insbesondere für Methyl und Ethyl;
- C₁-C₄-Alkyl und die Alkylteile von C₁-C₆-Alkoxy-C₁-C₄-alkyl, C₁-C₄-Alkoxy-C₁-C₄-alkyl, Di-(C₁-C₄-alkoxy)-C₁-C₄-alkyl, C₁-C₄-Alkylthio-C₁-C₄-alkyl, (C₁-C₆-Alkoxy)carbonyl-C₁-C₄-alkyl, Di-[(C₁-C₆-Alkoxy)carbonyl]-C₁-C₄-alkyl, (C₁-C₄-Alkoxy)carbonyl-C₁-C₄-alkyl, (C₃-C₆-Cycloalkoxy)carbonyl-C₁-C₄-alkyl, C₁-C₄-Alkoxy-(C₁-C₄-alkoxy)carbonyl-C₁-C₄-alkyl, C₁-C₄-Alkylsulfonyl-C₁-C₄-alkyl, (1,3-Dioxolan-2-yl)-C₁-C₄-alkyl, (1,3-Dioxan-2-yl)-C₁-C₄-alkyl und Heteroaryl-C₁-C₄-alkyl für: Methyl, Ethyl, n-Propyl, 1-Methylethyl, n-Butyl, 1-Methylpropyl, 2-Methylpropyl und 1,1-Dimethylethyl, vorzugsweise für Methyl und Ethyl;
- C₁-C₃-Alkylreste für: Methyl, Ethyl, n-Propyl und 1-Methylethyl, vorzugsweise für Methyl;
- C₂-C₈-Alkenyl für: C₂-C₆-Alkenyl wie Ethenyl, Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methylprop-1-en-1-yl, 1-Methyl-prop-2-en-1-yl, 2-Methylprop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethylprop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl, sowie z.B. für n-Hept-2-en-1-yl, Hept-3-en-1-yl, n-Oct-2-en-1-yl und Oct-3-en-1-yl, vorzugsweise für C₂-C₆-Alkenyl;
- C₃-C₆-Alkenyl und der Alkenyl-Teil von Heteroaryl-C₃-C₆-alkenyl für: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl, vorzugsweise für C₃- oder C₄-Alkenyl;
- C₂-C₈-Alkinyl z.B. für: Ethinyl, Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl, und 4-Methyl-pent-2-in-5-yl, vorzugsweise für C₂-C₆-Alkinyl, insbesondere für Ethinyl und Prop-2-in-3-yl;
- C₃-C₆-Alkinyl und der Alkinyl-Teil von Heteroaryl-C₃-C₆-alkinyl für: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise für C₃- oder C₄-Alkinyl, insbesondere für Ethinyl und Prop-2-in-3-yl;
- C₁-C₈-Halogenalkyl für: C₁-C₈-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl und Heptafluorpropyl, vorzugsweise für C₁-C₆-Halogenalkyl;
- C₁-C₆-Halogenalkyl für: C₁-C₆-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl und Heptafluorpropyl, vorzugsweise für C₁-C₄-Halogenalkyl, insbesondere für Trifluormethyl und 1,2-Dichlorethyl;
- C₁-C₄-Halogenalkyl für: C₁-C₄-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl und Heptafluorpropyl, vorzugsweise für Trifluormethyl und 1,2-Dichlorethyl;
- C₂-C₈-Halogenalkenyl für: C₂-C₈-Alkenyl wie vorstehend genannt, das partiell oder vollständig durch Fluor, Chlor und/ oder Brom substituiert ist, also z.B. für 2-Chlorallyl, 3-Chlorallyl und 3,3-Dichlorallyl, vorzugsweise für C₂-C₆-Halogenalkenyl;
- C₃-C₆-Halogenalkenyl für: C₃-C₆-Alkenyl wie vorstehend genannt das partiell oder vollständig durch Fluor, Chlor und/oder Brom substituiert ist, also z.B. für 2-Chlorallyl, 3-Chlorallyl und 3,3-Dichlorallyl;
- C₂-C₈-Halogenalkinyl für: C₂-C₈-Alkinyl wie vorstehend genannt, das partiell oder vollständig durch Fluor, Chlor und/oder Brom substituiert ist, vorzugsweise für C₂-C₆-Halogenalkenyl;
- Cyano-C₁-C₄-alkyl für: Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyanomethylprop-2-yl, vorzugsweise für 2-Cyanoeth-1-yl;
- Cyano-C₁-C₈-alkyl für: Cyano-C₁-C₄-alkyl wie verstehend genannt, vorzugsweise für 2-Cyanoeth-1-yl;
- Phenyl-C₁-C₄-alkyl für: Benzyl, 1-Phenyleth-1-yl, 2-Phenyleth-1-yl, 1-Phenylprop-1-yl, 2-Phenylprop-1-yl, 3-Phenylprop-1-yl, 1-Phenylprop-2-yl, 2-Phenylprop-2-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 1-Phenylbut-3-yl, 2-Phenylbut-3-yl, 1-Phenyl-2-methyl-prop-3-yl, 2-Phenyl-2-methyl-prop-3-yl, 3-Phenyl-2-methyl-prop-3-yl und 2-Benzyl-prop-2-yl;
- Carboxyl-C₁-C₄-alkyl für: Carboxylmethyl, 1-Carboxylethyl, 2-Carboxylethyl, 1-Carboxylprop-1-yl, 2-Carboxylprop-1-yl, 3-Carboxylprop-1-yl, 1-Carboxylbut-1-yl, 2-Carboxylbut-1-yl, 3-Carboxylbut-1-yl, 4-Carboxylbut-1-yl, 1-Carboxylbut-2-yl, 2-Carboxylbut-2-yl, 3-Carboxylbut-2-yl, 3-Carboxylbut-2-yl, 4-Carboxylbut-2-yl, 1-(Carboxylmethyl)-eth-1-yl, 1-(Carboxylmethyl)-1-(methyl)-eth-1-yl und 1-(Carboxylmethyl)-prop-1-yl, vorzugsweise für Carboxylmethyl und 2-Carboxylethyl;
- Carboxyl-C₁-C₆-alkyl für: Carboxyl-C₁-C₄-alkyl wie vorstehend genannt, sowie für 5-Carboxylpent-1-yl, vorzugsweise für Carboxyl-C₁-C₄-alkyl;
- C₁-C₄-Alkoxy und die Alkoxy-Teile von C₁-C₄-Alkoxy-C₁-C₄-alkyl, Di-(C₁-C₄-alkoxy)-C₁-C₄-alkyl, (C₁-C₄-Alkoxy)carbonyl-C₁-C₄-alkyl, C₁-C₄-Alkoxy-(C₁-C₄-alkoxy)carbonyl-C₁-C₆-alkyl, C₁-C₄-Alkoxy-(C₁-C₄-alkoxy)carbonyl-C₁-C₄-alkyl und C₁-C₄-Alkoxy-C₁-C₄-alkoxy für: Methoxy, Ethoxy, n-Propoxy, 1-Methylethoxy, n-Butoxy, 1-Methyl-propoxy, 2-Methylpropoxy und 1,1-Dimethylethoxy, vorzugsweise für Methoxy, Ethoxy und 1-Methylethoxy;
- C₁-C₆-Alkoxy und der Alkoxy-Teil von C₁-C₆-Alkoxy-C₁-C₄-alkyl für: C₁-C₄-Alkoxy wie vorstehend genannt, sowie für n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, vorzugsweise für Methoxy, Ethoxy und 1-Methylethoxy;
- C₁-C₈-Alkoxy für: C₁-C₆-Alkoxy wie vorstehend genannt, sowie z.B. für n-Heptoxy und n-Octoxy, vorzugsweise für C₁-C₆-Alkoxy, insbesondere für Methoxy, Ethoxy und 1-Methylethoxy;
- C₁-C₄-Alkylthio und der Alkylthio-Teil von C₁-C₄-Alkylthio-C₁-C₄-alkyl für: Methylthio, Ethylthio, n-Propylthio, 1-Methylethylthio, n-Butylthio, 1-Methylpropylthio, 2-Methylpropylthio und 1,1-Dimethylethylthio, vorzugsweise für Methylthio, Ethylthio und 1-Methylethylthio;
- C₁-C₆-Alkylthio für: C₁-C₄-Alkylthio wie vorstehend genannt, sowie für n-Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, n-Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise für Methylthio, Ethylthio und 1-Methylethylthio;
- (C₁-C₄-Alkyl)carbonyl für: Methylcarbonyl, Ethylcarbonyl, n-Propylcarbonyl, 1-Methylethyl-carbonyl, n-Butylcarbonyl, 1-Methylpropylcarbonyl, 2-Methylpropylcarbonyl und 1,1-Dimethylethylcarbonyl, vorzugsweise für Methylcarbonyl, Ethylcarbonyl und n-Propylcarbonyl;
- (C₁-C₄-Halogenalkyl)carbonyl für: C₁-C₄-Alkyl wie vorstehend genannt, das partiell oder vollständig durch Fluor, Chlor und/oder Brom substituiert ist, also z.B. Chlormethylcarbonyl, Dichlormethylcarbonyl, Trichlormethylcarbonyl, Fluormethylcarbonyl, Difluormethylcarbonyl, Trifluormethylcarbonyl, Chlorfluormethylcarbonyl, Dichlorfluormethylcarbonyl, Chlordifluormethylcarbonyl, 1-Fluorethylcarbonyl, 2-Fluorethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 3-Chlorpropylcarbonyl und Heptafluorpropylcarbonyl, vorzugsweise für Trifluormethylcarbonyl und 1,2-Dichlorethylcarbonyl;
- (C₁-C₆-Alkoxy)carbonyl und die Alkoxycarbonyl-Teile von (C₁-C₆-Alkoxy)carbonyl-C₁-C₄-alkyl und Di-[(C₁-C₆-Alkoxy)carbonyl]-C₁-C₄-alkyl für: Methoxycarbonyl, Ethoxycarbonyl, n-Propoxycarbonyl, 1-Methyl-ethoxycarbonyl, n-Butoxycarbonyl, 1-Methylpropoxycarbonyl, 2-Methylpropoxycarbonyl, 1,1-Dimethylethoxycarbonyl, n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl und 1-Ethyl-2-methyl-propoxycarbonyl, vorzugsweise für (C₁-C₄-Alkoxy)carbonyl, insbesondere für Methoxycarbonyl, Ethoxycarbonyl und 1-Methylethoxycarbonyl;
- der Alkoxycarbonyl-Teil von (C₁-C₈-Alkoxy)carbonyl-C₁-C₆-alkyl für: (C₁-C₆-Alkoxy)carbonyl wie vorstehend genannt, sowie z.B. für n-Heptoxycarbonyl und n-Octoxycarbonyl, vorzugsweise für (C₁-C₆-Alkoxy)carbonyl, insbesondere für Methoxycarbonyl, Ethoxycarbonyl und 1-Methylethoxycarbonyl;
- der Alkylsulfonylteil von C₁-C₄-Alkylsulfonyl-C₁-C₄-alkyl für: Methylsulfonyl, Ethylsulfonyl, n-Propylsulfonyl, 1-Methylethylsulfonyl, n-Butylsulfonyl, 1-Methyl-propylsulfonyl, 2-Methylpropylsulfonyl und 1,1-Dimethylethylsulfonyl, vorzugsweise für Methylsulfonyl und Ethylsulfonyl;
- der C₃-C₉-(α-Alkylalkyliden)-Teil von C₃-C₉-(α-Alkylalkyliden)iminooxy-C₁-C₆-alkyl z.B. für: α-Methylethyliden, α-Methylpropyliden und α-Ethylpropyliden, insbesondere für α-Methylethyliden;
- C₃-C₆-Cycloalkyl für: Cyclopropyl, Cyclobutyl, Cyclopentyl und Cyclohexyl, vorzugsweise für Cyclopropyl und Cyclopentyl;
- C₃-C₇-Cycloalkyl für: C₃-C₆-Cycloalkyl wie vorstehend genannt, sowie für Cycloheptyl, vorzugsweise für Cyclopropyl und Cyclopentyl;
- C₃-C₈-Cycloalkyl für: C₃-C₆-Cycloalkyl wie vorstehend genannt, sowie für Cycloheptyl und Cyclooctyl, vorzugsweise für Cyclopropyl, Cyclopentyl und Cyclohexyl;
- C₅-C₇-Cycloalkenyl für: Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, vorzugsweise für Cyclopent-1-enyl;
- der Cycloalkoxycarbonyl-Teil von (C₃-C₆-Cycloalkoxy)carbonyl-C₁-C₄-alkyl für: Cyclopropoxycarbonyl, Cyclobutoxycarbonyl, Cyclopentoxycarbonyl und Cyclohexoxycarbonyl, vorzugsweise für Cyclopropoxycarbonyl und Cyclopentoxycarbonyl;
- 5- oder 6-gliedriges Heteroaryl und der Heteroaryl-Teil von Heteroaryl-C₁-C₄-alkyl für: z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, vorzugsweise für 3-Pyrazolyl, 2-Pyridinyl und 2-Thienyl.

Unter landwirtschaftlich brauchbaren Kationen kommen vor allem diejenigen Kationen in Betracht, die die herbizide Wirkung der Verbindungen I nicht negativ beeinträchtigen, insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammonium-Ion, das gewünschtenfalls ein bis drei C₁-C₄-Alkyl-, Hydroxy-C₁-C₄-alkylsubstituenten und/oder einen Phenyl- oder Benzylsubstituenten tragen kann, vorzugsweise Diisopropylammonium, Tetramethylammonium, Tetrabutylammonium, Trimethylbenzylammonium und Trimethyl-(2-hydroxyethyl)-ammonium, des weiteren Phosphonium-Ionen, Sulfonium-Ionen, vorzugsweise Tri-(C₁-C₄-alkyl)sulfonium, und Sulfoxonium-Ionen, vorzugsweise Tri-(C₁-C₄-alkyl)sulfoxonium.

Die 3-Phenylpyrazol-Derivate der Formel I sind auf verschiedene Weise erhältlich, vorzugsweise nach einem der folgenden Verfahren:

### A) Umsetzung eines β-Ketocarbonsäurederivats III mit Hydrazin oder einem Hydrazin-Derivat in einem inerten Lösungsmittel (vgl. z.B. JP-A 04/225 937 und JP-A 03/072 460) und Alkylierung des Verfahrensproduktes IV:

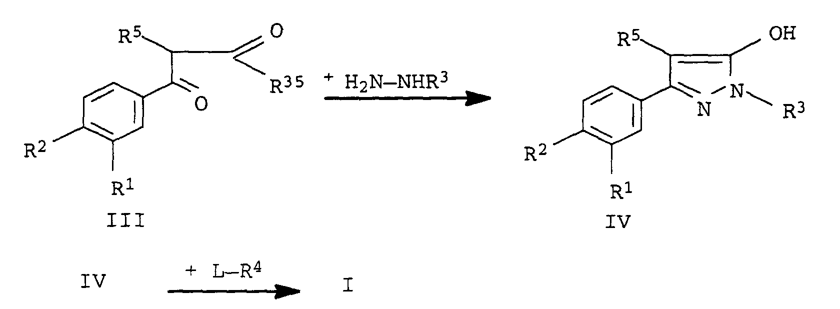

- L: steht für eine geeignete Abgangsgruppe wie Halogen, -O-SO₂CH₃, -O-SO₂CF₃, -O-SO₂C₄F₉ oder -O-SO₂(p-CH₃-C₆H₄);
- R³⁵: steht vorzugsweise für C₁-C₄-Alkoxy, C₁-C₄-Alkylcarbonyloxy oder Halogen.

Das Lösungsmittel kann aprotisch oder protisch sein. Geeignet sind z.B. organische Säuren wie Essigsäure, Kohlenwasserstoffe, halogenierte Kohlenwasserstoffe, Ether wie Ethylenglykoldimethylether, Alkohole wie Methanol und Ethanol, sowie Sulfoxide.

Die Reaktionstemperatur wird hauptsächlich vom Schmelzpunkt der Verbindung III und dem Siedepunkt des Reaktionsgemisches vorgegeben. Bevorzugt arbeitet man bei etwa 60 bis 120 °C.

Im allgemeinen setzt man etwa die 0,95- bis 5-fache molare Menge, zweckmäßigerweise die 1- bis 1,4-fache Menge, an Hydrazin oder Hydrazin-Derivat, bezogen auf den β-Ketocarbonsäurederivats III, ein.

Die Menge an Alkylierungsmittel L-R⁴ liegt üblicherweise ebenfalls bei der 0,95- bis zur 5-fachen molaren Menge, bezogen auf das Zwischenprodukt IV.

Mit Blick auf die bevorzugten Reste R³ an den 3-Phenylpyrazolen I sind unter den Hydrazin-Derivaten diejenigen besonders bevorzugt, die eine Alkylgruppe tragen.

Die Alkylierung erfolgt normalerweise mit dem Halogenid, vorzugsweise dem Chlorid oder Bromid, oder mit dem Sulfat eines Alkans oder Halogenalkans, gewünschtenfalls in Gegenwart einer organischen Base, z.B. einem Trialkylamin oder Pyridin, oder einer anorganischen Base, z.B. einem Alkalimetallcarbonat.

Zweckmäßigerweise wir die Alkylierung in einem inerten organischen Lösungsmittel vorgenommen, z.B. in einem aliphatischen oder cyclischen Ether wie 1,2-Dimethoxyethan, Tetrahydrofuran und Dioxan, in einem aliphatischen Keton wie Aceton, in einem Amid wie Dimethylformamid, in einem Sulfoxid wie Dimethylsulfoxid oder in einer Mischung aus einem dieser Lösungsmittel und Wasser.

Die Reaktion ist im allgemeinen bei einer Temperatur von 0 °C bis zur Siedetemperatur des Reaktionsgemisches ausführbar. Vorzugsweise arbeitet man bei etwa 20 bis 80 °C.

### B) Halogenierung von Verbindungen I mit R⁵ = Wasserstoff:

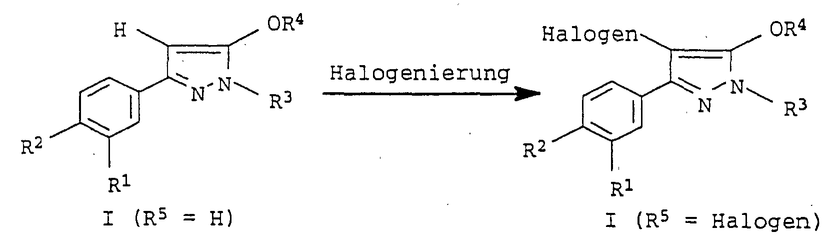

Die Umsetzung kann in einem inerten Lösungs- oder Verdünnungsmittel oder lösungsmittelfrei vorgenommen werden.

Beispiele für geeignete Lösungsmittel sind organische Säuren, anorganische Säuren, Kohlenwasserstoffe, halogenierte Kohlenwasserstoffe, aromatische Kohlenwasserstoffe, Ether, Sulfide, Sulfoxide und Sulfone.

Als Halogenierungsmittel kommen beispielsweise Chlor, Brom, N-Bromsuccinimide, N-Chlorsuccinimide oder Sulfurylchlorid in Betracht. Je nach Ausgangsverbindung und Halogenierungsmittel kann der Zusatz eines Radikalstarters, beispielsweise eines organischen Peroxides wie Dibenzoylperoxid oder einer Azoverbindung wie Azobisisobutyronitril, oder Bestrahlung mit Licht vorteilhaft auf den Reaktionsverlauf wirken.

Die Menge an Halogenierungsmittel ist nicht kritisch. Sowohl unterstöchiometrische Mengen als auch große Überschüsse an Halogenierungsmittel, bezogen auf die zu halogenierende Verbindung I mit R⁵ = Wasserstoff, sind möglich.

Bei Verwendung eines Radikalstarters ist in der Regel eine katalytische Menge ausreichend.

Die Reaktionstemperatur liegt normalerweise bei (- 100) bis 200° C, vornehmlich bei 10 bis 100° C oder dem Siedepunkt des Reaktionsgemisches.

### C) Nitrierung von Verbindungen I mit R¹ = Wasserstoff:

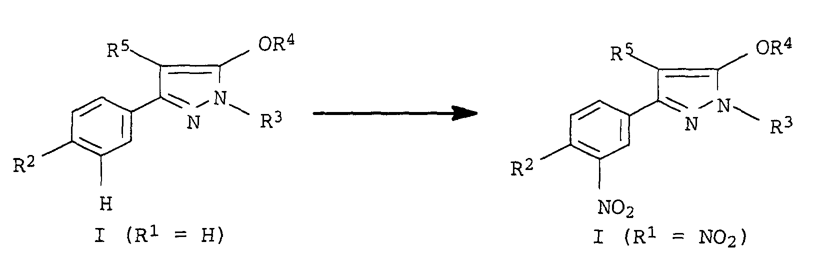

Als Nitrierungs-Reagenzien kommen beispielsweise Salpetersäure in unterschiedlicher Konzentration, auch konzentrierte und rauchende Salpetersäure, Mischungen von Schwefelsäure und Salpetersäure, Acetylnitrate und Alkylnitrate in Betracht.

Die Reaktion kann entweder lösungsmittelfrei in einem Überschuß des Nitrierungs-Reagenzes oder in einem inerten Lösungs- oder Verdünnungsmittel durchgeführt werden, wobei z.B. Wasser, Mineralsäuren, organische Säuren, Chlorkohlenwasserstoffe wie Methylenchlorid, Anhydride wie Essigsäureanhydrid und Mischungen dieser Solventien geeignet sind.

Die Ausgangsverbindung I mit R¹ = H und das Nitrierungs-Reagenz werden zweckmäßigerweise in etwa äquimolaren Mengen eingesetzt; zur Optimierung des Umsatzes an zu nitrierender Verbindung kann es jedoch vorteilhaft sein, das Nitrierungs-Reagenz im Überschuß zu verwenden, bis etwa zur 10-fachen molaren Menge. Bei der Reaktionsführung ohne Lösungsmittel im Nitrierungs-Reagenz liegt dieses in einem noch größeren Überschuß vor.

Die Reaktionstemperatur liegt normalerweise bei (- 100) bis 200 °C, bevorzugt bei (- 30) bis 50 °C.

Die Aufarbeitung des Reaktionsgemisches kann auf bekannte Weise erfolgen, beispielsweise durch Verdünnen der Reaktionslösung mit Wasser und anschließender Isolierung des Produktes mittels Filtration, Kristallisation oder Lösungsmittelextraktion.

### D) Reduktion von Verbindungen I mit R¹ = Nitro:

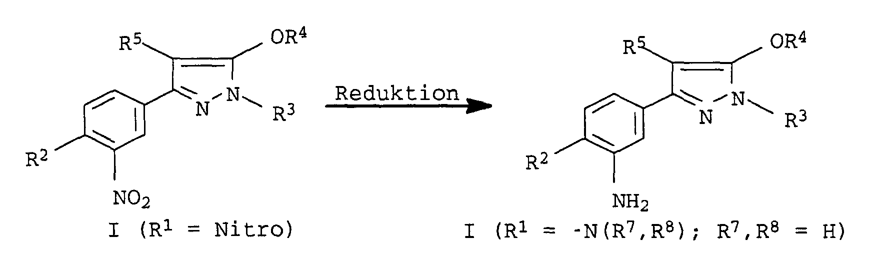

### D1) Reduktion mit einem Metall wie Eisen, Zink oder Zinn unter sauren Reaktionsbedingungen oder mittels komplexen Hydriden wie Lithiumaluminiumhydrid und Natriumborhydrid:

Das Lösungsmittel, z.B. Wasser, ein Alkohol wie Methanol, Ethanol und Isopropanol oder ein Ether wie Diethylether, Methyl-tert.-butylether, Dioxan, Tetrahydrofuran und Ethylenglykoldimethylether, ist abhängig vom gewählten Reduktionsmittel.

Bei der Reduktion mit einem Metall arbeitet man vorzugsweise lösungsmittelfrei in einer anorganische Säure, insbesondere konzentrierter oder verdünnter Salzsäure, oder einer organischen Säure wie Essigsäure. Es ist aber auch möglich, der Säure ein inertes Lösungsmittel wie vorstehend genannt zuzumischen.

Die Ausgangsverbindung I (R¹ = NO₂) und das Reduktionsmittel werden zweckmäßigerweise in etwa äquimolaren Mengen eingesetzt; zur Optimierung des Reaktionsverlaufes kann es jedoch vorteilhaft sein, eine der beiden Komponenten im Überschuß zu verwenden, bis etwa zur 10-fachen molaren Menge.

Die Menge an Säure ist nicht kritisch. Um die Ausgangsverbindung möglichst vollständig zu reduzieren verwendet man zweckmäßigerweise mindestens eine äquivalente Menge an Säure.

Die Reaktionstemperatur liegt im allgemeinen bei (-30) bis 200 °C, bevorzugt bei 0 bis 80 °C.

Nach Beendigung der Reaktion wird die Reaktionsmischung üblicherweise mit Wasser verdünnt und das Produkt durch Filtration, Kristallisation oder Extraktion mit einem Lösungsmittel, das mit Wasser weitgehend unmischbar ist, z.B. mit Essigsäureethylester, Diethylether oder Methylenchlorid, isoliert. Gewünschtenfalls kann das Produkt anschließend auf bekannte Weise gereinigt werden.

### D2) Katalytische Hydrierung mit Wasserstoff:

Geeignete Katalysatoren sind beispielsweise Raney-Nickel, Palladium auf Kohle, Palladiumoxid, Platin und Platinoxid, wobei im allgemeinen eine Katalysatormenge von 0,05 bis 10,0 mol-%, bezogen auf die zu reduzierende Verbindung, ausreichend ist.

Man arbeitet entweder lösungsmittelfrei oder in einem inerten Lösungs- oder Verdünnungsmittel, z.B. in Essigsäure, einem Gemisch aus Essigsäure und Wasser, Essigsäureethylester, Ethanol oder in Toluol.

Nach Abtrennen des Katalysators kann die Reaktionslösung wie üblich auf das Produkt hin aufgearbeitet werden.

Die Hydrierung kann bei Normaldruck oder unter erhöhtem Druck durchgeführt werden.

### E) Verbindungen I, bei denen R¹ für -N(R⁷,R⁸) und R⁷ und R⁸ für Wasserstoff stehen, können auf an sich bekannte Weise z.B. alkyliert, acyliert, sulfonyliert, hydroxyliert oder diazotiert werden (siehe hierzu z.B. Houben-Weyl, Methoden der Organischen Chemie, Band E16d, Teil 2, Georg Thieme Verlag, Stuttgart, 4. Auflage 1992, Seiten 1241 bis 1314 sowie die dort zitierte Literatur):

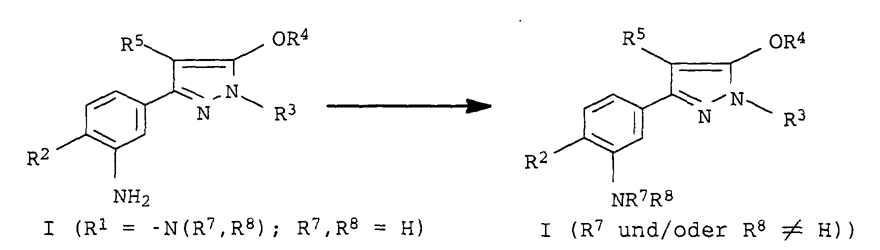

Ebenso sind weitere Umsetzung möglich, z.B. Sandmeyerreaktionen {vgl. z.B. H.H. Hodgson, Chem. Rev. 40, 251 (1947) und Houben-Weyl, Methoden der Organischen Chemie, Bd. 5/4, Georg Thieme Verlag, Stuttgart, 4. Auflage 1960, S. 438}, Meerweinreaktionen {siehe z.B. M. Doyle, B. Siegfried. R.C. Elliot, J.F. Dellaria, J. Org. Chem. 42, 2431 (1977)} oder die Reduktion des Diazoniumsalzes von I (R¹ = NH₂) zum entsprechenden Arylhydrazin (siehe z.B. Houben-Weyl, Methoden der Organischen Chemie, Bd. E16a, Georg Thieme Verlag, Stuttgart, 4. Auflage 1990, S. 656ff.). Die Arylhydrazine können ihrerseits in üblicher Weise acyliert werden (vgl. hierzu z.B. Houben-Weyl, Methoden der Organischen Chemie, Bd. E16a, Georg Thieme Verlag, Stuttgart, 4. Auflage 1990, S. 828ff.).

Die so erhaltenen Acylhydrazine sind wiederum zu Azoverbindungen oxidierbar (siehe z.B. Houben-Weyl, Methoden der Organischen Chemie, Bd. E16d, Georg Thieme Verlag, Stuttgart, 4. Auflage 1992, S. 102f.).

### F) Halogenierung von Verbindungen I, bei denen R¹ für Methyl steht:

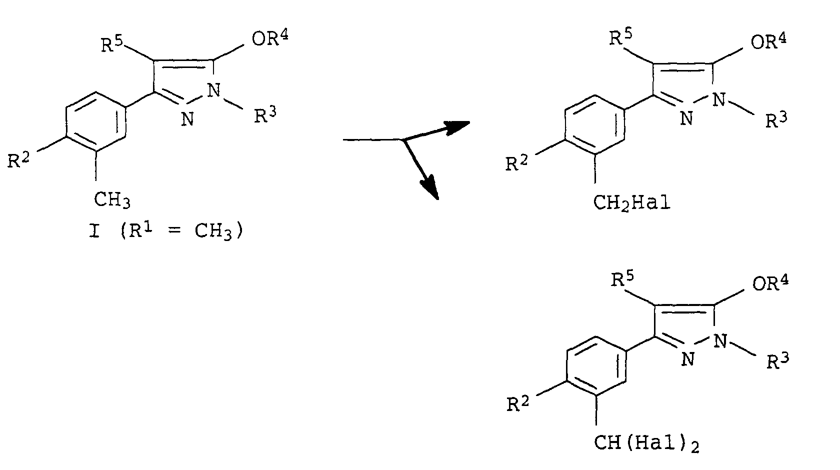

Hal = Halogen, vorzugsweise Chlor oder Brom.

Bezüglich der Lösungsmittel, Mengenverhältnisse und der Reaktionstemperatur sei auf die Angaben unter Methode B) verwiesen.

### G) Nukleophile Substitution eines Halogenatoms an Verbindungen I mit R¹ = CH₂Hal:

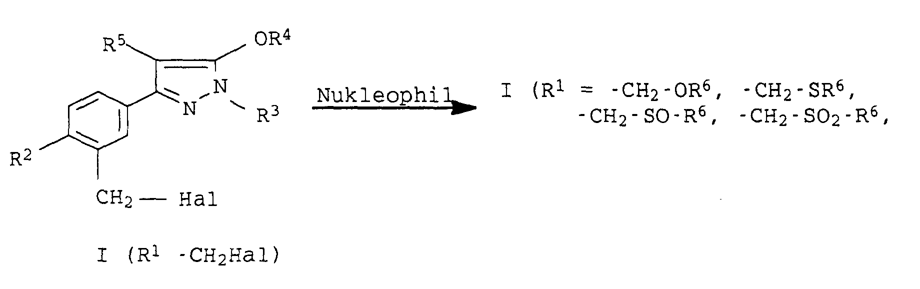

Als Nukleophile kommen beispielsweise Alkoxide, Thioalkoxide, Aryl- oder Alkyl-Anionen, Cyanide, Alkohole, Thiolen oder Salze von Carbonsäuren in Betracht.

Je nach Ausgangsverbindung und Nucleophil kann der Zusatz einer Base vorteilhaft sein, wobei beispielsweise organische Basen, z.B. Trialkylamine oder Diazabicycloundecen oder anorganische Basen wie Kalium- oder Natriumcarbonat oder die Alkalimetallhydroxide geeignet sind.

Die Menge an Base beträgt vorzugsweise 0,95 bis 10 mol, insbesondere etwa 1 bis 3 mol, pro mol Ausgangsverbindung.

Bevorzugte Lösungsmittel sind insbesondere Dimethylformamid, Dimethylacetamid, Aceton, Dimethylsulfoxid, Dioxan, Wasser und eine Mischung dieser Solventien.

Es besteht ferner die Möglichkeit, in einem 2-Phasen-System aus Wasser und einem mit Wassser weitgehend unmischbaren organischen Lösungsmittel, z.B. Methylenchlorid, zu arbeiten. Zur Verbesserung des Reaktionsverlaufes ist bei dieser Variante der Zusatz eines Phasen-Transfer-Katalysators, z. B. eines Ammonium-Salzes wie Benzyltrialkylammonium- und Tetrabutylammonium-Halogenid, zweckmäßig (bezüglich der Phasentransferkatalyse siehe auch Synthesis 1976, 113).

Im allgemeinen ist eine katalytische Menge an Phasentransferkatalysator, etwa zwischen 1 und 10 Mol%, bezogen auf die Ausgangsverbindung, ausreichend.

Die Reaktionstemperatur ist abhängig von der Wahl des Nukleophils. Bei der Verwendung von Aryl- oder Alkyl-Anionen liegt sie bei etwa (- 150) bis 0°C, bevorzugt bei (- 78) bis (- 20) °C. Für die übrigen o.g. Nukleophile ist normalerweise eine höhere Reaktionstemperatur notwendig, etwa von 0 bis 100 °C.

Diejenigen Endprodukte I, bei denen R¹ -CH₂-OR⁶ bedeutet, mit R⁶ = (C₁-C₄-Alkyl)carbonyl, können anschließend auf an sich bekannte Weise zu Verbindungen I mit R¹ = -CH₂OH gespalten werden. Die Spaltprodukte können dann gewünschtenfalls alkyliert, acyliert oder sulfoniert werden, wobei weitere definitionsgemäße Verbindungen I mit R¹ = -CH₂OR⁶ (R⁶ ≠ H) erhalten werden (siehe z.B. Houben-Weyl, Methoden der Organischen Chemie, Georg Thieme Verlag, Stuttgart: Bd. 6/1a, 4. Aufl. 1979, S. 262ff.; Bd. 8, 4. Auflage 1952; S. 516ff.; Bd. 6/3, 4. Aufl. 1965, S. 10ff.; Bd. 9, S. 343ff., 4. Aufl. 1955, S. 343ff. und 659ff.).

### H) Saure Hydrolyse von Verbindungen I, bei denen R¹ Dihalogenmethyl bedeutet:

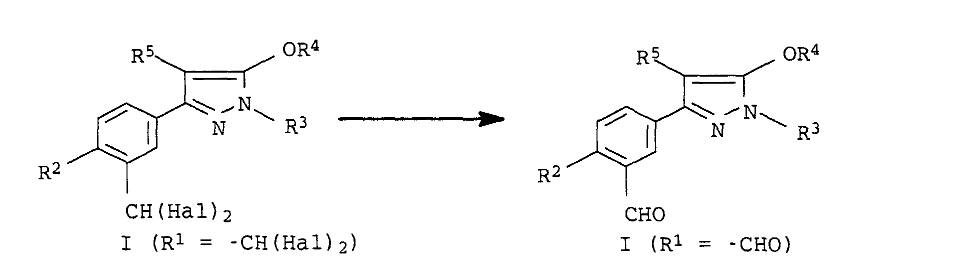

Die Hydrolyse erfolgt vorzugsweise lösungsmittelfrei in einer Säure wie Salzsäure, Schwefelsäure und Essigsäure, insbesondere konzentrierte Schwefelsäure, oder in einer Mischung aus Essigsäure und Wasser (z.B. 3:1).

Die Reaktionstemperatur liegt normalerweise bei 0 bis 120°C.

Die Aufarbeitung des Reaktionsproduktes kann in der Regel auf an sich bekannte Weise erfolgen.

### I) Oxidation von Verbindungen I, bei denen R¹ Halogenmethyl bedeutet, auf an sich bekannte Weise, z.B. nach Kornblum (siehe hierzu insbesondere die Seiten 179 bis 181 des Bandes "Methods for the Oxidation of Organic Compounds" von Alan H. Haines, Academic Press 1988, in der Serie "Best Synthetic Methods"):

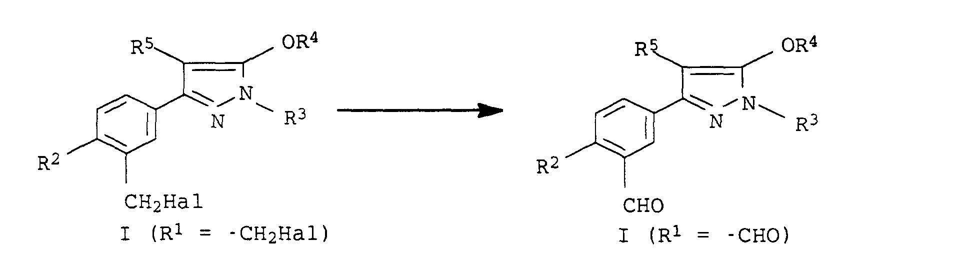

Als Lösungsmittel ist z.B. Dimethylsulfoxid geeignet.

### K) Oxidation von Verbindungen I, bei denen R¹ für Formyl steht:

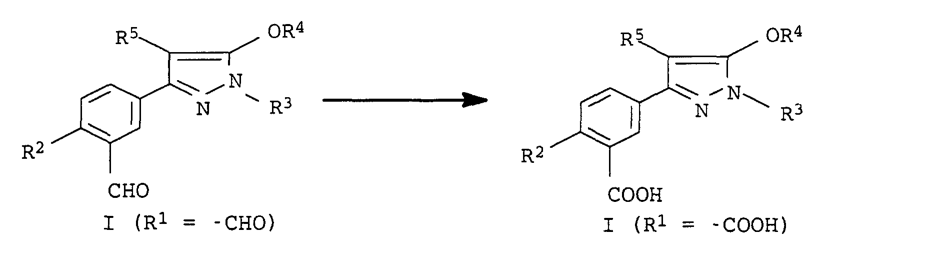

Als inerte Lösungsmittel kommen z.B. Wasser, Kohlenwasserstoffe, aromatische Kohlenwasserstoffe oder Pyridin und seine Derivate in Betracht.

Geeignete Oxidationsmittel sind beispielsweise Kaliumpermanganat, Kaliumdichromat, Natriumperborat, Natriumchlorit, Wasserstoffperoxid und Sauerstoff.

Die Reaktionstemperatur ist vor allem abhängig vom Reaktionsmedium. Sie liegt vorzugsweise bei 0 bis 120° C.

Die Aufarbeitung auf das Produkt hin erfolgt wie üblich.

Die weitere Umsetzung des Verfahrensproduktes zu den entsprechenden Säure- bzw. Ketonderivaten kann auf an sich bekannte Weise erfolgen (siehe hierzu insbesondere die Teile 1 und 2 des Bandes E5 von Houben-Weyl, Methoden der Organischen Chemie).

### L) Halosulfonierung von Verbindungen I, bei denen R¹ für -SO₂-Cl steht:

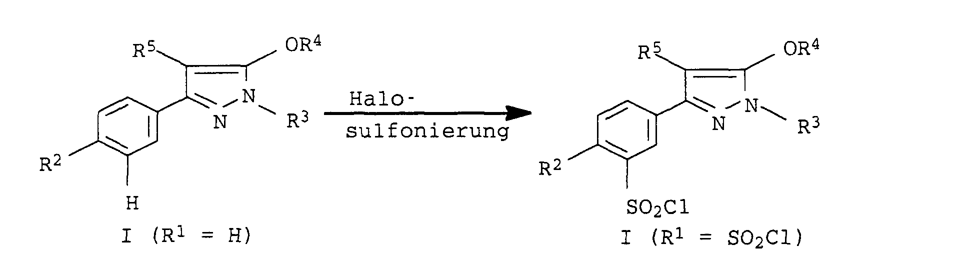

Die Halosulfonierung kann ohne Lösungsmittel in einem Überschuß an Sulfonierungsreagenz oder in einem inerten Lösungsmittel, z.B. in einem halogenierten Kohlenwasserstoff, einem Ether, einem Alkylnitril oder einer Mineralsäure durchgeführt werden.

Chlorsulfonsäure stellt sowohl das bevorzugte Reagenz wie auch Lösungsmittel dar.

Das Sulfonierungsreagenz wird normalerweise in einem leichten Unterschuß (bis etwa 95 mol-%) oder in einem Überschuß von der 1- bis 5-fachen molaren Menge, bezogen auch die Ausgangsverbindung I (R¹ = H) eingesetzt. Arbeitet man ohne inertes Lösungsmittel, so kann auch ein noch größerer Überschuß zweckmäßig sein.

Die Reaktionstemperatur liegt normalerweise zwischen 0° C und dem Siedepunkt des Reaktionsgemisches.

Zur Aufarbeitung wird die Reaktionsmischung z.B. mit Wasser versetzt, wonach sich das Produkt wie üblich isolieren läßt.

### M) Umsetzung von Verbindungen I, bei denen R¹ -SO₂-Cl bedeutet, mit Kohlenstoff-, Sauerstoff- oder Stickstoffnucleophilen zu den entsprechenden Sulfonsäuren, Sulfonsäureester- oder -amid-Derivaten auf an sich bekannte Weise (siehe hierzu insbesondere Seite 351 des Bandes "The Chemistry of Sulphonic Acids, Esters and their Derivatives", John Wiley & Sons 1991, in der Serie "The Chemistry of Functional Groups" sowie S. 530f. in Houben-Weyl, Methoden der Organischen Chemie, Bd. 9, Georg Thieme Verlag, Stuttgart, 4. Auflage 1955):

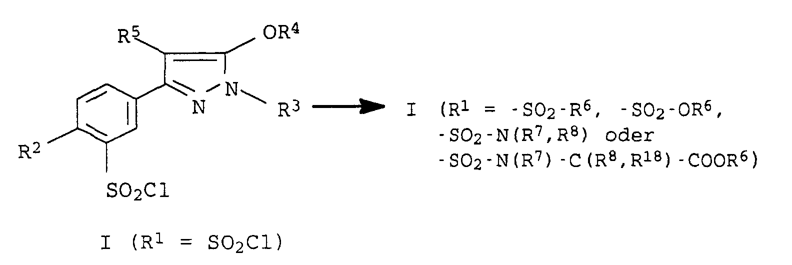

### N) Reduktion von Verbindungen I, bei denen R¹ -SO₂-Cl bedeutet zu den entsprechenden Mercaptanen auf an sich bekannte Weise (siehe hierzu vor allem Seite 216 des Bandes "The Chemistry of the Thiol Groups", John Wiley & Sons 1974, in der Serie "The Chemistry of Functional Groups"):

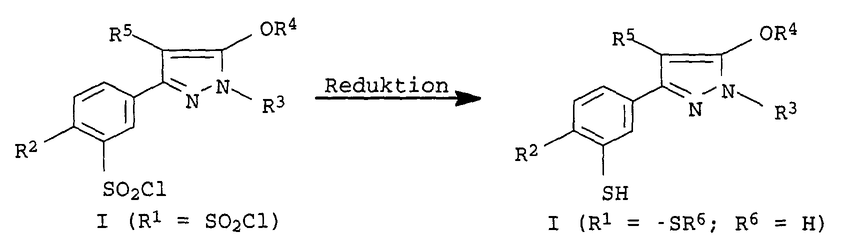

Geeignete Reduktionsmittel sind z.B. Übergangsmetalle wie Eisen, Zink und Zinn.

Die Verbindungen I, bei denen R¹ die Thiolgruppe bedeutet, können auf an sich bekannte Weise (vgl. z.B. S. 721 des Bandes "The Chemistry of Sulphonic Acids, Esters and their Derivatives", John Wiley & Sons 1991, in der Serie "The Chemistry of Functional Groups") in Verbindungen der Formel I überführt werden, bei denen R¹ für -SR⁶ (R⁶ ≠ Wasserstoff) steht. Die Gruppe -SR⁶ wiederum kann auf an sich bekannte Weise zu -SO₂-R⁶ oxidiert werden (vgl. z.B. Houben-Weyl, Methoden der organischen Chemie, Bd. 9, Georg Thieme Verlag, Stuttgart, 4. Auflage 1955, S. 211 ff und S. 227 ff; Org. Synth. Coll. Vol. V, 791).

### O) Überführung von Verbindungen I, bei denen R¹ Formyl bedeutet, auf an sich bekannte Weise in die entsprechenden Acetale (s. hierzu z.B. Houben-Weyl, Methoden der Organischen Chemie, Bd. 6/3, Georg Thieme Verlag, Stuttgart, 4. Auflage 1965, S. 221ff. und S. 250ff.) oder Alkene (vgl. z.B. Houben-Weyl, Methoden der Organischen Chemie, Bd. 5/16, Georg Thieme Verlag, Stuttgart, 4. Auflage 1972, S. 383ff.):

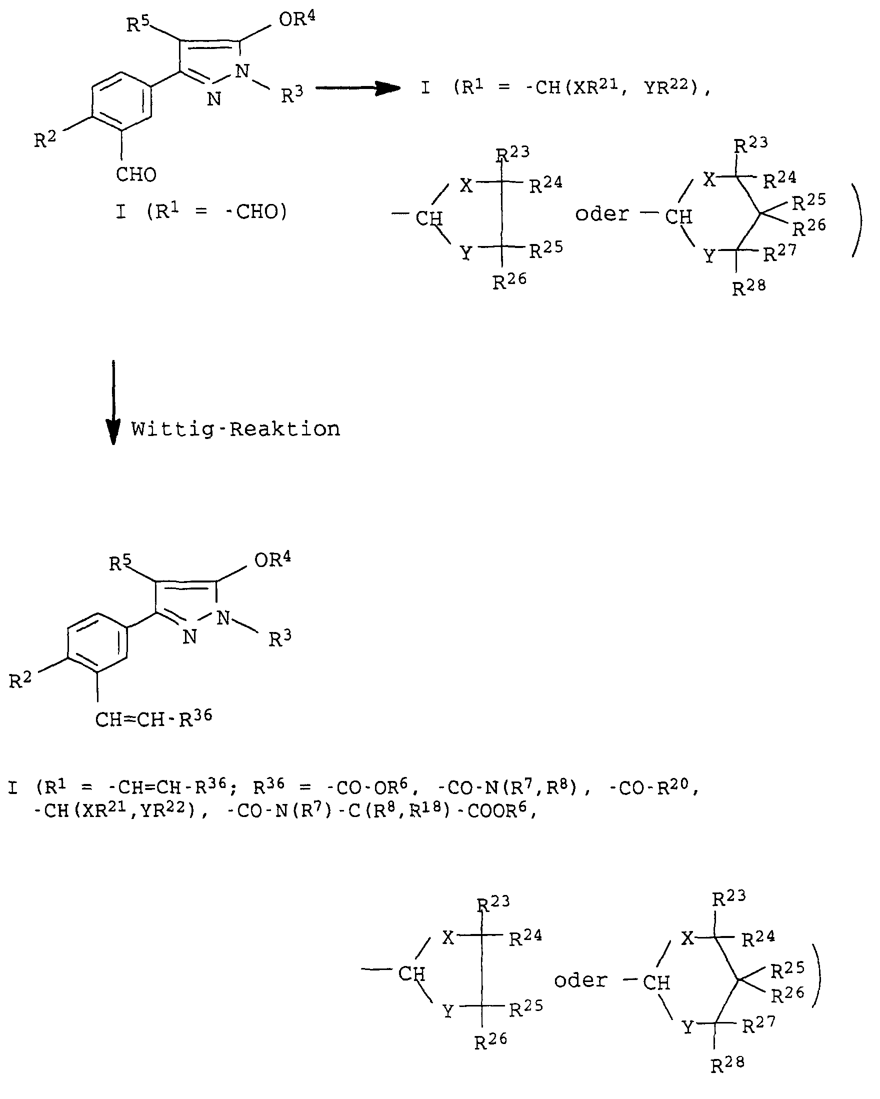

### P) Nucleophile Cyanid-Substitution von Verbindungen I mit R¹ = NO₂ und R² = F:

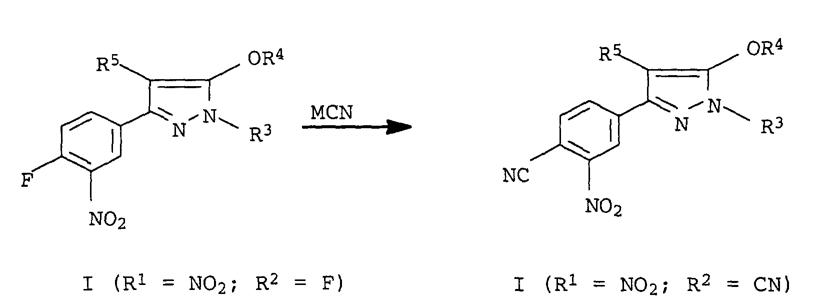

M steht für ein metallisches oder organisches Kation, vorzugsweise für ein Alkalimetall- oder Tetraalkylammoniumion.

Die Reaktion wird üblicherweise in einem polaren aprotischen Lösungsmittel wie Dimethylsulfoxid, N,N-Dimethylformamid und Sulfolan vorgenommen, wobei die Reaktionstemperatur zwischen dessen Schmelz- und Siedepunkt, insbesondere bei 0 bis 100°C liegt.

Vorzugsweise kommt ein geringfügiger molarer Überschuß des Cyanids MCN zur Anwendung. Zur Optimierung des Umsatzes kann es jedoch vorteilhaft sein, einen großen Überschuß an MCN zu verwenden, bis etwa zur fünffachen molaren Menge, bezogen auf die Menge an Ausgangsverbindung I mit R² = Fluor.

Die Aufarbeitung des Reaktionsgemisches kann auf bekannte Weise erfolgen, etwa durch Verdünnen des Reaktionsgemisches mit Wasser und nachfolgender Isolierung des Produkts mittels Filtration, Kristallisation oder Lösungsmittelextraktion.

Sofern nicht anders angegeben werden die vorstehend beschriebenen Reaktionen zweckmäßigerweise bei Atmosphärendruck oder unter dem Eigendruck des jeweiligen Reaktionsgemisches vorgenommen.

Die substituierten 3-Phenylpyrazole I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die reinen Isomeren getrennt werden können. Reine optisch aktive Isomere lassen sich vorteilhaft aus entsprechenden optisch aktiven Ausgangsprodukten herstellen.

Substituierte 3-Phenylpyrazole I, bei denen R¹⁰ für ein Alkalimetall steht, können durch Behandeln von Verbindungen I mit R¹⁰ = Wasserstoff z.B.
- mit Natrium- oder Kaliumhydroxid in wäßriger Lösung oder in einem organischen Lösungsmittel wie Methanol, Ethanol, Aceton und Toluol oder
- mit Natriumhydrid in einem organischen Lösungsmittel wie Dimethylformamid
erhalten werden.

Substituierte 3-Phenylpyrazole I, bei denen R¹⁰ ein landwirtschaftlich brauchbares Kation ist, das nicht zu der Gruppe der Alkalimetalle gehört, können üblicherweise durch Umsalzen der entsprechenden Verbindung I mit R¹⁰ = Alkalimetallion hergestellt werden.

Verbindungen I, bei denen R¹⁰ z.B. ein Mangan-, Kupfer-, Zink-, Eisen-, Calcium-, Magnesium- und Bariumion ist, können aus den Verbindungen I mit R¹⁰ = Natrium in üblicher Weise hergestellt werden, ebenso Verbindungen I mit R¹⁰ = Ammonium- oder Phosphoniumion mittels Ammoniak, Phosphonium-, Sulfonium- oder Sulfoxoniumhydroxiden.

Mittels des beschriebenen Verfahrens können auch alle anderen Salze landwirtschaftlich brauchbarer Kationen der Verbindungen I erhalten werden, beispielsweise solche, die sich von Verbindungen I (R¹ = -SO₂-OR⁶; R⁶ = H) oder (R¹ = -N(R¹⁰)-SO₂-R¹¹; R¹⁰ = H) ableiten.

Diejenigen Verbindungen I, die eine basische funktionelle Gruppe tragen, z.B. wenn R¹ für -N(R⁷,R⁸), -N(R⁷) -N(R⁸,R³²), -N=N-COR⁹ oder -N(R⁷)-N(R⁸)-CO-R⁹ steht, sowie solche Verbindungen I, die die funktionelle Gruppe >C=N- enthalten, können durch Reaktion mit der entsprechenden Säure in ihre Säureadditionssalze übergeführt werden.

Die Säureaddition kann in wäßriger Lösung oder in einem organischen Lösungsmittel wie Methanol, Ethanol, Aceton, Toluol und Ether durchgeführt werden. Auch die Säureadditionssalze von I können umgesalzt werden, wodurch landwirtschaftlich brauchbare Salze mit weiteren Anionen zugänglich sind.

Die Salzbildungen verlaufen normalerweise bereits bei etwa 20 °C mit ausreichender Geschwindigkeit.

Die Isolierung der Salze kann z.B. durch Fällen mit einem geeigneten inerten Lösungsmittel oder durch Abdampfen des Lösungsmittels erfolgen.

Die substituierten 3-Phenylpyrazole I und deren Salze eignen sich, sowohl als Isomerengemische als auch in Form der reinen Isomeren, als Herbizide. Sie können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I in Kulturen, die durch Züchtung und/oder gentechnische Methoden gegen die Wirkung von I weitgehend resistent sind, eingesetzt werden.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden, etwa zwischen 0,01 und 95 Gew. %, vorzugsweise zwischen 0,5 und 90 Gew.%. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
1. 20 Gewichtsteile der Verbindung Nr. Ia.022 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung aus Beispiel 18 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. Ia.019 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des nicht erfindungsgemäßen Wirkstoffs I mit R¹ = CHNOC₂H₅, R² = Cl, R³ = CH₃, R⁴ = CHF₂ und R³ = C1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
V. 3 Gewichtsteile des Wirkstoffs Nr. Ia.097 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. Ia.107 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten 3-Phenylpyrazole I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1- Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1 3-(4-Chlorphenyl)-5-difluormethoxy-1-methyl-1H-pyrazol

Zu einer Lösung von 169,1 g (0,811 mol) 3-(4-Chlorphenyl)-1-methyl-2-pyrazolin-5-on in 2,5 1 Dioxan wurden 162 g (4,0 mol) Natriumhydroxid, gelöst in 1 1 Wasser, gegeben. In diese Mischung leitete man bei 60 - 65 °C 5 Std. lang Chlordifluormethan ein, wonach die Reaktionslösung in 1,5 1 Wasser eingerührt wurde. Zur Aufarbeitung extrahierte man dreimal (jeweils ca. 1000 ml) Methyl-tert-butylether. Die vereinigten organischen Phasen wurden getrocknet und unter reduziertem Druck eingeengt. Die Reinigung des Rohproduktes erfolgte mittels Chromatographie an Kieselgel (Laufmittel: Hexan/Essigester = 7:3). Ausbeute: 142,9 g;
¹H-NMR (400 MHz, in CDCl₃): δ [ppm] = 7,60 (d,2H), 7,28 (d,2H), 6,54 (t,1H), 6,08 (s,1H), 3,69 (s,3H).

### Vorstufe α): 4-Chlorbenzoyl-essigsäureethylester

Zu einer Mischung aus 296,4 g (2,59 mol) Kalium-tert-butylat und 2,25 1 Diethylcarbonat wurden bei 60° C 200 g (1,29 mol) 4-Chloracetophenon (gelöst in 500 ml Diethylcarbonat) zugetropft. Die schwer rührbare Suspension wurde 3 Std. bei 60° C gerührt und danach in 2,7 1 10 gew.-%ige Schwefelsäure eingetragen. Anschließend extrahierte man das Produkt mit Essigester, wonach dieser über Magnesiumsulfat getrocknet und dann unter reduziertem Druck eingeengt wurde. Die Reinigung des erhaltenen Rohproduktes erfolgte destillativ. Ausbeute: 268 g; Sdp.: 130° C bei 0,4 mbar.

### Vorstufe β): 3-(4-Chlorphenyl)-1-methyl-2-pyrazolin-5-on

Zu einer Suspension von 267 g (1,19 mol) 4-Chlorbenzoylessigsäureethylester in 1,5 1 Essigsäure wurden innerhalb von 40 Minuten 71,2 g (1,55 mol) Methylhydrazin getropft, wobei die Reaktionstemperatur auf 50° C anstieg. Nach beendeter Zugabe rührte man 2 Std. bei 100° C, ließ dann die Reaktionsmischung abkühlen und versetzte sie schließlich mit ca. 1,5 1 Ether und ca. 1,5 1 Wasser. Das gebildete feste Produkt (ca. 66,5 g) wurde abgetrennt und mit einem Gemisch aus Petrolether und Diethylether (1:1) gewaschen. Die organische Phase, die noch gelöstes Produkt enthielt, wurde 4 mal mit je 500 ml gesättigter wässriger Natriumhydrogencarbonat-Lösung gewaschen und danach aufkonzentriert (Restmenge ca. 200 ml). Mittels Zugabe von etwa 1000 ml Wasser wurde eine weitere Menge an Produkt ausgefällt. Auch dieses Produkt wurde nach Abtrennung mit einem Gemisch aus Petrolether und Diethylether (1:1) gewaschen. Gesamtproduktmenge: 171 g.; Smp. 189° C.

### Beispiel 2

### 4-Chlor-3-(4-chlorphenyl)-5-difluormethoxy-1-methyl-1H-pyrazol (Nr. Ia.098)

74,0 g (0,549) Sulfurylchlorid, gelöst in 200 ml Tetrachlorkohlenstoff, wurden langsam zu einer Lösung von 128,9 g (0,5 mol) 3-(4-Chlorphenyl)-5-difluormethoxy-1-methyl-1H- pyrazol in 500 ml Tetrachlorkohlenstoff getropft, wobei die Reaktionspartner in einer exothermen Reaktion unter Gasentwicklung abreagierten. Nach Beendigung der Reaktion rührte man das Gemisch noch 2 Std. bei ca. 20 °C. Anschließend wurde die Reaktionslösung mit jeweils ca. 300 ml Wasser, gesättigter Natriumhydrogencarbonat-Lösung und gesättigter Natriumchlorid-Lösung gewaschen, dann getrocknet und eingeengt. Ausbeute: 139,3 g;
¹H-NMR (270 MHz, in CDCl₃): δ [ppm] = 7,79 (d,2H), 7,43 (d,2H), 6,70 (t,1H), 3,85 (s,3H).

### Beispiel 3

### 3-(3-Brommethyl-4-chlorphenyl)-4-chlor-5-difluormethoxy-1-methyl-1H-pyrazol (Nr. Ia.024)

Eine Mischung aus 20,0 g (65 mmol) 4-Chlor-5-difluormethoxy-3-(4-chlor-3-methylphenyl)-1-methyl-1H-pyrazol (hergestellt analog Beispiel 2), 17,4 g (98 mmol) N-Bromsuccinimid und 200 ml Tetrachlorkohlenstoff wurde drei Std. auf Rückflußtemperatur erhitzt und dabei mit einer UV-Lampe bestrahlt. Anschließend filtrierte man die festen Bestandteile ab und wusch sie mit wenig Methylenchlorid. Nach Entfernen des Lösungsmittels bei reduziertem Druck wurde der Rückstand mittels Chromatographie an Kieselgel (Laufmittel: Methylenchlorid/Hexan = 8:2) gereinigt. Ausbeute: 11,0 g;
¹H-NMR (270 MHz, in CDCl₃): δ [ppm] = 7,98 (s,1H), 7,80 (d,1H), 7,46 (d,1H), 6,70 (t,1H), 4,64 (s,2H), 3,87 (s,3H).

### Beispiel 4

### 3- (3-Dibrommethyl-4-chlorphenyl)-4-chlor-5-difluormethoxy-1-methyl-1H-pyrazol (Nr. Ia.097)

zu einer Lösung von 3,0 g (9,8 mmol) 4-Chlor-5-difluormethoxy-3-(4-chlor-3-methylphenyl)-1-methyl-1H-pyrazol, gelöst in 100 ml Tetrachlorkohlenstoff wurden 10,5 g (58,8 mmol) N-Bromsuccinimid gegeben. Diese Mischung erhitzte man unter Bestrahlung mit einer 150 Watt Quecksilber-Hochdrucklampe 1 Stunde lang auf Rückflußtemperatur. Danach wurde das Reaktionsgemisch filtriert. Einengen des Filtrates unter reduziertem Druck ergab 3,9 g Rohprodukt, das ohne weitere Reinigung für die folgenden Umsetzungen verwendet wurde.
¹H-NMR (400 MHz, in CDCl₃): δ [ppm] = 8,56 (s,1H), 7,79 (d,1H), 7,46 (d,1H), 7,15 (d,1H), 6,70 (t,1H), 3,85 (s,3H).

### Beispiel 5

### 4-Chlor-3-(4-chlor-3-formylphenyl)-5-difluormethoxy-1-methyl-1H-pyrazol (Nr. Ia.055)

4,65 g (10 mmol) 3-(3-Dibrommethyl-4-chlorphenyl)-4-chlor-5-difluormethoxy-1-methyl-1H-pyrazol wurden bei 85 °C portionsweise in 7 ml konzentrierte Schwefelsäure eingetragen. Anschließend rührte man noch 5 Minuten bei 100 °C, wonach die Reaktionslösung in 40 ml Eiswasser eingerührt wurde. Das gebildete feste Produkt wurde abgetrennt und getrocknet. Ausbeute 3,0 g; ¹H-NMR (270 MHz, in CDCl₃) : δ [ppm] = 10,50 (s,1H), 8,45 (s,1H), 8,06 (d,1H), 7,52 (d,1H), 6,71 (t,1H), 3,82 (s,3H).

### Beispiel 6

### 3-(3-Carboxy-4-chlorphenyl)-4-chlor-5-difluormethoxy-1-methyl-1H-pyrazol (Nr. Ia.044)

Zu einer Lösung von 40 g (125 mmol) 4-Chlor-3-(4-chlor-3-formylphenyl)-5-difluormethoxy-1-methyl-1H-pyrazol in 125 ml Acetonitril wurden bei 10-15°C eine Lösung von 4 g (34 mmol) Natriumdihydrogenphosphat in 40 ml Wasser und dann 12,5 ml einer 35%igen Wasserstoffperoxid-Lösung gegeben. Anschließend versetzte man die Mischung bei 10°C tropfenweise mit einer Lösung von 18,1 g (200 mmol) Natriumchlorit in 158 ml Wasser. Nach einer Stunde Rühren wurde mit 3N Salzsäure bis pH = 1 angesäuert. Das im Reaktionsgemisch nun suspendierte Produkt wurde abgetrennt und aus Methanol/Wasser umkristallisiert. Ausbeute: 24,5 g.

### Beispiel 7

### 4-Chlor-3-(4-chlor-3-[N-(methoxycarbonylmethyl)-methylaminocarbonyl]-phenyl)-5-difluormethoxy-1-methyl-1H-pyrazol (Nr. Ia.135)

Zu einer Lösung von 3 g (8,4 mmol) 4-Chlor-3-(4-chlor-3-chlorcarbonylphenyl)-5-difluormethoxy-1-methyl-1H-pyrazol in 80 ml Tetrahydrofuran wurden 3,5 g (25 mmol) Kaliumcarbonat (gemahlen) und 1,2 g (8,4 mmol) Methylaminoessigsäuremethylester-Hydrochlorid gegeben. Anschließend erwärmte man das Gemisch 5 Stunden auf 60°C, wonach es abgekühlt und eingeengt wurde. Den Rückstand versetzte man mit Wasser und Ethylacetat. Die organische Phase wurde abgetrennt, über Magnesiumsulfat getrocknet und eingeengt. Die Reinigung des so erhaltenen Rohprodukts erfolgte mittels Säulenchromatographie an Kieselgel (Eluent: Hexan/Ethylacetat = 1:1). Ausbeute: 1,9 g.

### Vorstufe α): 4-Chlor-3-(4-chlor-3-chlorcarbonylphenyl)-5-difluormethoxy-1-methyl-1H-pyrazol

Zu einer Lösung von 11 g (29 mmol) 3-(3-Carboxy-4-chlorphenyl)-4-chlor-5-difluormethoxy-1-methyl-1H-pyrazol in 100 ml Toluol wurden nacheinander tropfenweise 11,2 g (88 mmol) Oxalylchlorid und ein Tropfen Dimethylformamid gegeben. Nach 5 Stunden Rühren bei Raumtemperatur engte man das Reaktionsgemisch ein. Ausbeute: quantitativ.

### Beispiel 8

### 4-Chlor-3-(4-chlor-3-[(2-methoxyimino)ethoxycarbonyl]phenyl)-5-difluormethoxy-1-methyl-1H-pyrazol (Nr. Ia.085)

Zu einer Lösung von 3 g (8,9 mmol) 3-(3-Carboxy-4-chlorphenyl)-4-chlor-5-difluormethoxy-1-methyl-1H-pyrazol in 60 ml Dimethylformamid wurden 1,7 g (12 mmol) gemahlenes Kaliumcarbonat gegeben. Nach Zutropfen von 1,0 g (8,9 mmol) 1-Chlor-2-methoxyiminoethan erwärmte man das Gemisch 5 Stunden auf 60°C. Danach wurde es abgekühlt und eingeengt. Aus dem Rückstand extrahierte man, nach Zusatz von Wasser, das Produkt mit Ethylacetat. Die Ethylacetat-Lösung wurde anschließend nochmals mit Wasser gewaschen, dann über Magnesiumsulfat getrocknet und eingeengt. Die Reinigung des Rohproduktes erfolgte mittels Säulenchromatographie an Kieselgel (Eluent: Hexan/Ethylacetat = 2:1). Ausbeute: 1,3 g (als E/Z-Gemisch).

### Beispiel 9

### 4-Chlor-3-[4-chlor-3-(4,4-diethoxy-3-oxo-but-1-en-1-yl)-phenyl]-5-difluormethoxy-1-methyl-1H-pyrazol (Nr. Ia.139)

Zu einer Lösung von 2 g (6,2 mmol) 4-Chlor-3-(4-chlor-3-formylphenyl)-5-difluormethoxy-1-methyl-1H-pyrazol in 20 ml Dimethylformamid wurden 5,1 g (3,3-Diethoxy-2-oxopropyliden)(triphenyl)phosphoran gegeben, wonach man für 5 Stunden auf 80-90°C erwärmte. Anschließend wurde das Gemisch eingeengt. Die Reinigung des so erhaltenen Rohproduktes erfolgte mittels Chromatographie an Kieselgel (Eluent: Hexan/Ethylacetat = 4:1). Ausbeute: 2,1 g.

### Beispiel 10

### 4-Chlor-3-[4-chlor-3-(dimethoxymethyl)-phenyl]-5-difluormethoxy-1-methyl-1H-pyrazol (Nr. Ia.056)

Zu einer Lösung von 16 ml Trimethylorthoformiat in 80 ml Dichlormethan wurden 15 g Montmorillonit K10 gegeben. Zu dieser Mischung tropfte man bei 5°C eine Lösung von 6,4 g (20 mmol) 4-Chlor-3-(4-chlor-3-formylphenyl)-5-difluormethoxy-1-methyl-1H-pyrazol in 20 ml Dichlormethan. Anschließend wurde das Gemisch über Nacht bei Raumtemperatur gerührt. Dann trennte man den Feststoffanteil ab, wonach die nun klare Reaktionslösung eingeengt wurde. Ausbeute: 6,7 g.

### Beispiel 11

### 4-Chlor-3-[4-chlor-3-(1,3-dithian-2-yl)-phenyl]-5-difluormethoxy-1-methyl-1H-pyrazol (Nr. Ia.067)

Zu einer Lösung von 1,7 g (4,6 mmol) 4-Chlor-3-[4-chlor-3-(dimethoxymethyl)-phenyl]-5-difluormethoxy-1-methyl-1H-pyrazol in 50 ml Toluol wurden 0,5 g (4,6 mmol) 1,3-Propandithiol gegeben. Nach Erwärmen auf 60-70°C versetzte man die Mischung noch mit einer Spatelspitze p-Toluolsulfonsäure. Für 3 Stunden wurden bei Rückflußtemperatur gerührt. Nach Abkühlen der Reaktionsmischung verdünnte man mit 50 ml Toluol. Die organische Phase wurde mit 10 gew.-%iger Natriumhydrogencarbonat-Lösung und Wasser gewaschen, dann über Magnesiumsulfat getrocknet und eingeengt. Die Reinigung des so erhaltenen Rohprodukts erfolgte mittels Chromatographie an Kieselgel (Eluent: Hexan/Ethylacetat = 4:1). Ausbeute: 1,8 g.

### Beispiel 12

### 3-(3-Acetoxymethyl-4-chlorphenyl)-4-chlor-5-difluormethoxy-1-methyl-1H-pyrazol (Nr. Ia.084)

Eine Lösung von 6,8 g (18 mmol) 3-(3-Brommethyl-4-chlorphenyl)-4-chlor-5-difluormethoxy-1-methyl-1H-pyrazol und 1,5 g (18 mmol) Natriumacetat in 20 ml Dimethylformamid wurde über Nacht gerührt und dann in 100 ml kaltes Wasser eingerührt. Aus der wäßrigen Phase extrahierte man das Produkt mittels Ethylacetat. Die Ethylacetatphase wurde über Magnesiumsulfat getrocknet und eingeengt. Die Reinigung des so erhaltenen Rohprodukts erfolgte mittels Kieselgelchromatographie (Eluent: Hexan/Ethylacetat = 9:1). Ausbeute: 4,7 g.

### Beispiel 13

### 4-Chlor-3-(4-chlor-3-hydroxymethylphenyl)-5-difluormethoxy-1-methyl-1H-pyrazol (Nr. Ia.079)

Zu einer Lösung von 3,2 g (8,8 mmol) 3-(3-Acetoxymethyl-4-chlorphenyl)-4-chlor-5-difluormethoxy-1-methyl-1H-pyrazol in 10 ml Dioxan und 10 ml Wasser wurden 6,3 ml (16 mmol) einer 10%igen Natronlauge getropft. Nach einer Stunde Rühren neutralisierte man mit 3N Salzsäure. Aus der wäßrigen Phase wurde das Produkt mit Ethylacetat extrahiert, wonach die organische Phase über Magnesiumsulfat getrocknet und dann eingeengt wurde. Die Reingung des so erhaltenen Rohprodukts erfolgte mittels Säulenchromatographie an Kieselgel (Eluent: Hexan/Ethylacetat = 4:1). Ausbeute: 1,3 g.

### Beispiel 14

### 4-Chlor-3-(4-chlor-3-methoxymethylphenyl)-5-difluormethoxy-1-methyl-1H-pyrazol (Nr. Ia.025)

Zu einer Lösung von 2,3 g (6,0 mmol) 3-(3-Brommethyl-4-chlorphenyl)-4-chlor-5-difluormethoxy-1-methyl-1H-pyrazol in 60 ml Methanol wurden 1,6 g (9,0 mmol) einer 30 gew.-%igen Lösung von Natriummethylat in Methanol getropft. Nach 3 Stunden Rühren bei 40°C engte man das Reaktionsgemisch ein. Der Rückstand wurde in Wasser und Dichlormethan aufgenommen, wonach man konzentrierte Salzsäure bis zur sauren Reaktion zugab. In der wäßrigen Phase verbliebene Produktmengen wurden mit Dichlormethan extrahiert. Die vereinigten Dichlormethan-Phasen wurden über Magnesiumsulfat getrocknet und eingeengt. Ausbeute: 1,8 g.

### Beispiel 15

### 4-Chlor-3-[4-Chlor-3-(ethylthiomethyl)-phenyl]-5-difluormethoxy-1-methyl-1H-pyrazol (Nr. Ia.040)

Zu einer Lösung von 6 g (16 mmol) 3-(3-Brommethyl-4-chlorphenyl)-4-chlor-5-difluormethoxy-1-methyl-1H-pyrazol in 100 ml Aceton wurden 1,3 g (16 mmol) Natriumthioethylat gegeben. Nach Rühren über Nacht wurde das Gemisch mit Wasser versetzt. Aus der wäßrigen Phase extrahierte man das Produkt mit Ethylacetat. Anschließend wurde die Ethylacetat-Phase über Magnesiumsulfat getrocknet und dann eingeengt. Die Reinigung des so erhaltenen Rohprodukts erfolgte mittels Säulenchromatographie an Kieselgel (Eluent: Hexan/Ethxlyacetat = 4:1). Ausbeute: 4,5 g.

### Beispiel 16

### 4-Chlor-3-[4-chlor-3-(ethylsulfinylmethyl)-phenyl]-5-difluormethoxy-1-methyl-1H-pyrazol (Nr. Ia.103)

Zu einer Lösung von 1,5 g (4 mmol) 4-Chlor-3-[4-chlor-3-(ethylthiomethyl)-phenyl]-5-difluormethoxy-1-methyl-1H-pyrazol in 40 ml Dichlormethan wurden bei 0°C portionsweise 1,4 g (4 mmol) m-Chlorperbenzoesäure gegeben. Nach 30 Minuten Rühren bei 0°C versetzte man die Mischung mit 0,5 g Calciumhydroxid. Dann wurde noch 4 Stunden bei Raumtemperatur nachgerührt. Schließlich filtrierte man den Feststoffanteil ab. Das Filtrat wurde eingeengt. Das so erhaltene Rohprodukt wurde chromatographisch an Kieselgel (Eluent: Hexan/Ethylacetat = 1:1) gereinigt. Ausbeute: 0,2 g.

### Beispiel 17

### 4-Chlor-3-[4-chlor-3-(ethylsulfonylmethyl)-phenyl]-5-difluormethoxy-1-methyl-1H-pyrazol (Nr. Ia.104)

Zu einer Lösung von 1,6 g (4,4 mmol) 4-Chlor-3-[4-chlor-3-(ethylthiomethyl)-phenyl]-5-difluormethoxy-1-methyl-1H-pyrazol in 12 ml Essigsäure wurden 0,2 g Dinatriumwolframat(VI) gegeben. In diese Mischung tropfte man anschließend 2 ml einer 30%igen Wasserstoffperoxid-Lösung. Nach 1 Stunde Rühren versetzte man das Reaktionsgemisch mit 100 ml Wasser. Das Produkt wurde aus der abgetrennten wäßrigen Phase mit Ethylacetat extrahiert. Danach trocknete man die Ethylacetat-Phase über Magnesiumsulfat und engte sie anschließend ein. Das so erhaltene Rohprodukt wurde mittels Säulenchromatographie an Kieselgel (Eluent: Hexan/Ethylacetat = 4:1) gereinigt. Ausbeute: 1,4 g.

### Beispiel 18

### 4-Chlor-3-(4-chlor-3-chlorsulfonylphenyl)-5-difluormethoxy-1-methyl-1H-pyrazol

47,9 g (0,16 mol) 4-Chlor-3-(4-chlorphenyl)-5-difluormethoxy-1-methyl-1H-pyrazol wurden bei 0°C portionsweise zu 91 ml Chlorsulfonsäure gegeben. Nach 4 Stunden Rühren bei 130°C kühlte man ab und goß die Lösung vorsichtig auf Eiswasser. In der wäßrigen Phase gelöstes Produkt wurde zweimal mit Dichlormethan extrahiert. Die vereinigten organischen Phasen wurden anschließend mit gesättigten Natriumhydrogencarbonat- und mit Natriumchlorid-Lösungen gewaschen, dann über Magnesiumsulfat getrocknet und schließlich eingeengt. Ausbeute: 47,9 g. (Schmelzpunkt 102-105°C)

### Beispiel 19

### 4-Chlor-3-[4-chlor-3-(cyclopropylaminosulfonyl)-phenyl]-5-difluormethoxy-1-methyl-lH-pyrazol (Nr. Ia.107)

Zu einer Lösung von 3 g (7,6 mmol) 4-Chlor-3-(4-chlor-3-chlorsulfonylphenyl)-5-difluormethoxy-1-methyl-1H-pyrazol in 25 ml Tetrahydrofuran wurden 0,9 g (16 mmol) Cyclopropylamin gegeben. Nach 4 Stunden Rühren bei Raumtemperatur engte man bis zur Trockene ein. Der feste Rückstand wurde mit wenig Diisopropylether gewaschen. Ausbeute: 3 g.

### Beispiel 20

### 4-Chlor-3-(4-chlor-3-[N-(methylaminocarbonylmethyl)-methylaminosulfonyl]-phenyl)-5-difluormethoxy-1-methyl-1H-pyrazol (Nr. Ia.120)

Zu einer Lösung von 1,5 g (3,3 mmol) 4-Chlor-3-(4-chlor-3-[N-(methoxycarbonylmethyl)-methylaminosulfonyl]-phenyl)-5-difluormethoxy-1-methyl-lH-pyrazol in 40 ml Tetrahydrofuran wurden 2,5 g einer 40%igen wäßrigen Methylaminlösung gegeben. Nach 2 Stunden Rühren bei 50°C versetzte man das Reaktionsgemisch mit weiteren 3 ml der Methylaminlösung. Dann erwärmte man die Mischung noch 6 Stunden lang auf 50°C. Anschließend wurde eingeengt, wonach man den Rückstand in Ethylacetat aufnahm. Die Ethylacetat-Phase wurde mit Wasser, verdünnter Salzsäure und gesättigter Natriumchlorid-Lösung gewaschen, dann über Magnesiumsulfat getrocknet und schließlich eingeengt. Nach Waschen mit wenig Hexan/Ethylacetat (9:1) erhielt man 1,1 g Wertprodukt.

### Beispiel 21

### 4-Chlor-3-(4-chlor-3-nitrophenyl)-5-difluormethoxy-1-methyl-1H-pyrazol (Nr. Ia.012)

Bei (-40)°C wurden zu 600 ml 98%iger Salpetersäure portionsweise 113,4 g (0,387 mol) 4-Chlor-3-(4-chlorphenyl)-5-difluormethoxy-1-methyl-1H-pyrazol gegeben. Nach 2 Stunden Rühren bei dieser Temperatur wurde die Lösung in Eis eingerührt. Das Produkt extrahierte man aus der wäßrigen Phase mit Dichlormethan. Die Dichlormethan-Phase wurde anschließend mehrmals mit Wasser, dann mit gesättigter Natriumchlorid-Lösung gewaschen. Nach Trocknen über Magnesiumsulfat und Entfernen des Dichlormethans erhielt man 119,6 g Wertprodukt.

### Beispiel 22

### 3-(3-Amino-4-chlorphenyl)-4-chlor-5-difluormethoxy-1-methyl-1H-pyrazol (Nr. Ia.013)

Eine Aufschlämmung von 32,1 g (0,576 mol) Eisenpulver in 305 ml Ethanol wurde mit 155 ml Essigsäure versetzt. Zu dieser Mischung gab man bei 70-75°C portionsweise 64,9 g (0,192 mol) 4-Chlor-3-(4-chlor-3-nitrophenyl)-5-difluormethoxy-1-methyl-1H-pyrazol. Nach 1 Stunde wurden 0,5 1 Ethylacetat zugesetzt, wonach man die Lösung über ein Kieselgelbett filtrierte und dann einengte. Der Rückstand wurde in Ethylacetat aufgenommen. Die Ethylacetat-Phase wurde mit Wasser, gesättigter Natriumhydrogencarbonat-Lösung und gesättigter Natriumchlorid-Lösung gewaschen, dann über Magnesiumsulfat getrocknet und schließlich eingeengt. Ausbeute: 56,4 g.

### Beispiel 23

### 4-Chlor-3-[4-Chlor-3-(cyclopropylcarbonylamino)-phenyl]-5-difluormethoxy-1-methyl-1H-pyrazol (Nr. Ia.078)

Zu einer Lösung von 2,0 g (6,5 mmol) 3-(3-Amino-4-chlorphenyl)-4-chlor-5-difluormethoxy-1-methyl-1H-pyrazol in 30 ml Tetrahydrofuran wurden 1,0 g (13 mmol) Pyridin und 0,7 g (6,5 mmol) Cyclopropancarbonsäurechlorid gegeben. Die Lösung wurde über Nacht bei Raumtemperatur und für 2 Stunden bei 50°C gerührt, wonach man sie einengte. Den Rückstand nahm man in Ethylacetat auf. Die Ethylacetat-Phase wurde nacheinander mit kalter 1N Salzsäure, Wasser und gesättigter Natriumchlorid-Lösung gewaschen, dann über Magnesiumsulfat getrocknet und schließlich eingeengt. Ausbeute: 2,2 g.

### Beispiel 24

### 4-Chlor-3-(4-chlor-3-[di(methylsulfonyl)amino]-phenyl)-5-difluormethoxy-1-methyl-1H-pyrazol (Nr. Ia.015)

Zu einer Lösung von 3 g (10 mmol) 3-(3-Amino-4-chlorphenyl)-4-chlor-5-difluormethoxy-1-methyl-1H-pyrazol in 50 ml Dichlormethan wurden 2,3 g (22 mmol) Triethylamin gegeben. Zu dieser Mischung tropfte man bei 0°C 2,5 g (21 mmol) Methansulfonsäurechlorid. Nach 2 Stunden Rühren bei Raumtemperatur wurde das Reaktionsgemisch mit Wasser gewaschen. Dann trocknete man die organische Phase über Magnesiumsulfat und engte sie ein. Das feste Produkt wurde mit wenig Diethylether gewaschen. Ausbeute: 3,1 g.

### Beispiel 25

### 4-Chlor-3-[4-chlor-3-(methylsulfonylamino)-phenyl]-5-difluormethoxy-1-methyl-1H-pyrazol (Nr. Ia.014)

Zu einer Lösung von 0,9 g (2,3 mmol) 4-Chlor-3-(4-chlor-3-[di(methylsulfonyl)-amino]-phenyl)-5-difluormethoxy-1-methyl-1H-pyrazol in 30 ml Methanol wurden 0,3 g (4,7 mmol) Kaliumhydroxid (gelöst in wenig Wasser) gegeben. Nach 18 Stunden Rühren bei Raumtemperatur engte man ein. Der Rückstand wurde in 1N Salzsäure aufgenommen, wonach man mit Ethylacetat extrahierte. Nach Trocknen der organischen Phase über Magnesiumsulfat und einengen erhielt man 0,7 g Wertprodukt.

### Beispiel 26

### 4-Chlor-3-[4-chlor-3-hydrazinophenyl]-5-difluormethoxy-1-methyl-1H-pyrazol-Hydrochlorid (Nr. Ia.125)

4,45 g (14,5 mmol) 3-(3-Amino-4-chlorphenyl)-4-chlor-5-difluormethoxy-1-methyl-1H-pyrazol wurden durch kurzes Erwärmen auf 80°C in 26 ml konzentrierter Salzsäure gelöst. Dann kühlte man auf 0°C und tropfte bei dieser Temperatur eine Lösung von 1,0 g (14,5 mmol) Natriumnitrit in 4 ml Wasser zu. Anschließend wurde noch 30 Minuten bei 0°C nachgerührt (Diazoniumsalzlösung).

Die Diazoniumsalzlösung wurde in eine auf 0 - 5°C gekühlte Lösung von 8,1 g (3,6 mmol) Zinn(II)chlorid-Dihydrat in 5 ml konzentrierter Salzsäure getropft. Anschließend rührte man noch zwei Stunden bei Raumtemperatur, wonach die Reaktionsmischung auf 200 ml Wasser gegossen wurde. Aus der erhaltenen Suspension trennte man das feste Wertprodukt ab. Ausbeute: 3,7 g.

### Beispiel 27

### 4-Chlor-3-(4-cyano-3-nitrophenyl)-5-difluormethoxy-1-methyl-1H-pyrazol (Nr. Ie.012)

Zu einer Lösung von 5,0 g (15,6 mmol) 4-Chlor-3-(4-fluor-3-nitrophenyl)-5-difluormethoxy-1-methyl-1H-pyrazol in 70 ml Dimethylsulfoxid wurden 1,1 g (17 mmol) Kaliumcyanid gegeben. Anschließend rührte man 5 Stunden bei 50°C und 3 Tage bei Raumtemperatur. Nach Gießen der Reaktionsmischung auf Eiswasser wurde das Produkt mit Methyl-tert.-butylether extrahiert. Die Etherphase wurde über Magnesiumsulfat getrocknet und eingeengt. Die Reinigung des Rohproduktes erfolgte mittels Säulenchromatographie an Kieselgel (Eluent: Hexan/Essigester = 3:1). Ausbeute: 2,7 g.

In den folgenden Tabellen 1 - 6 sind weitere Verbindungen angegeben, die auf die gleiche Weise hergestellt wurden oder entweder nach den vorstehend beschriebenen Verfahren oder nach an sich bekannten Methoden herstellbar sind.

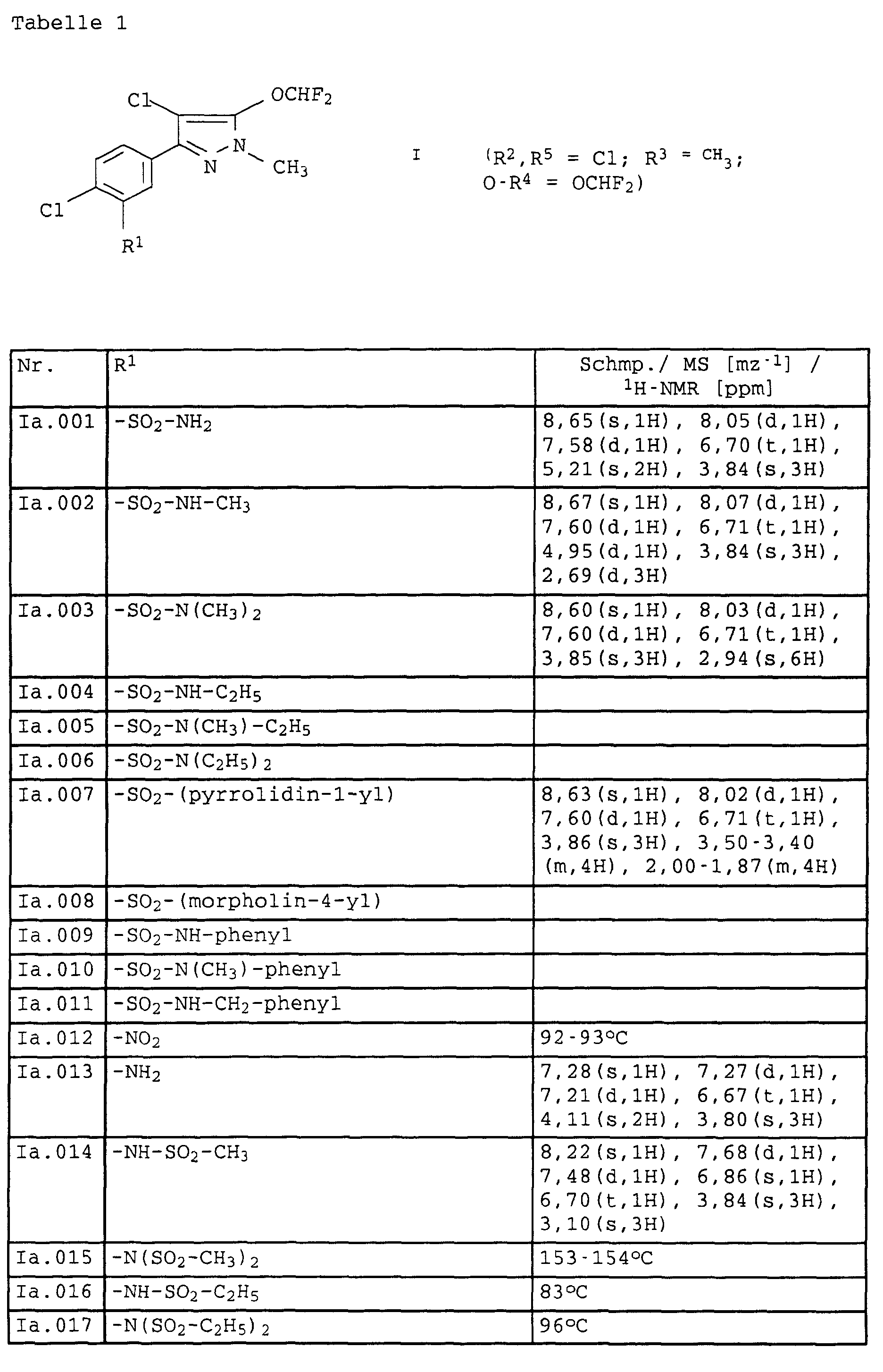

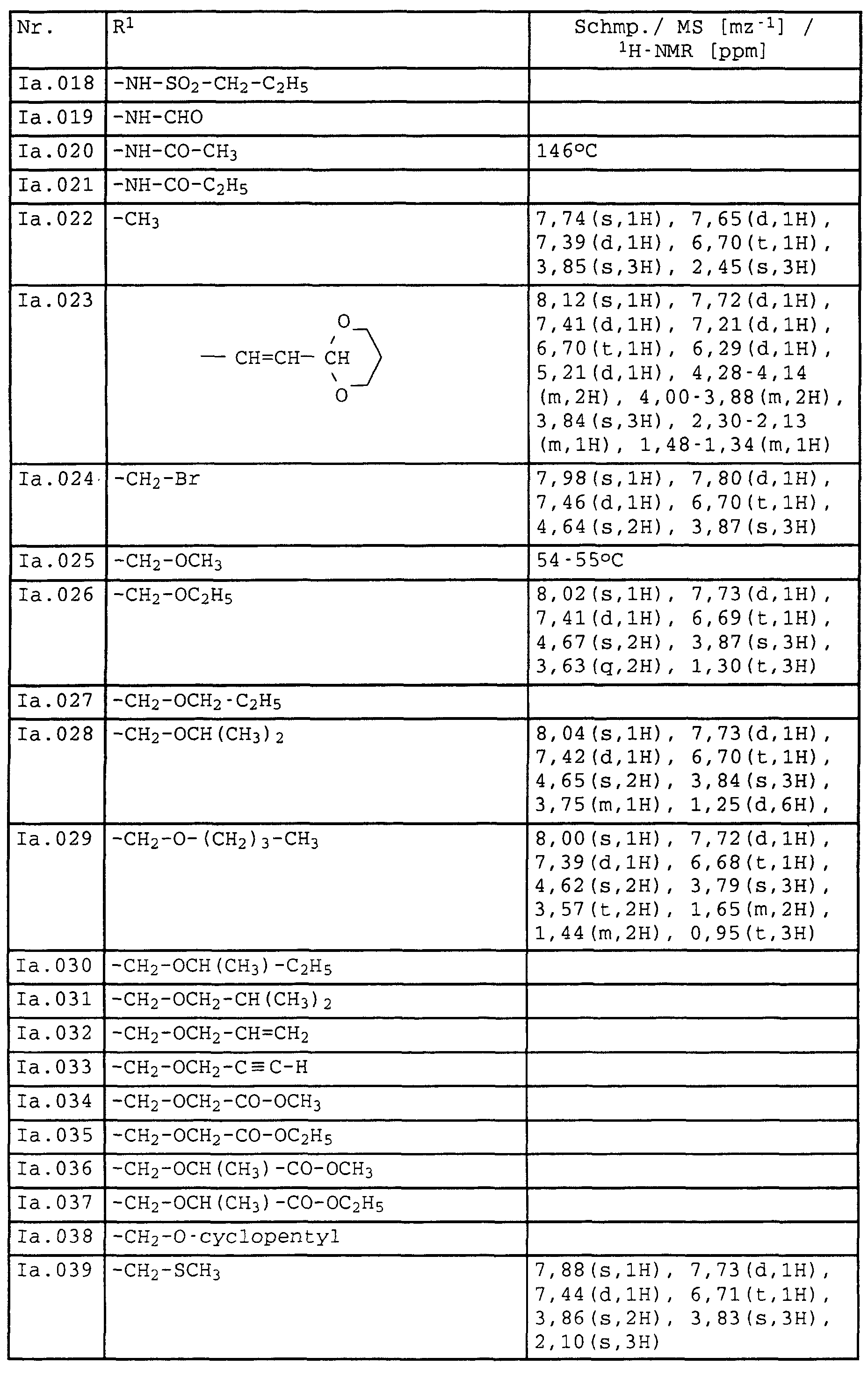

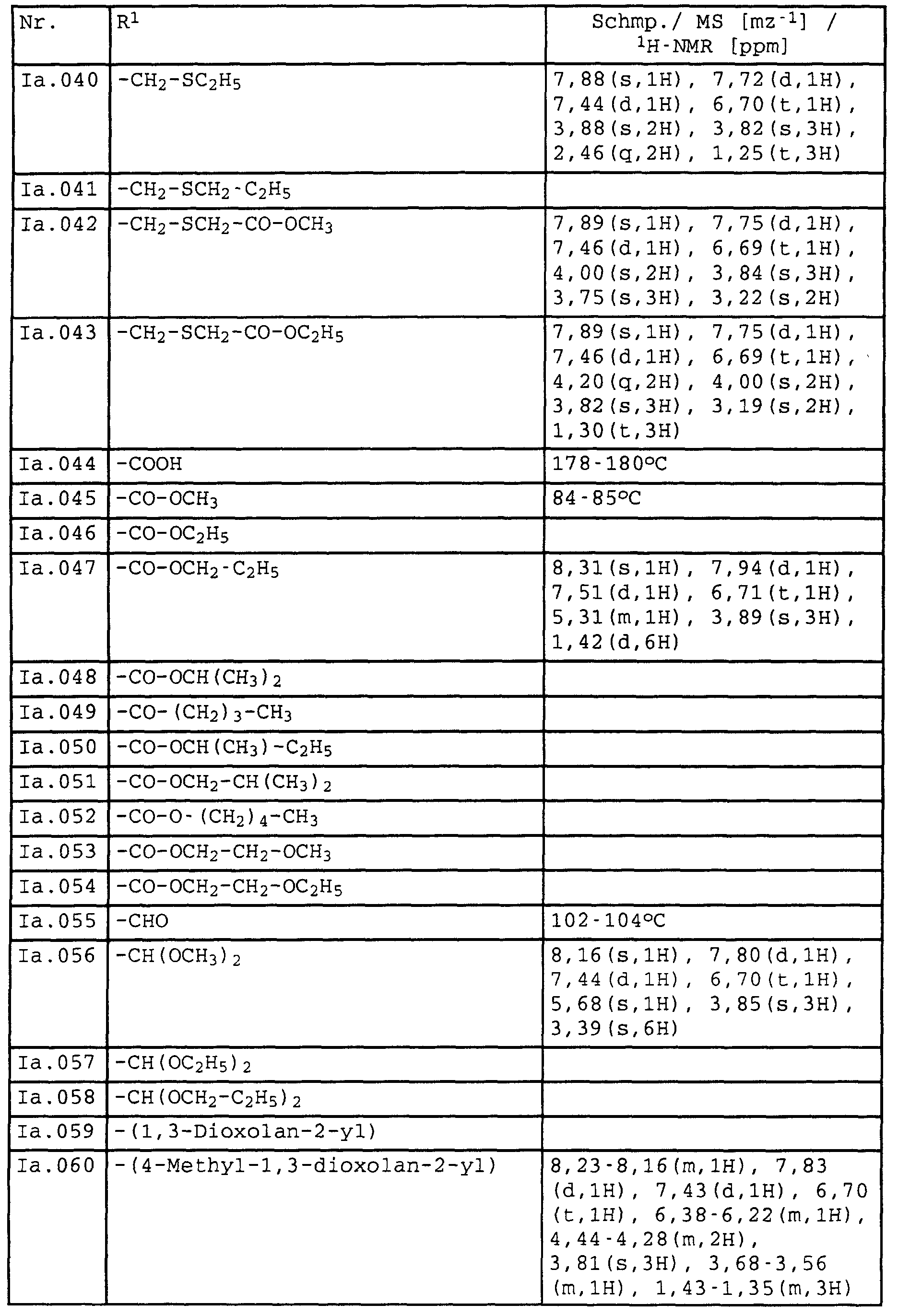

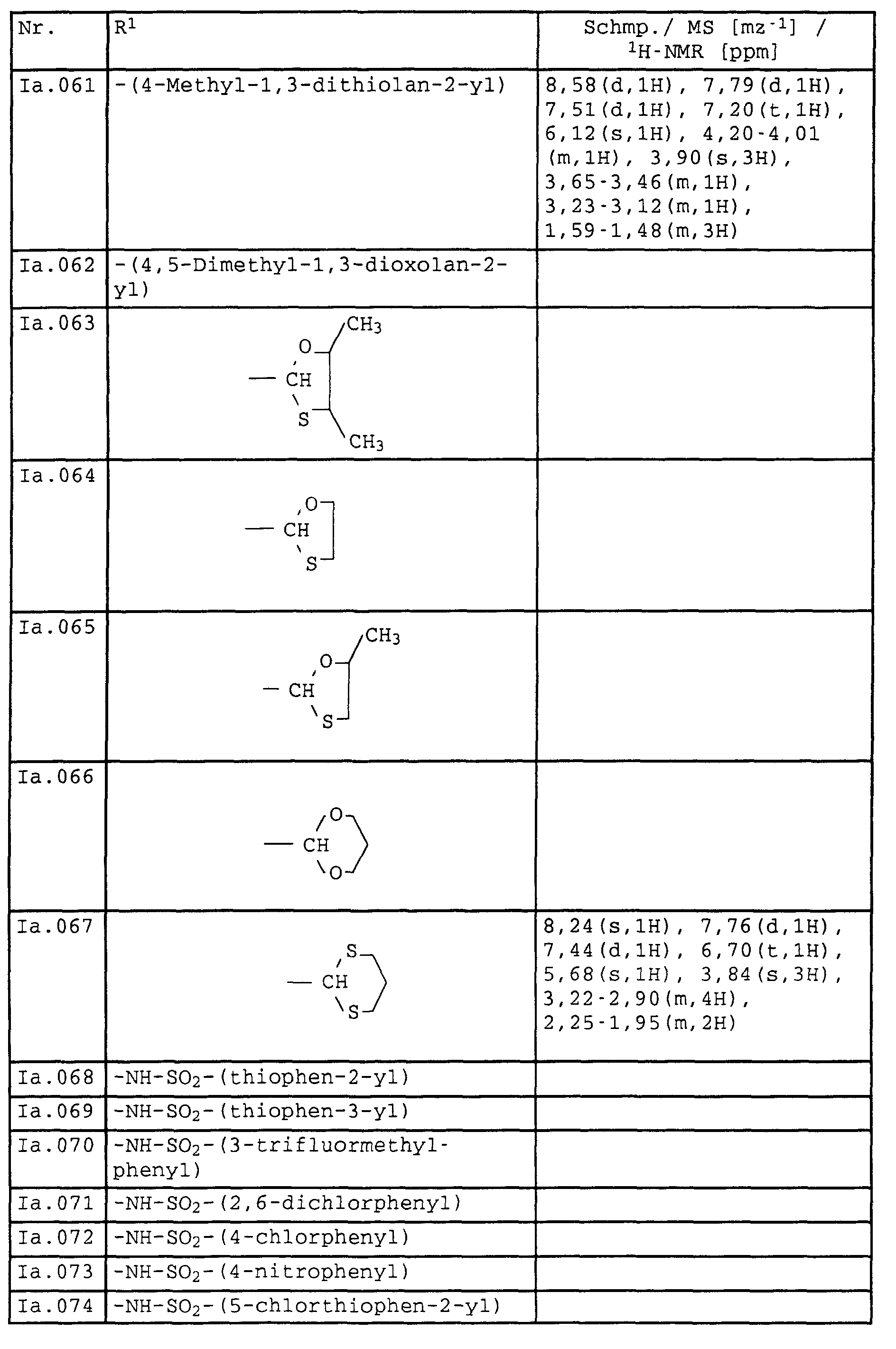

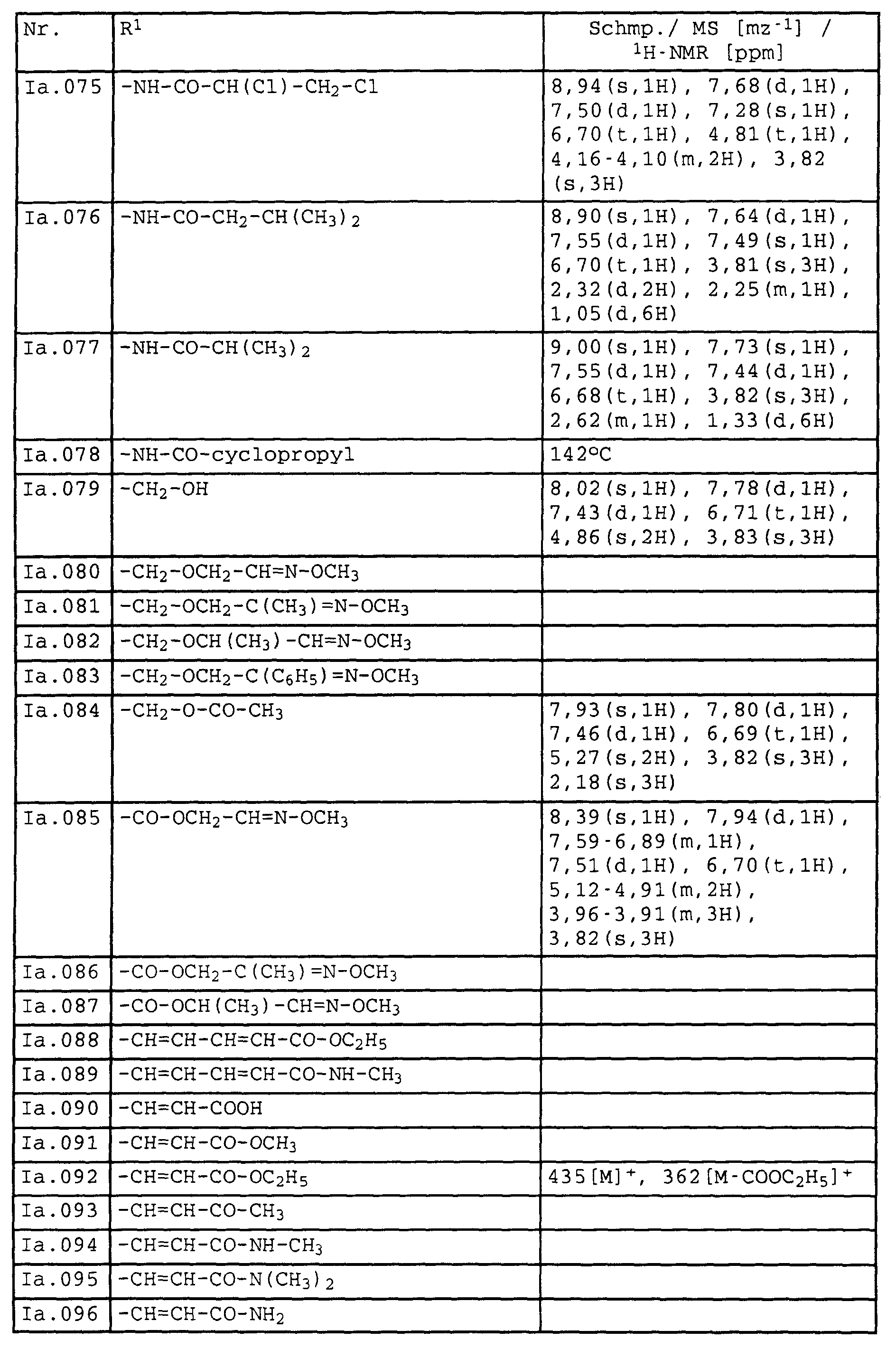

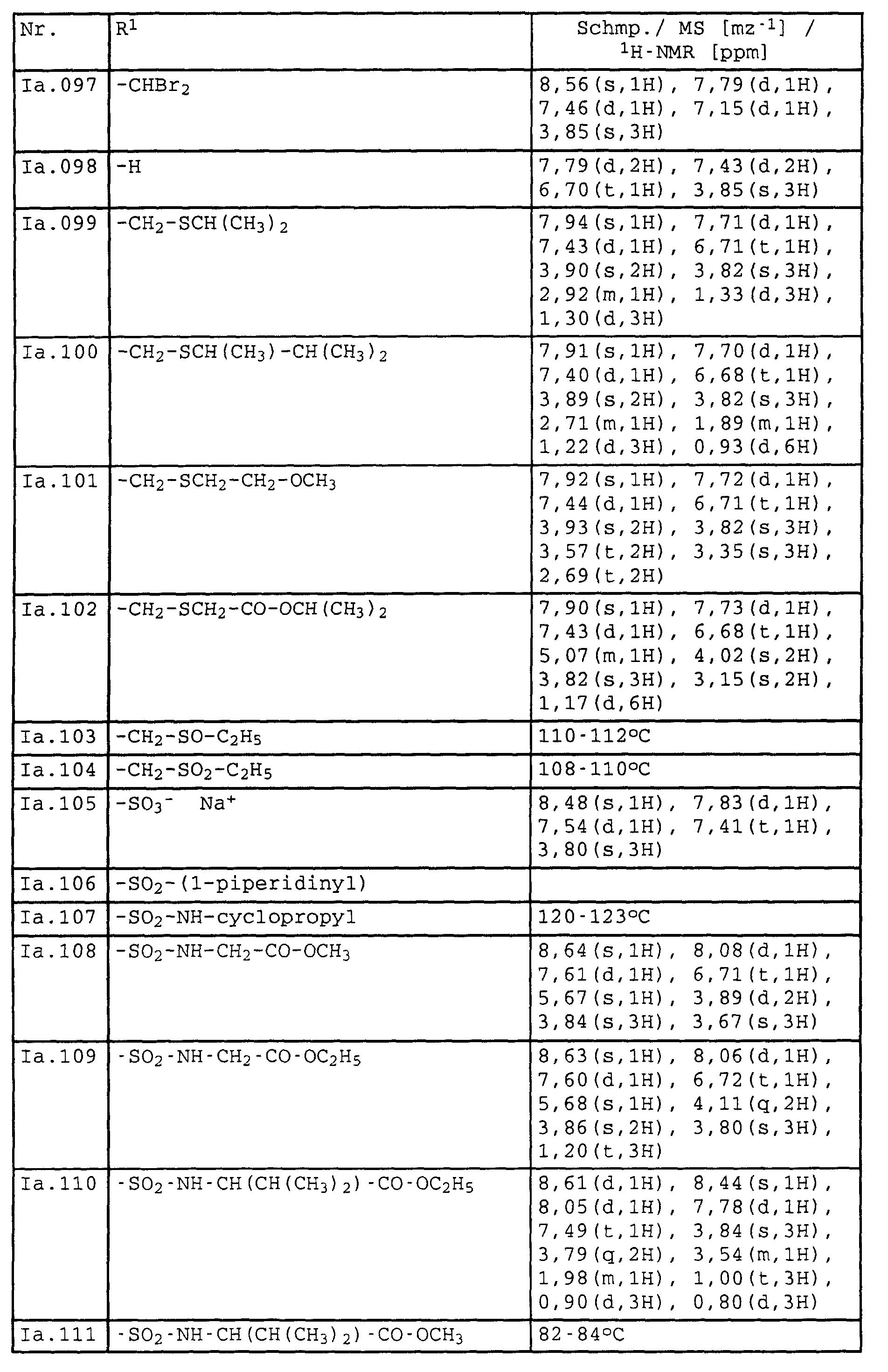

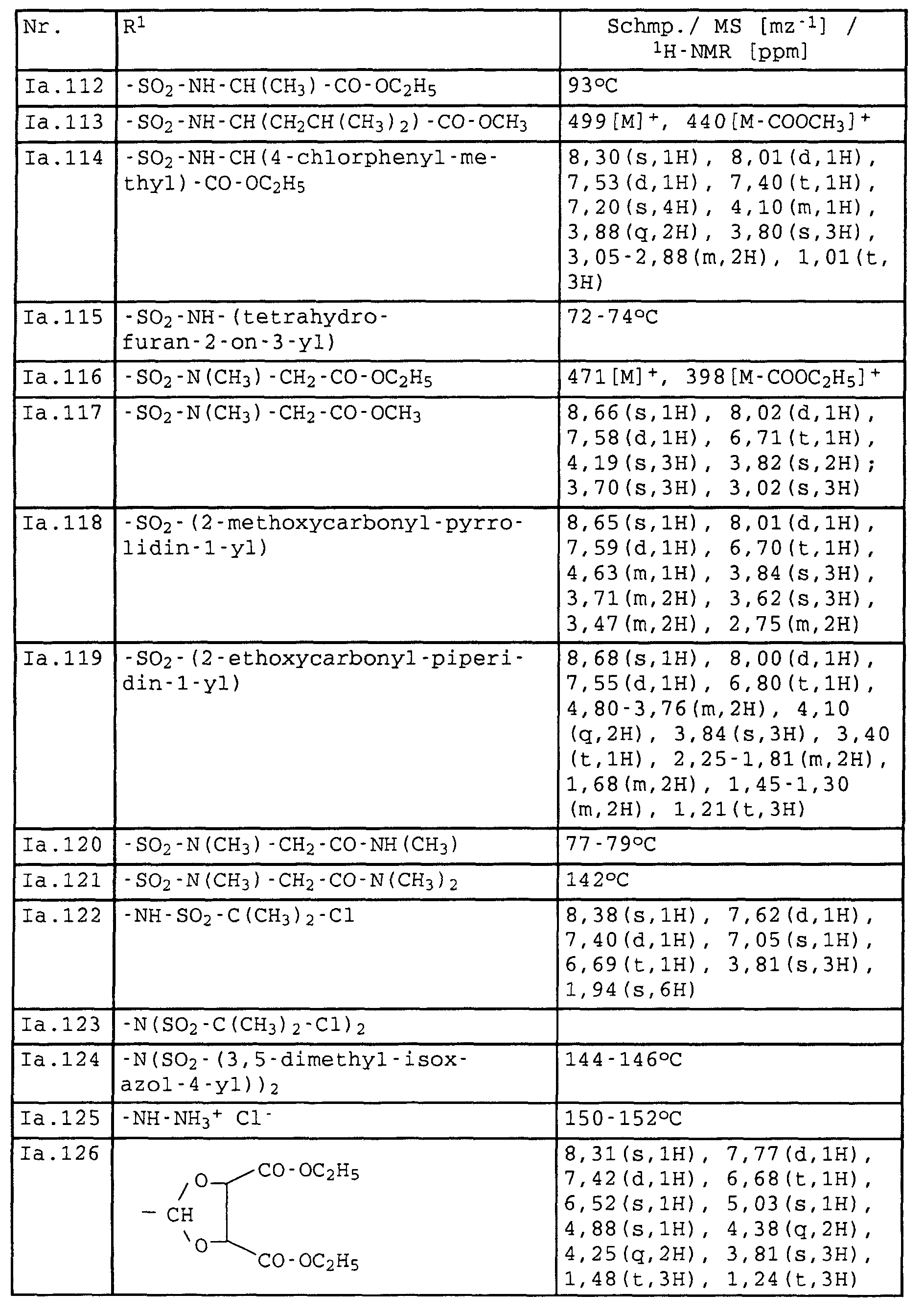

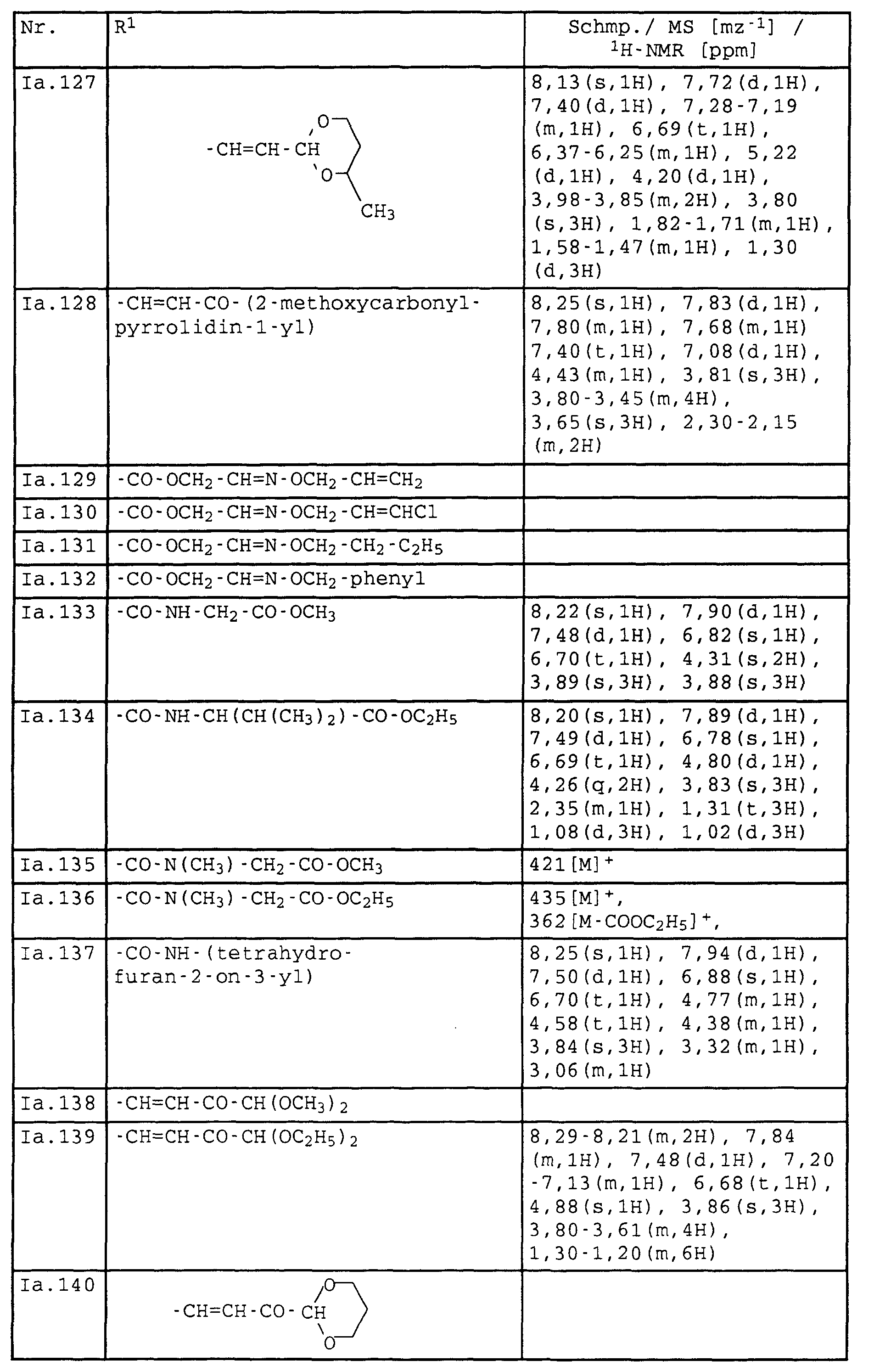

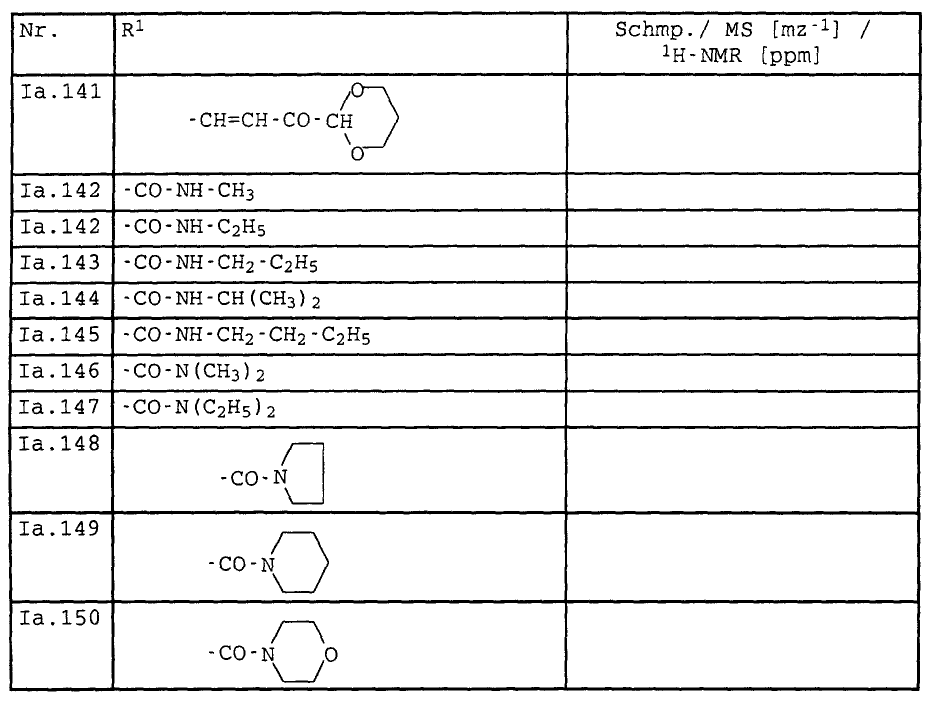

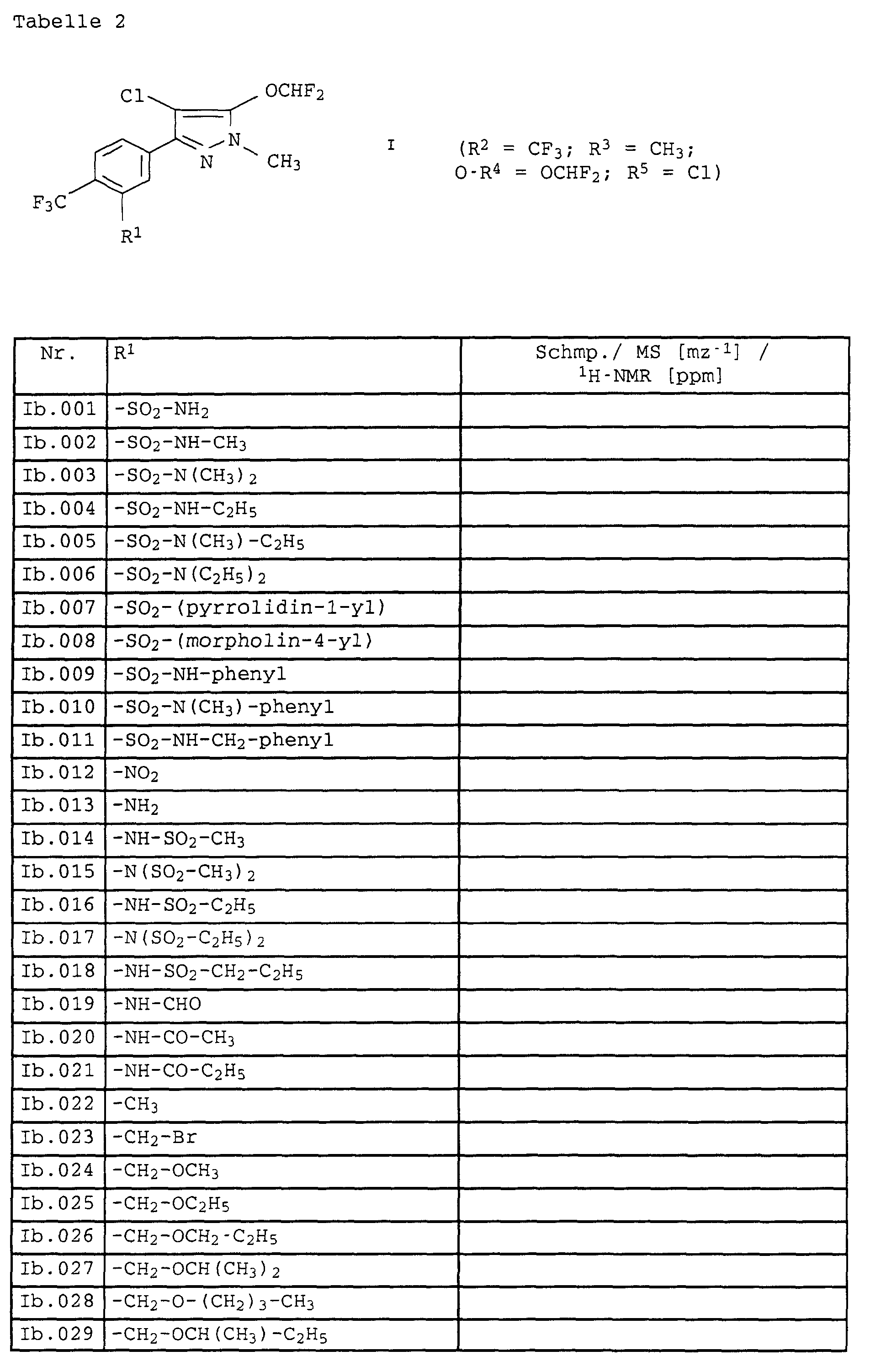

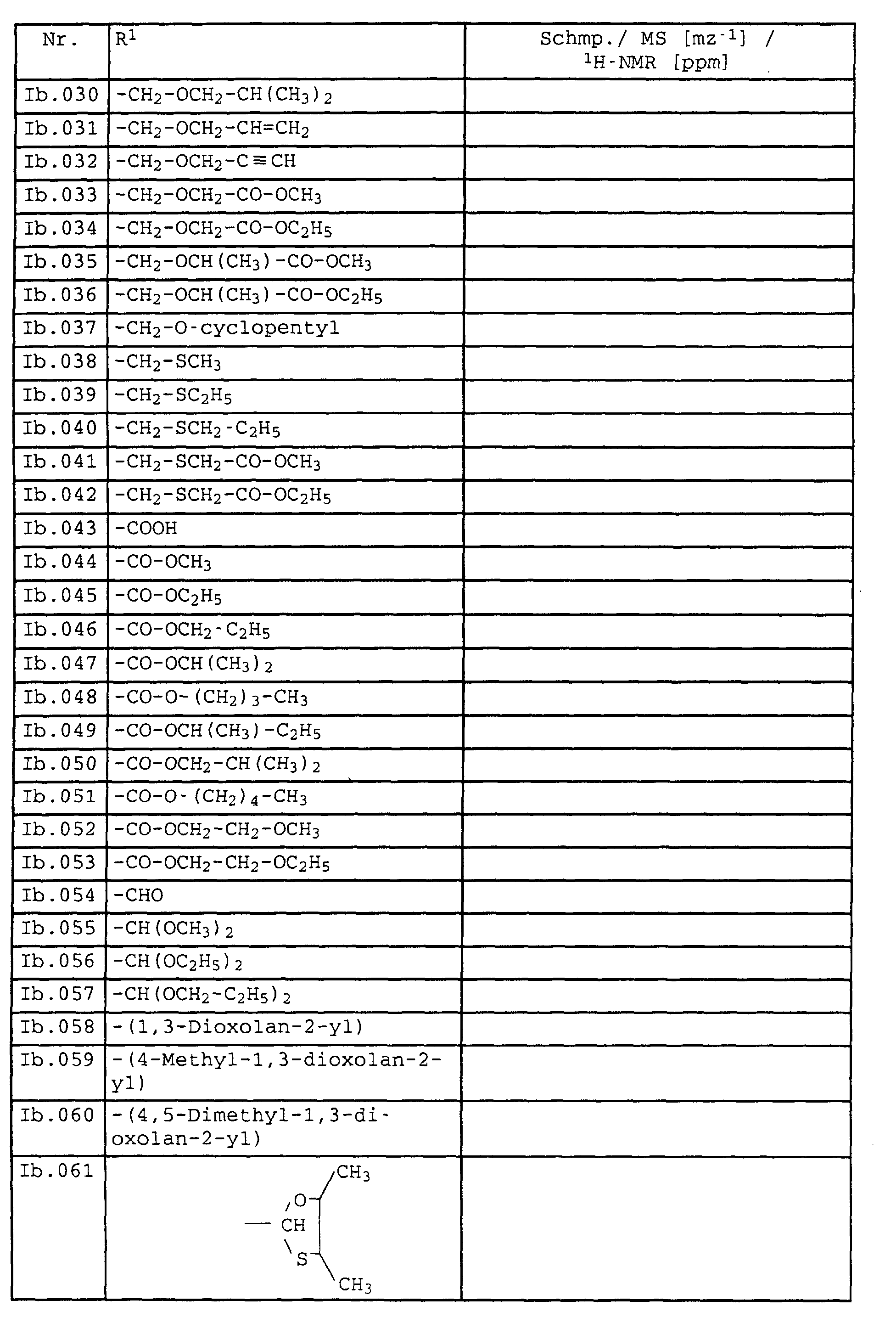

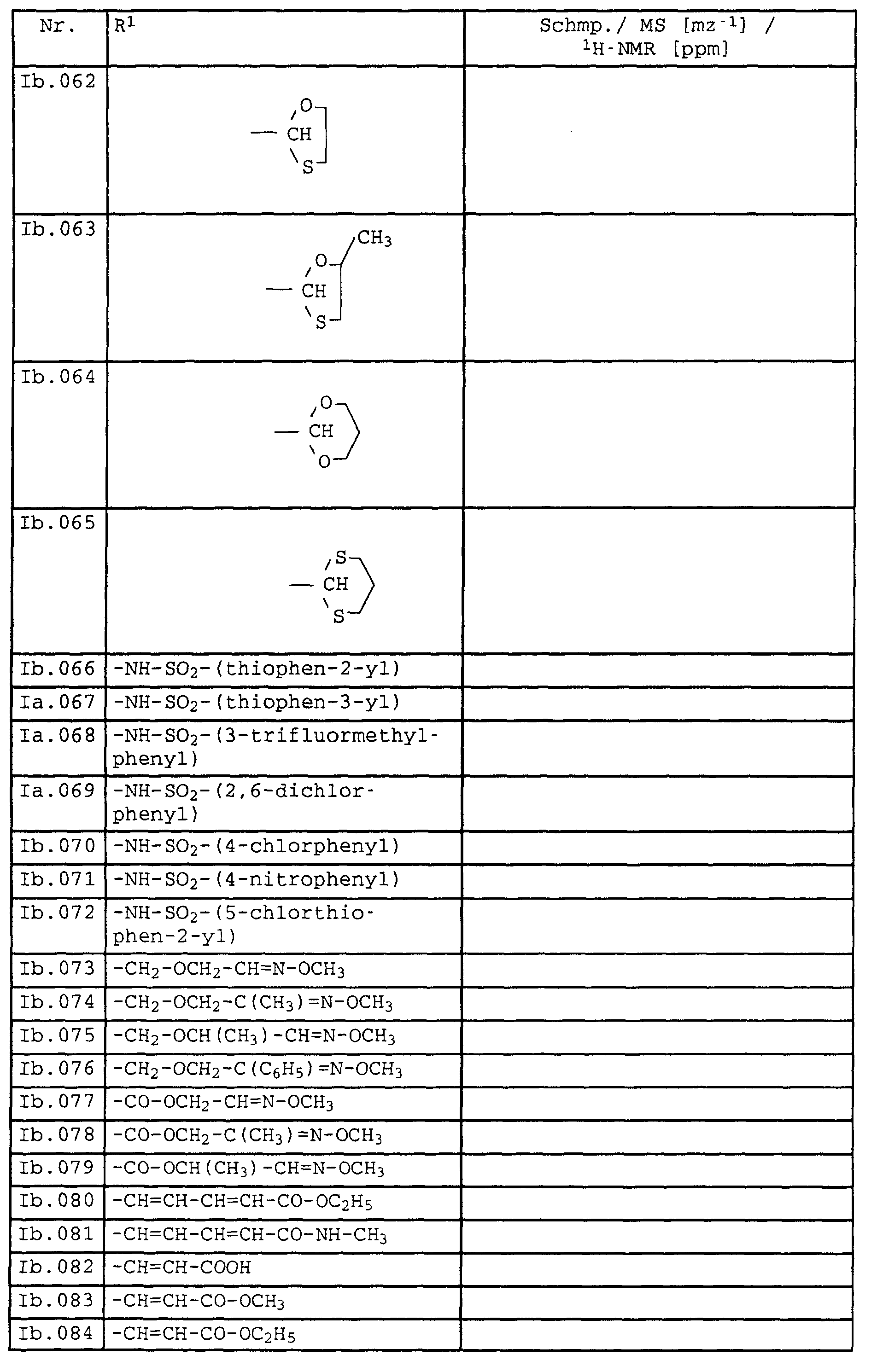

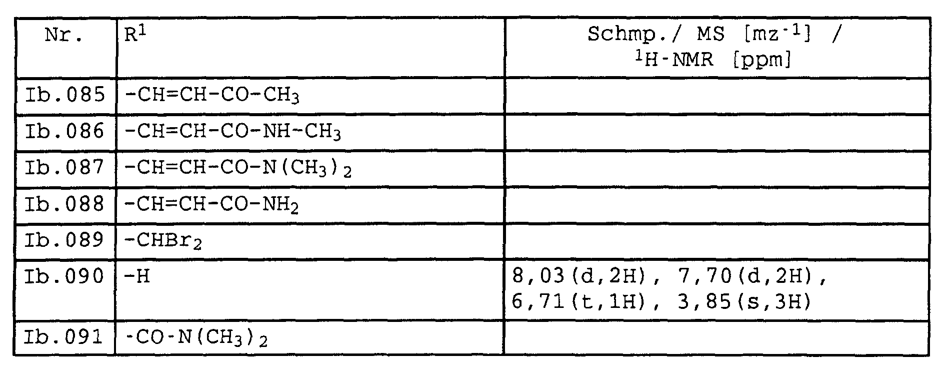

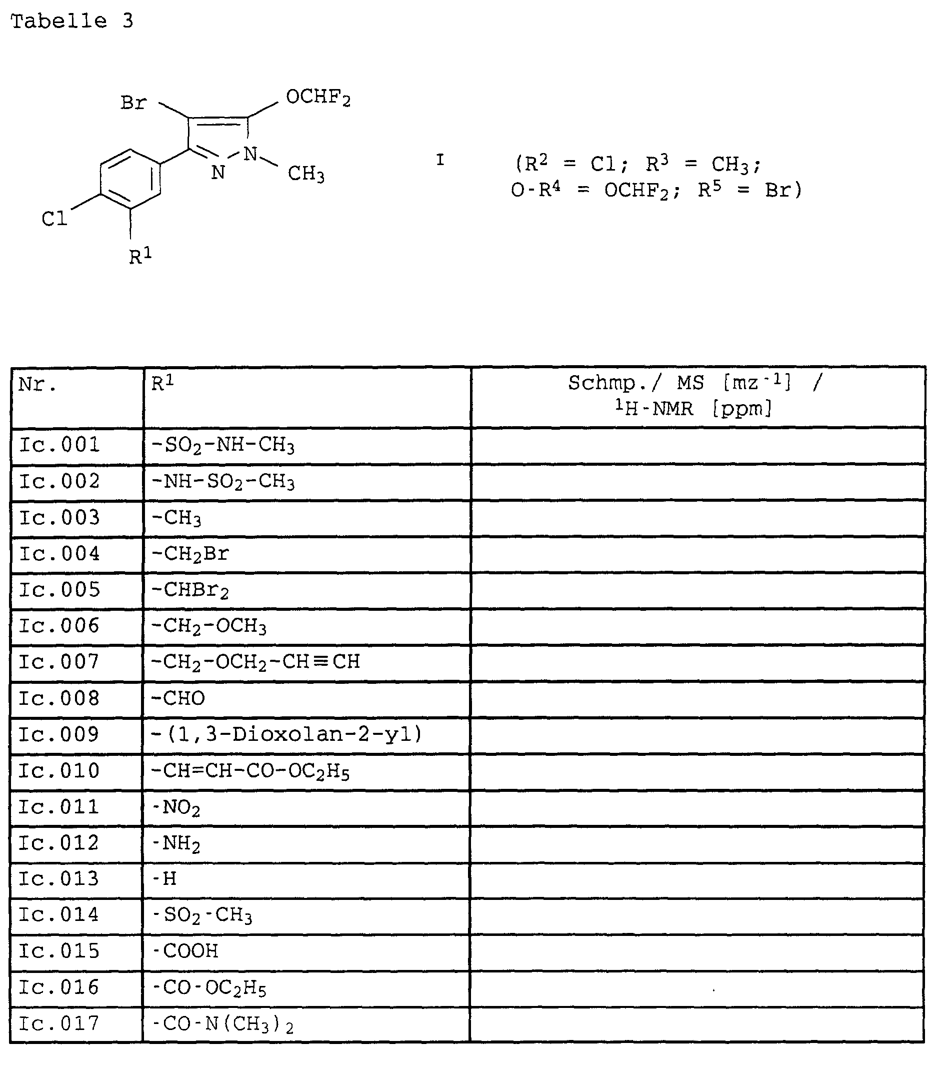

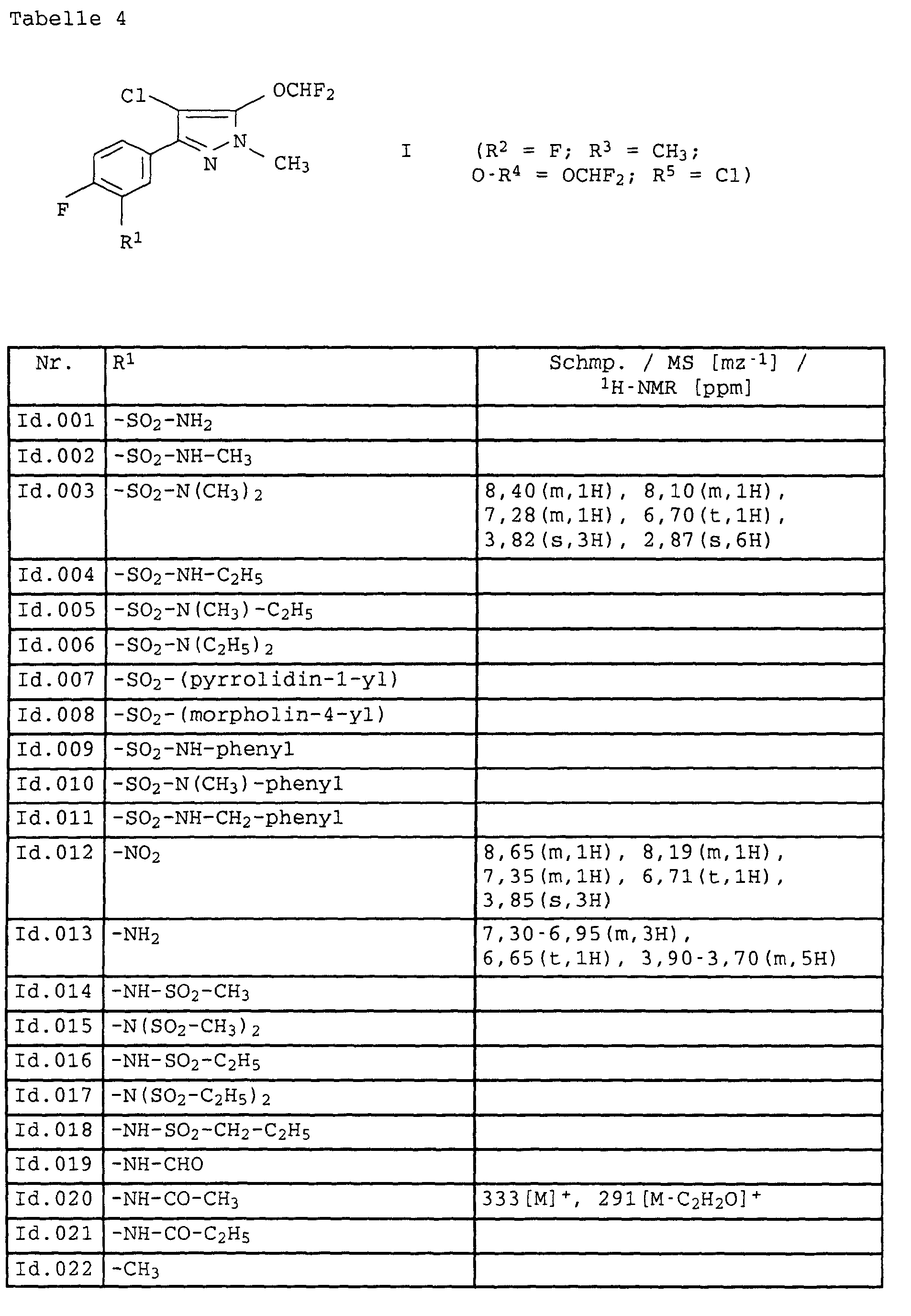

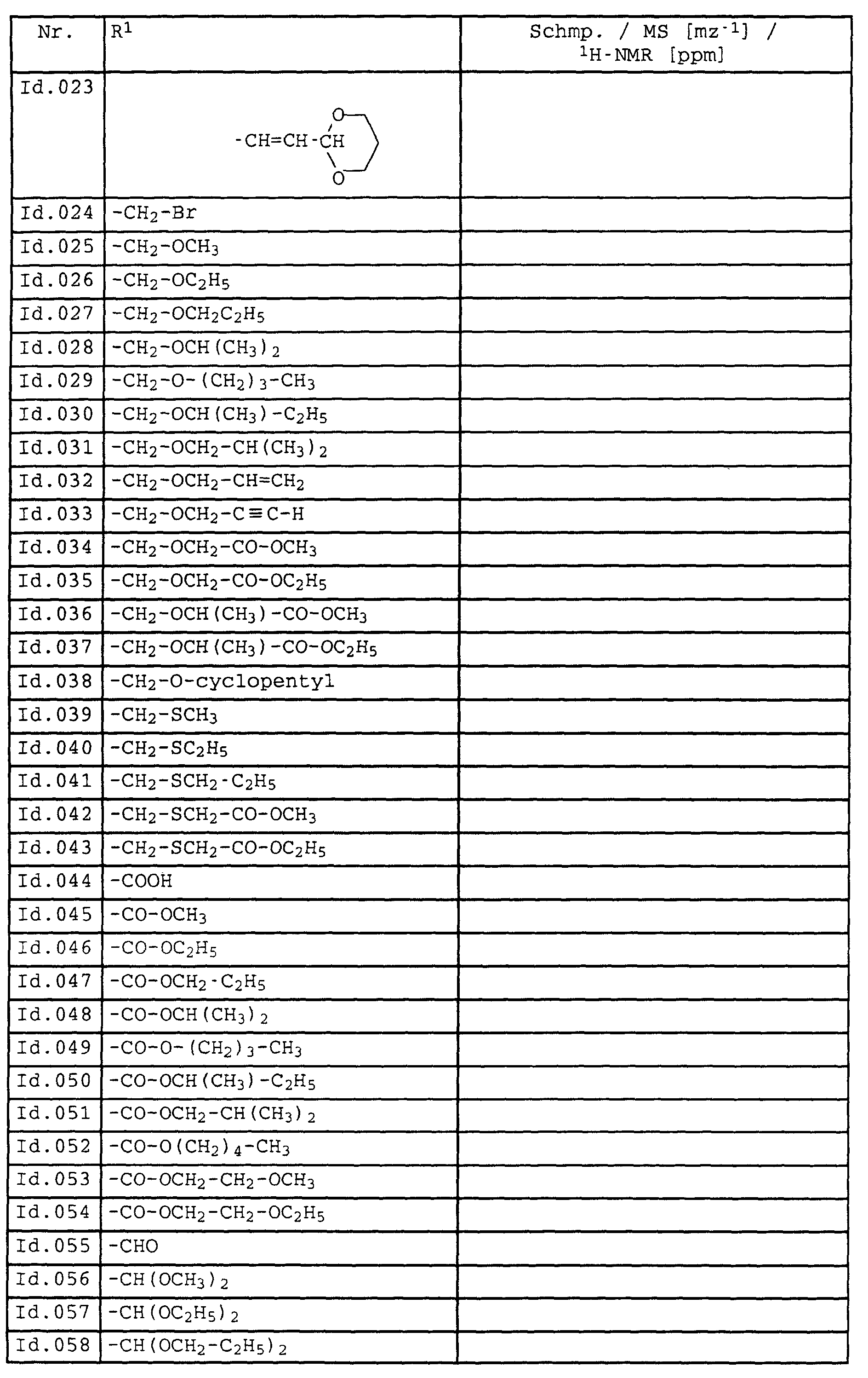

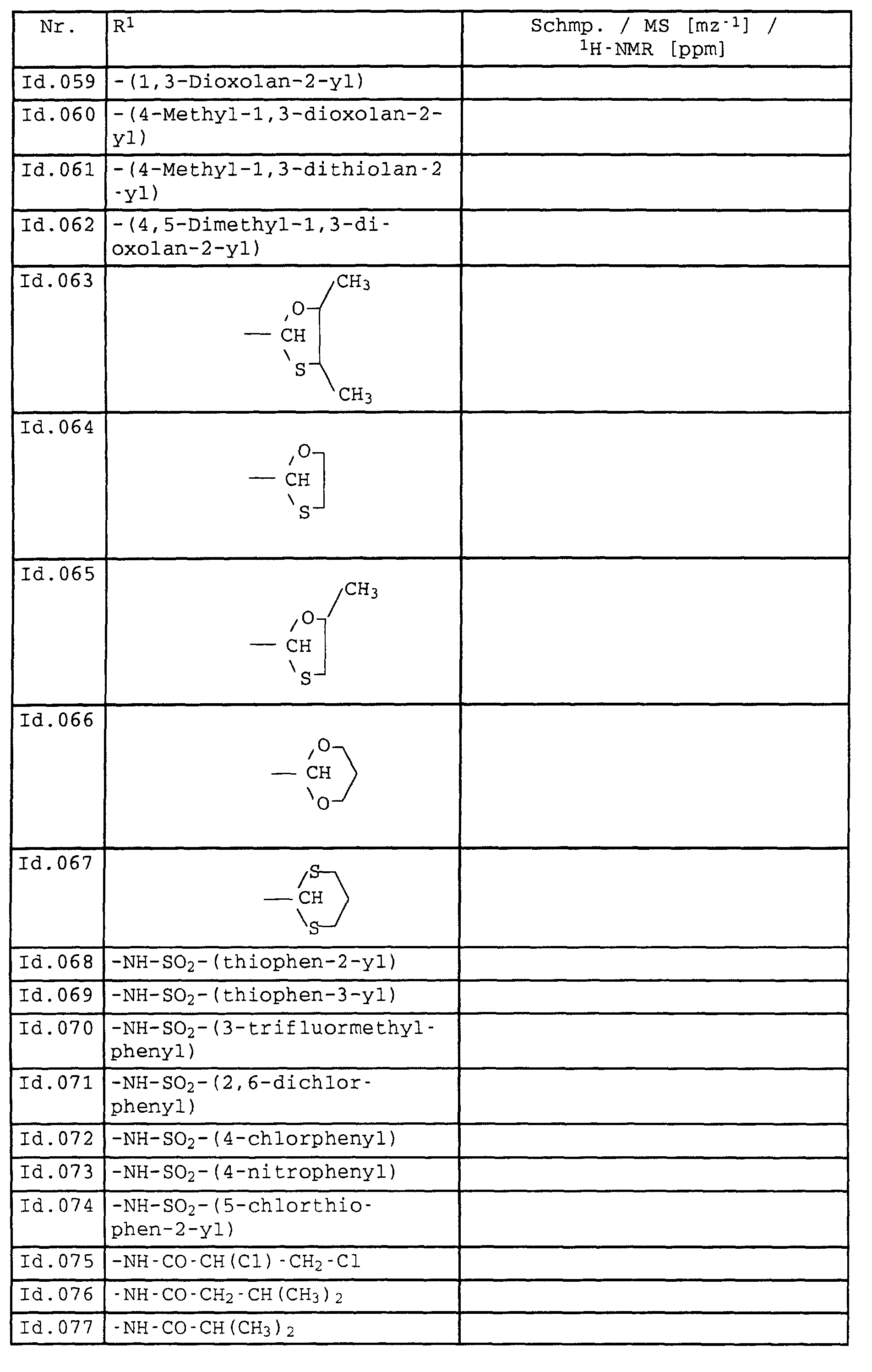

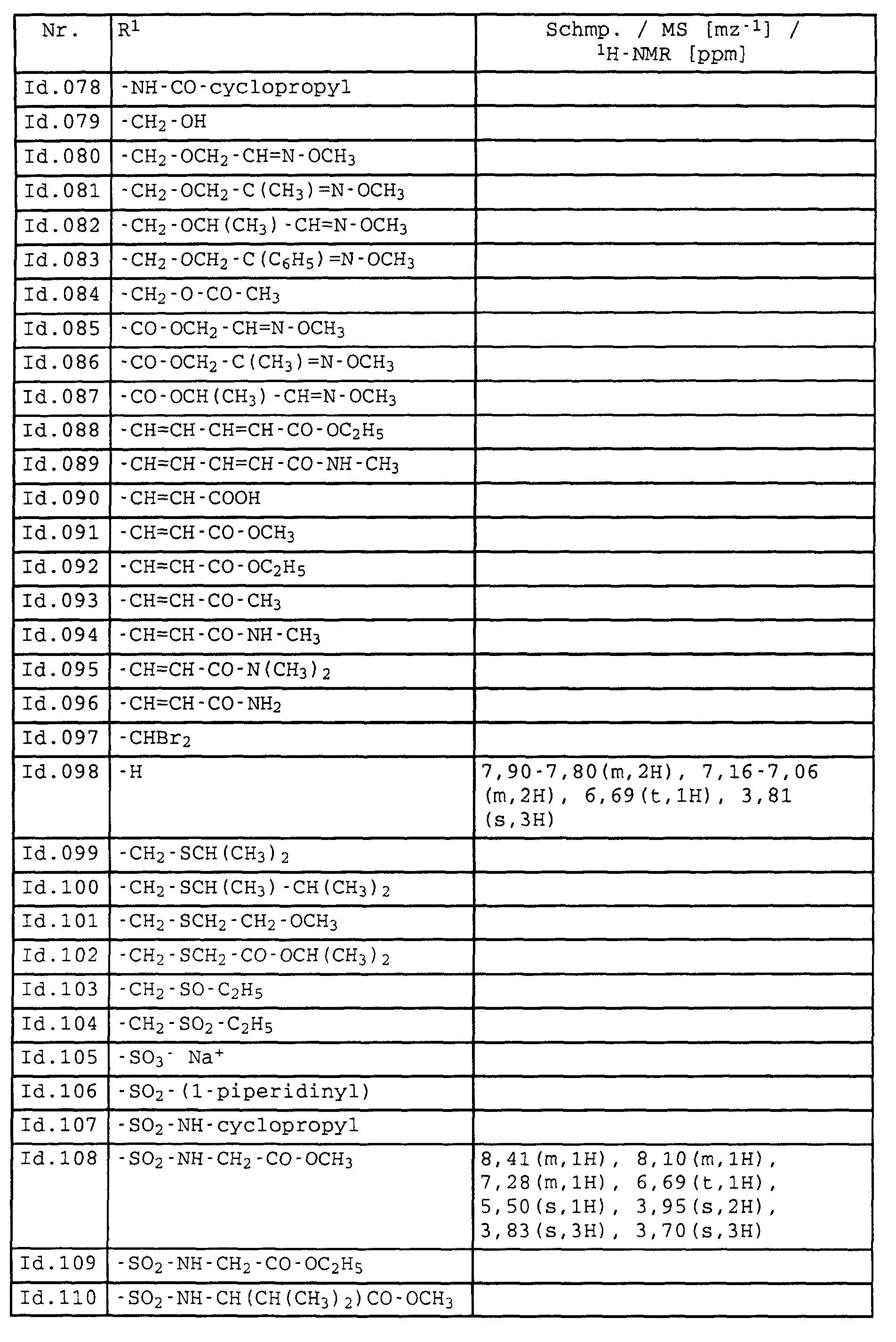

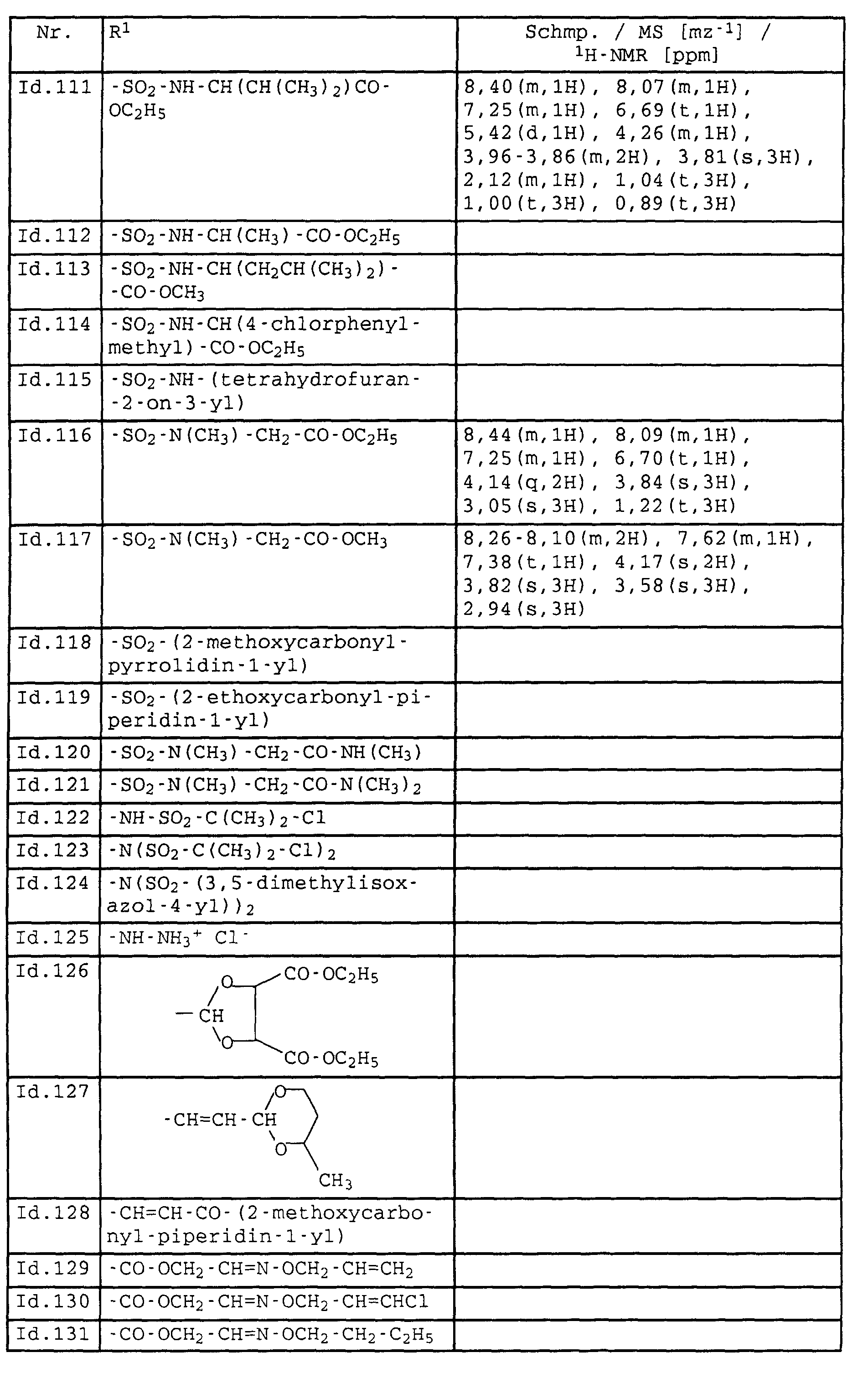

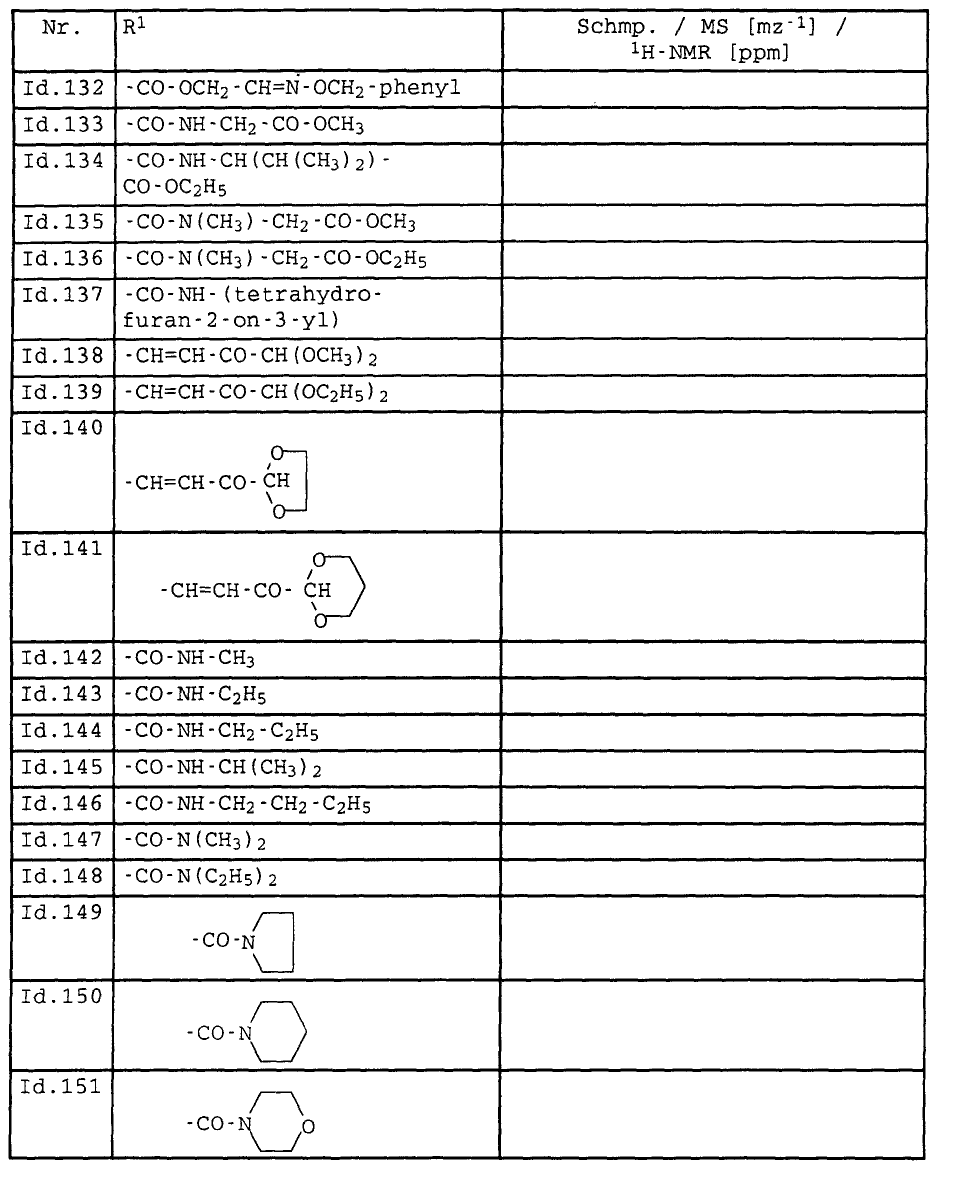

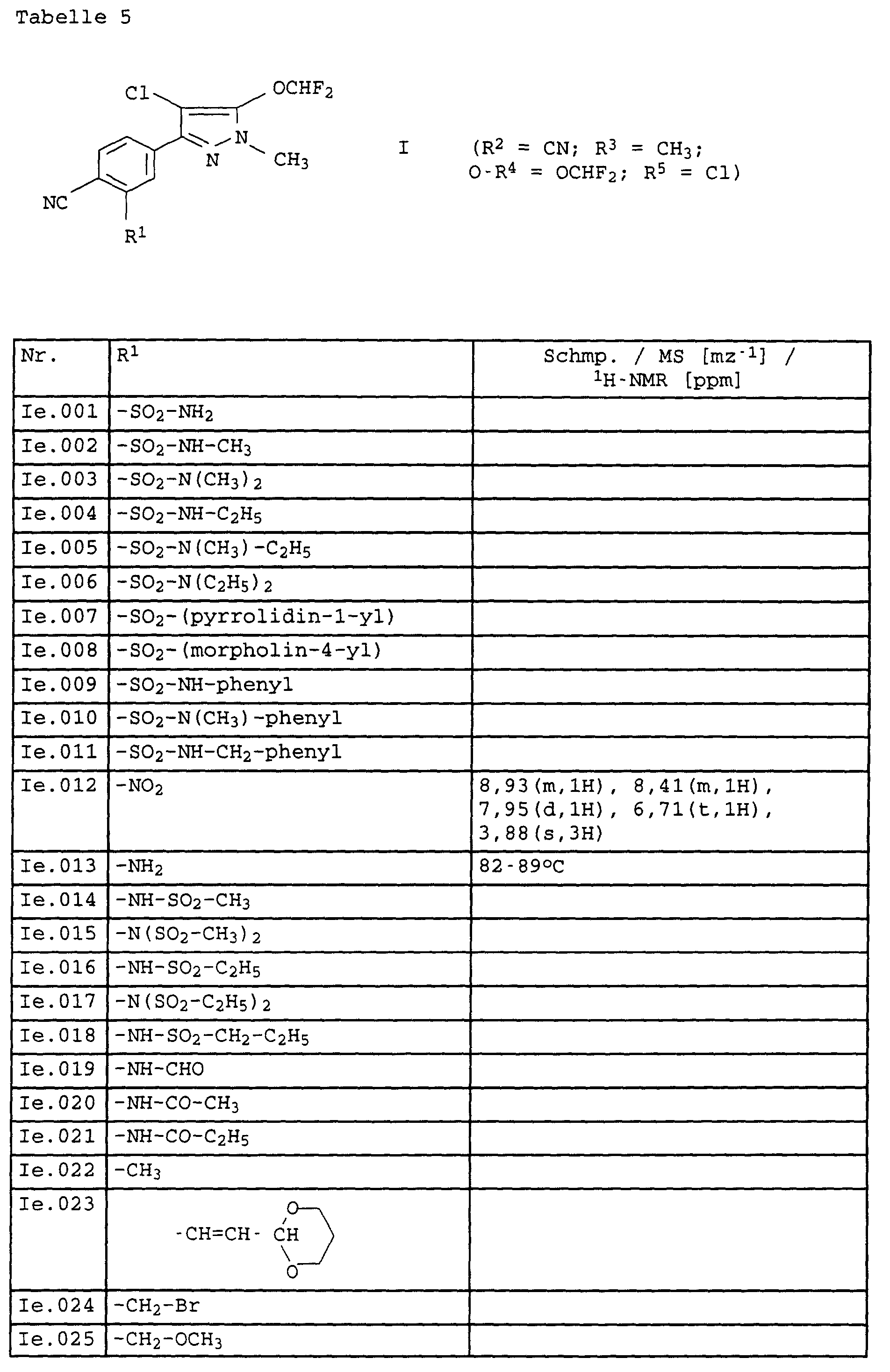

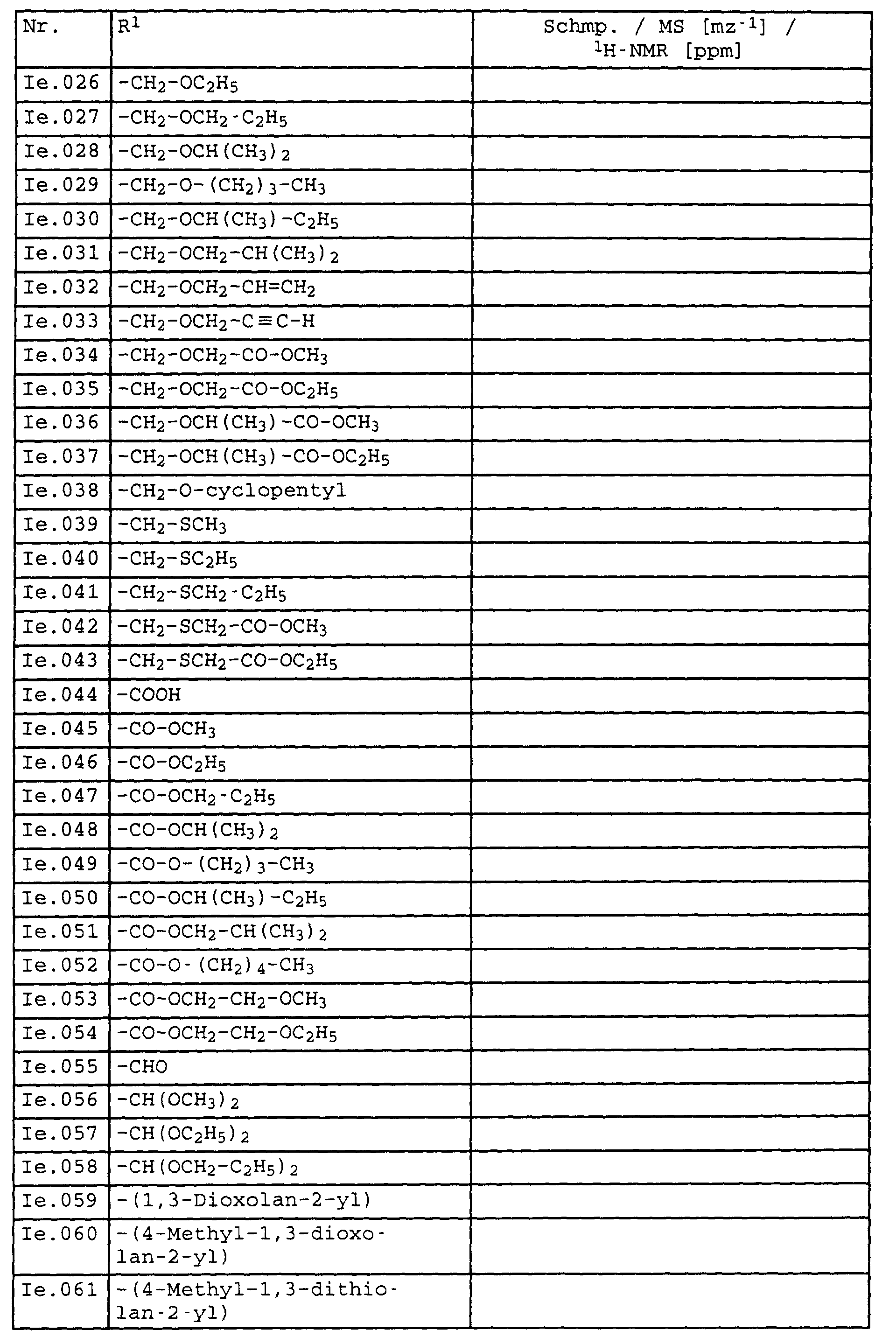

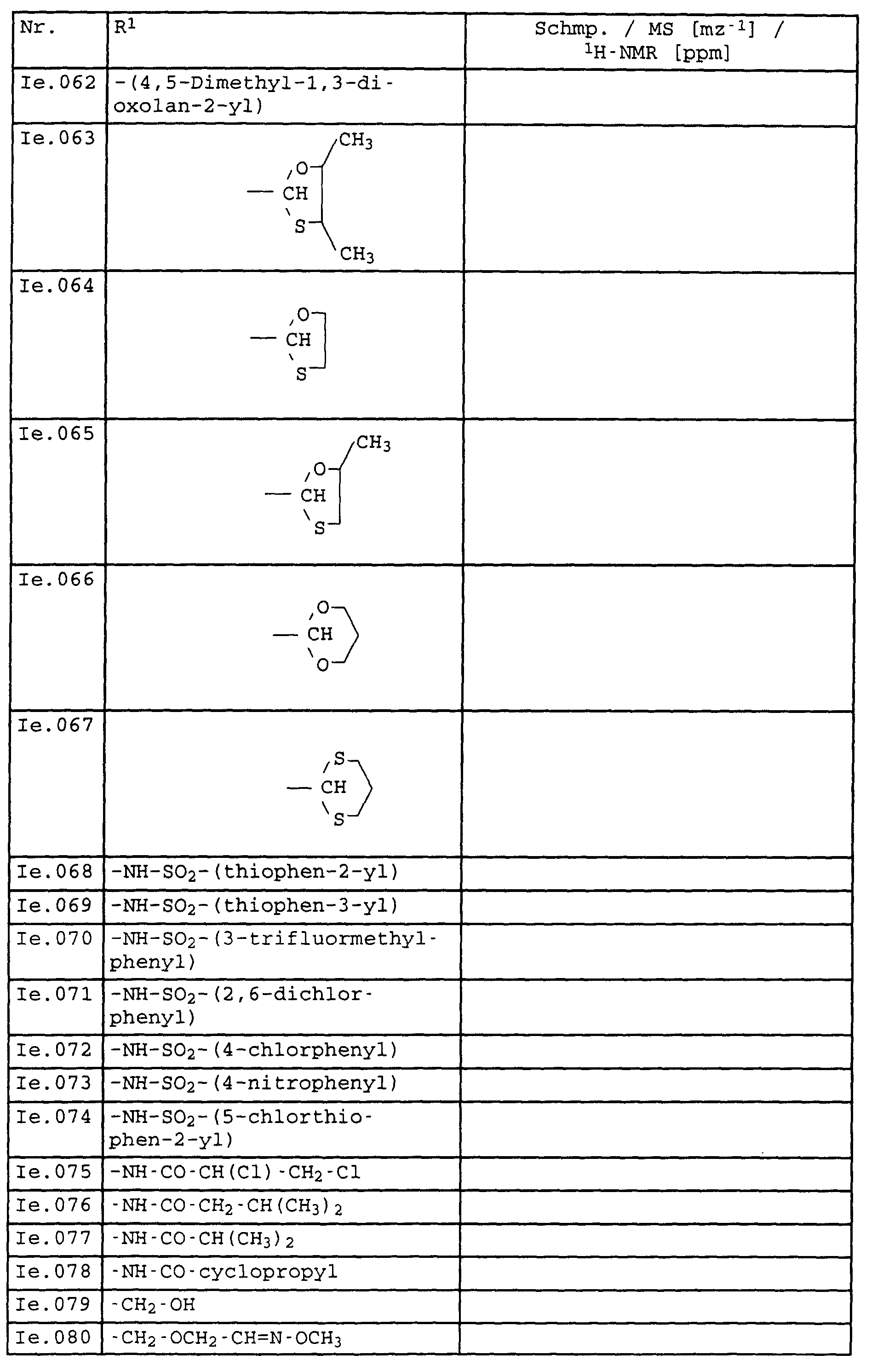

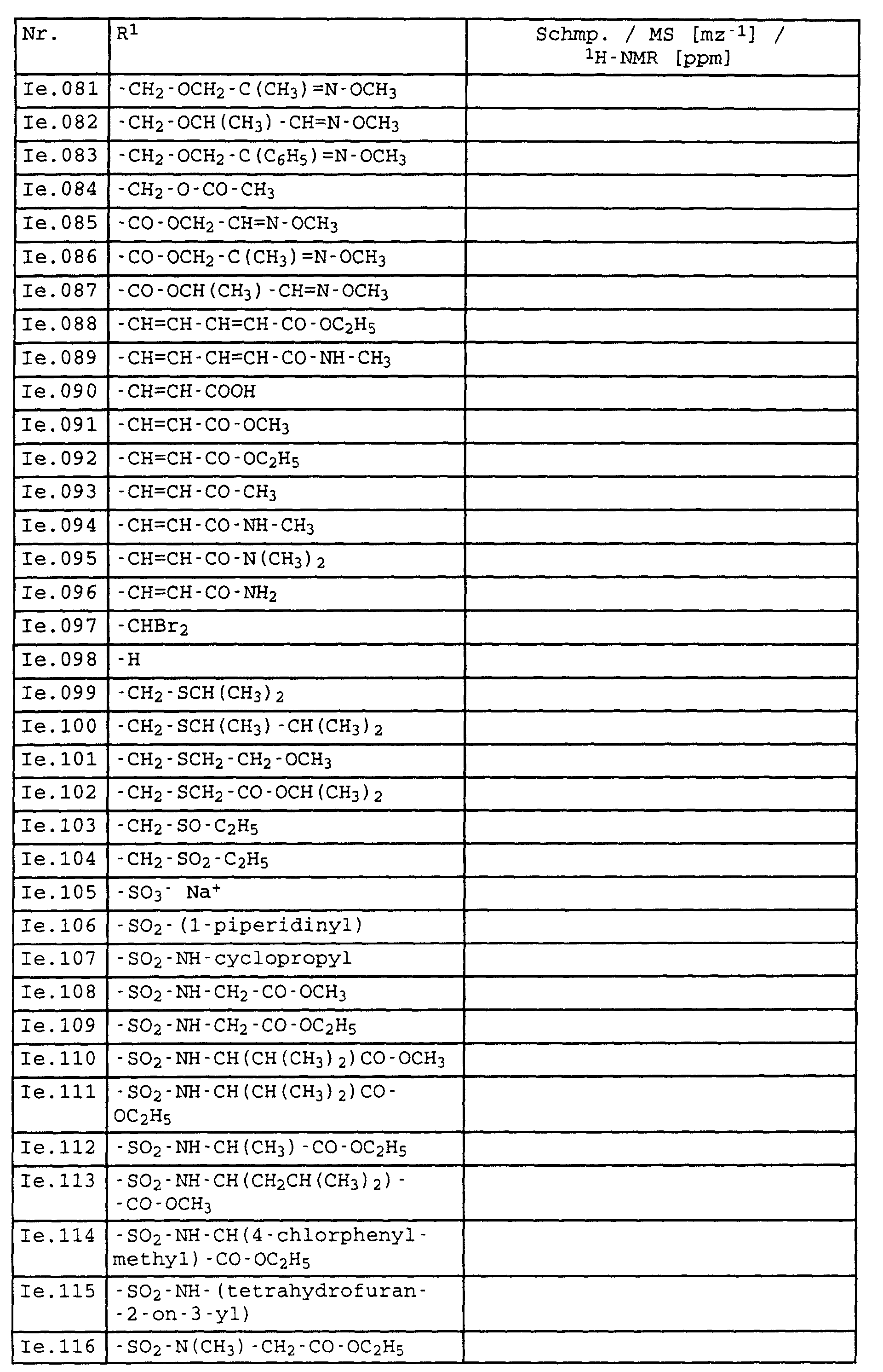

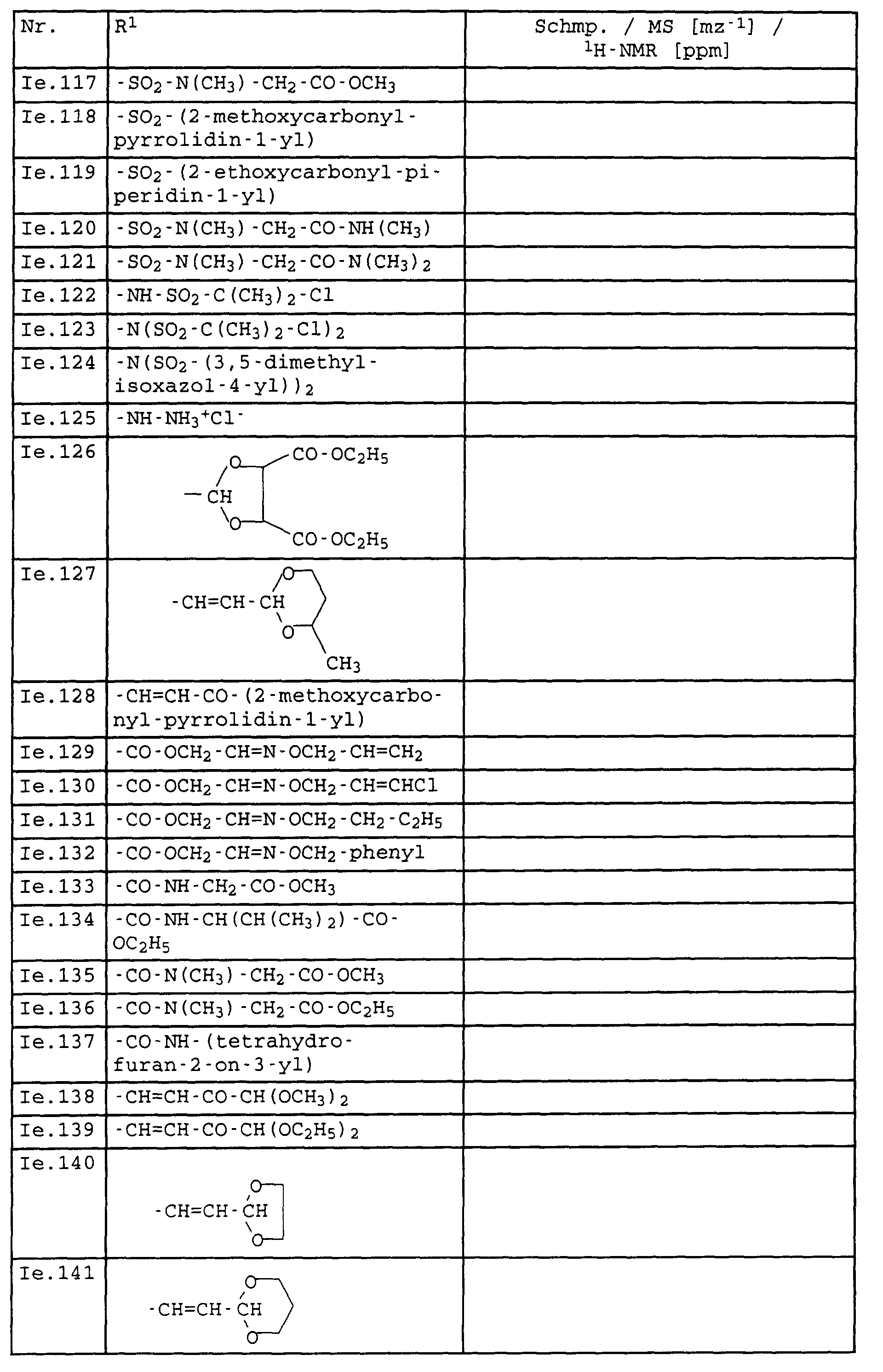

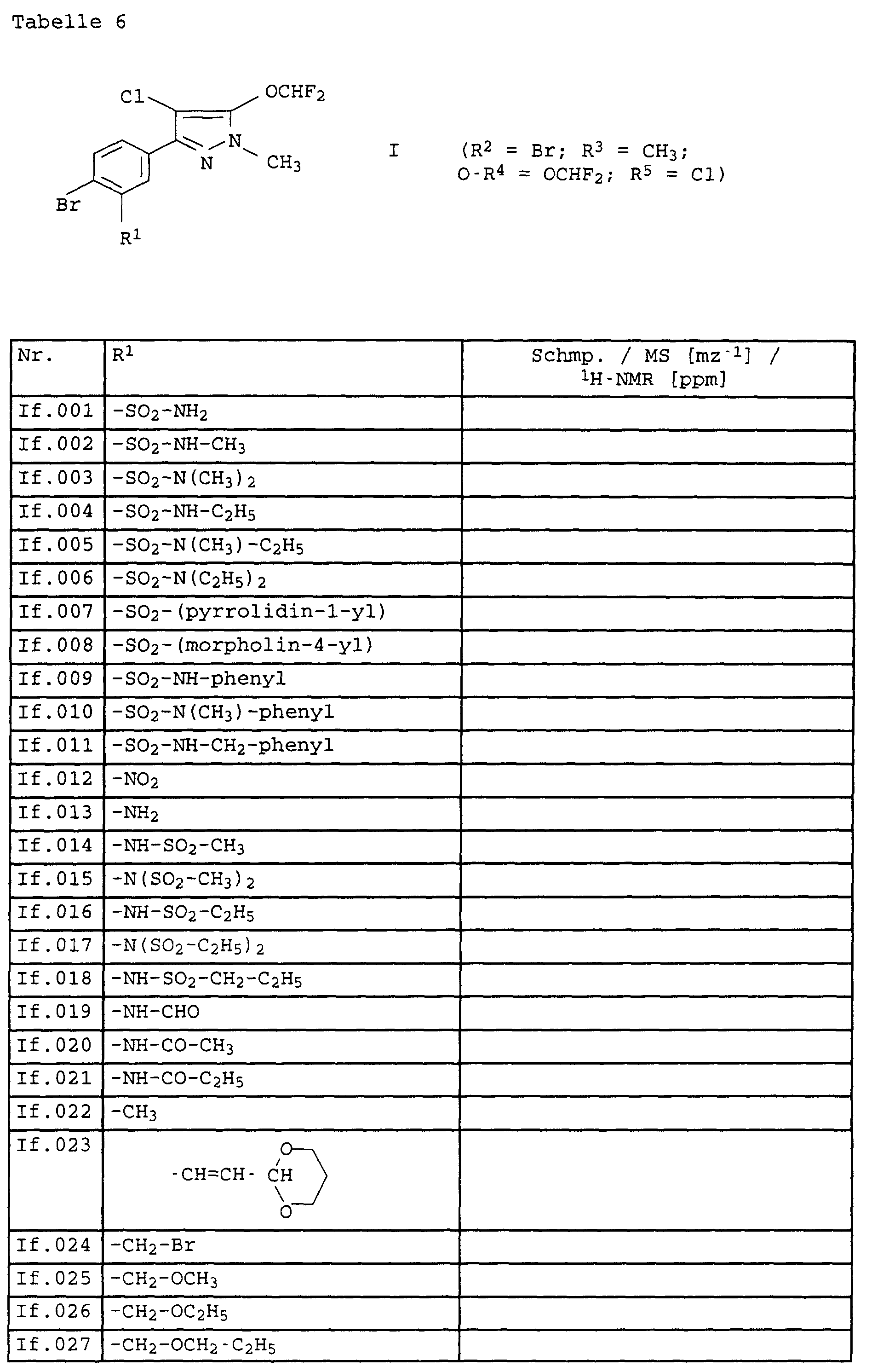

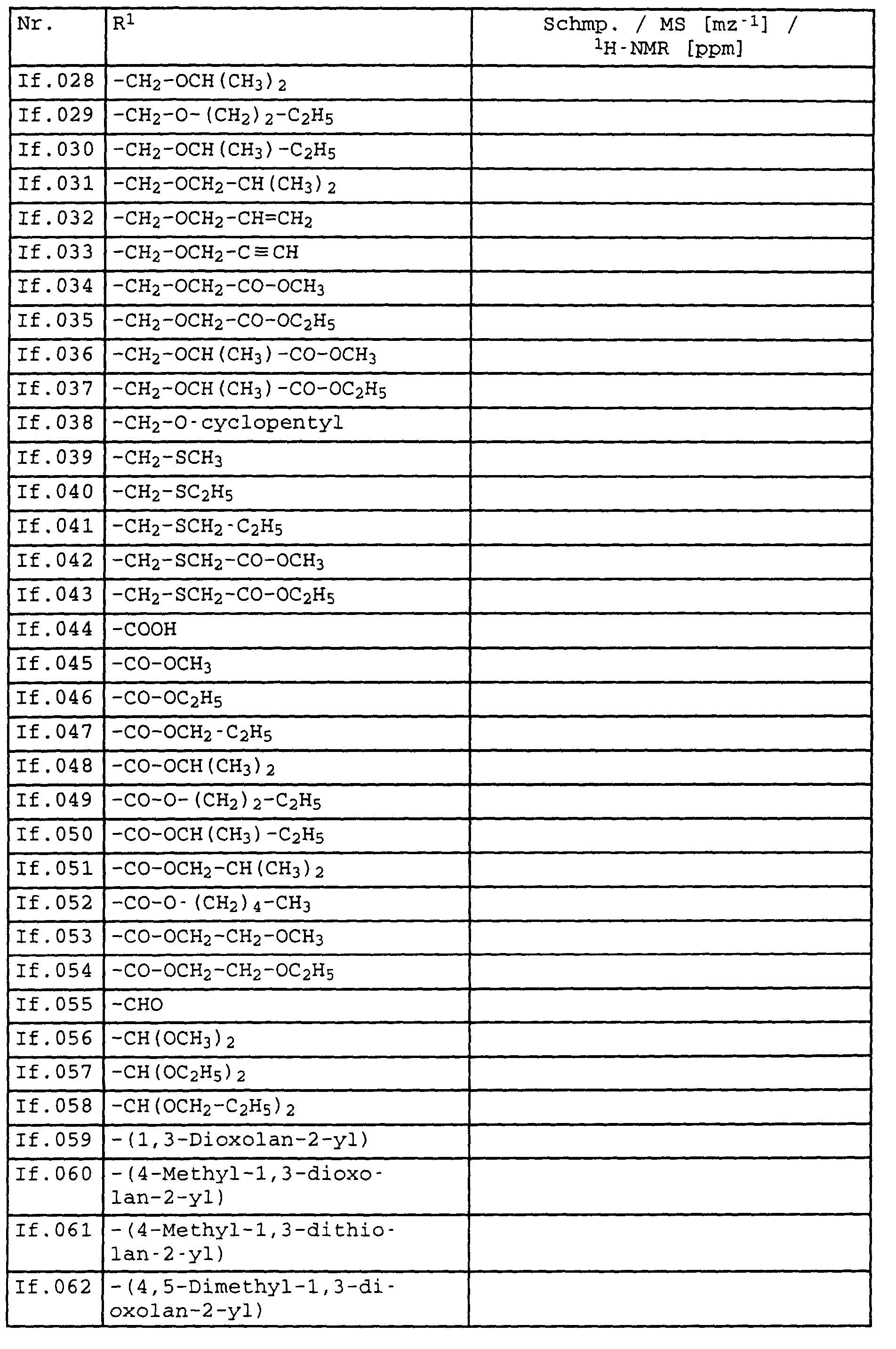

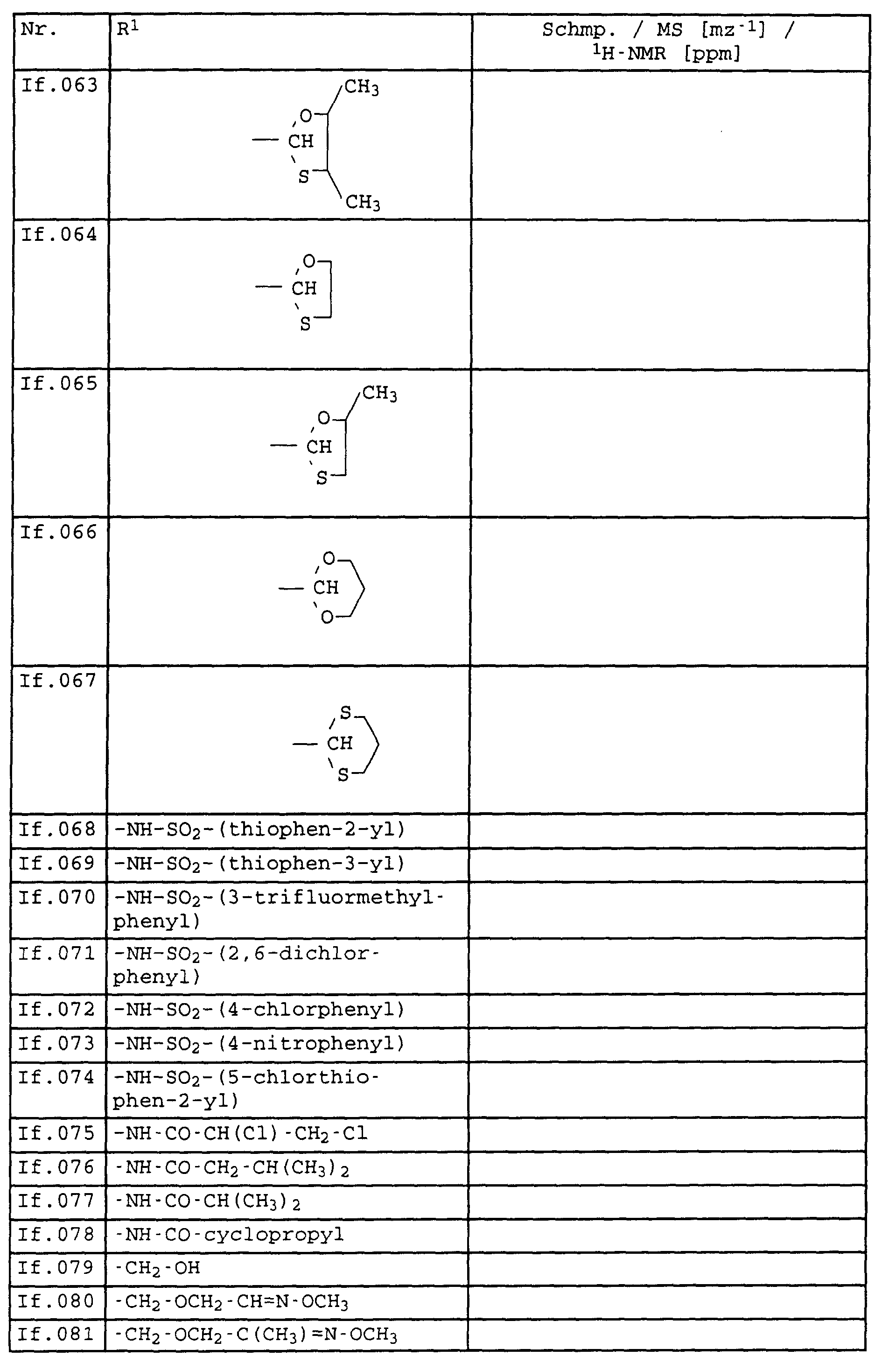

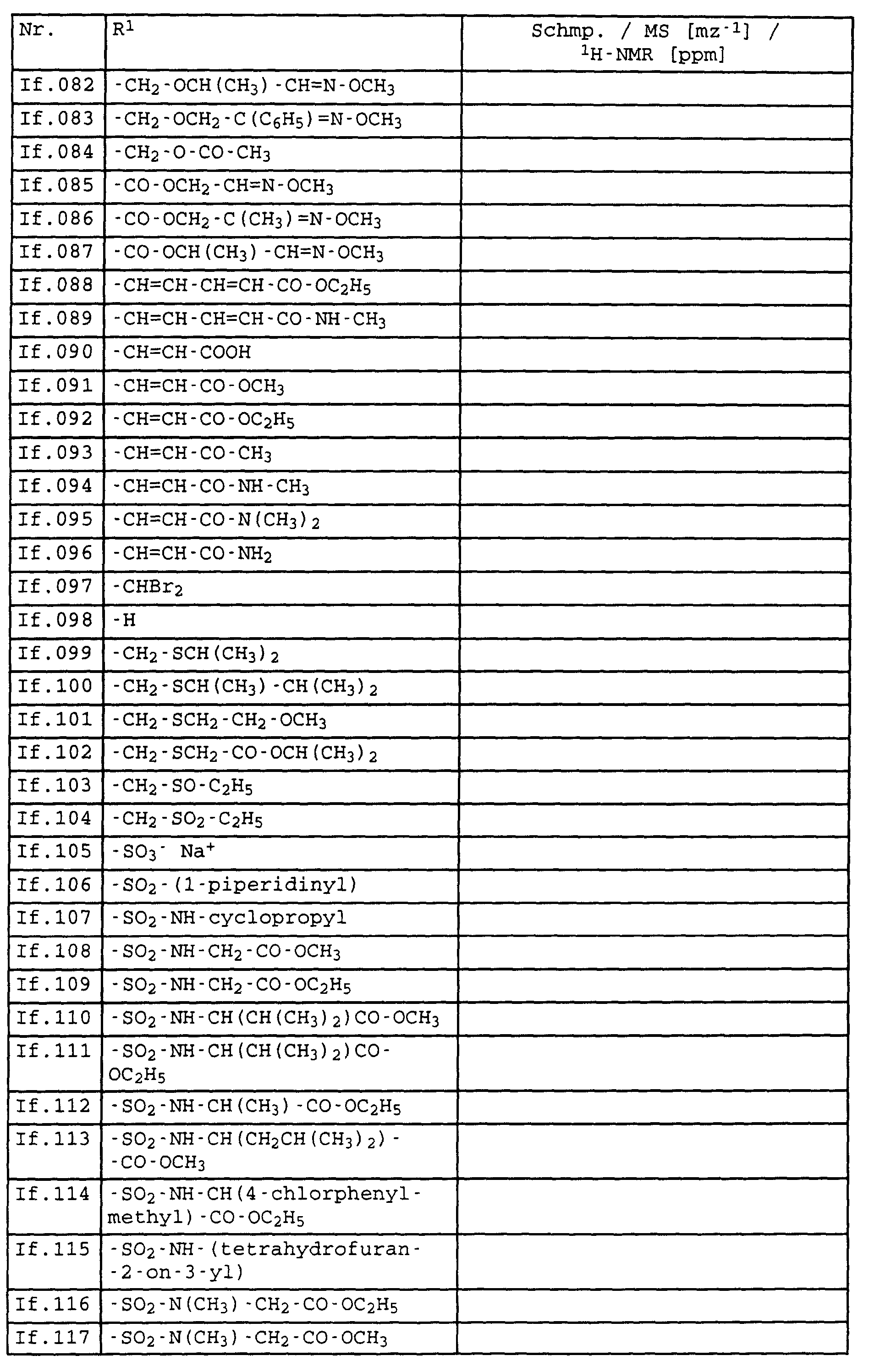

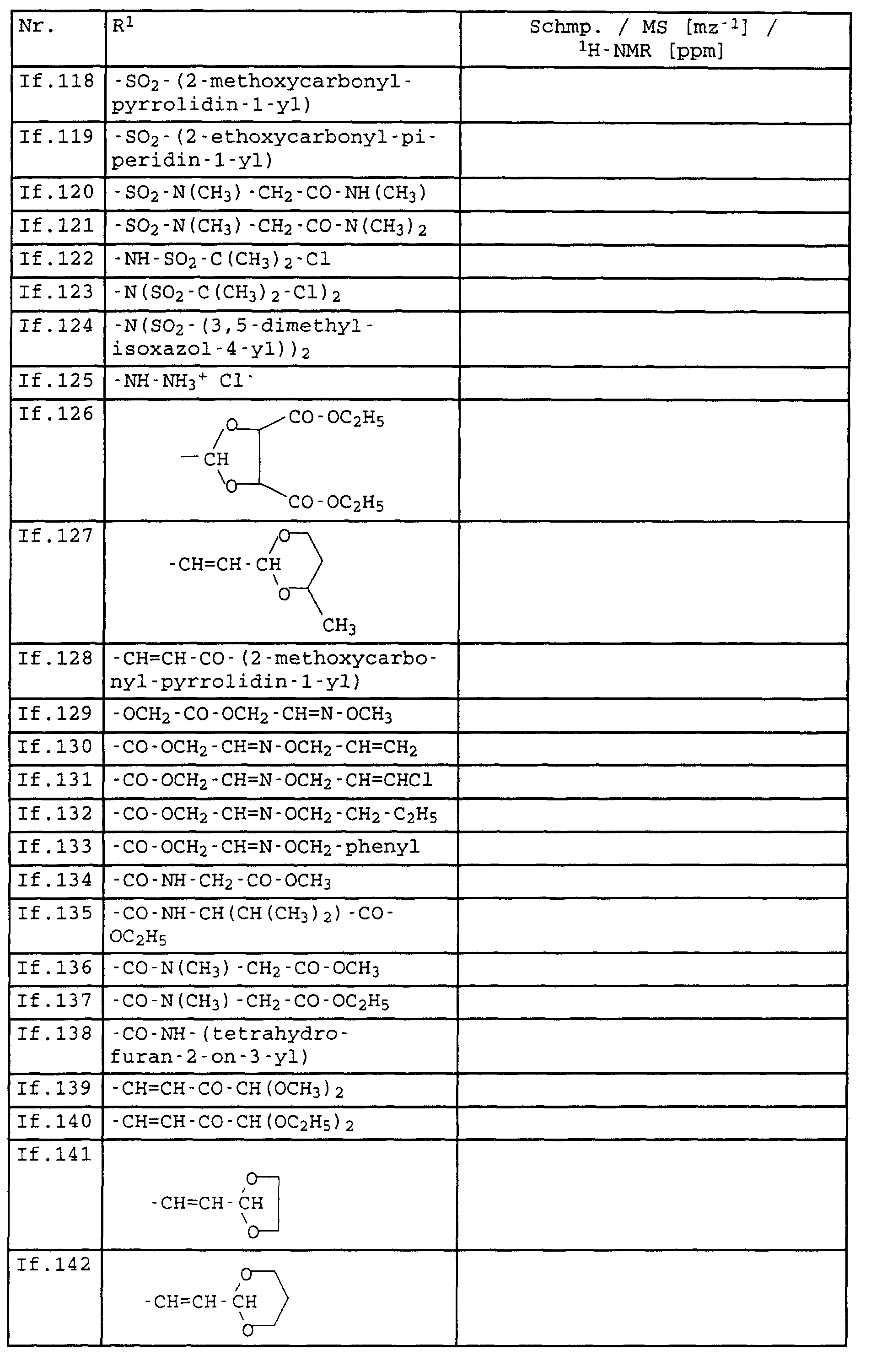

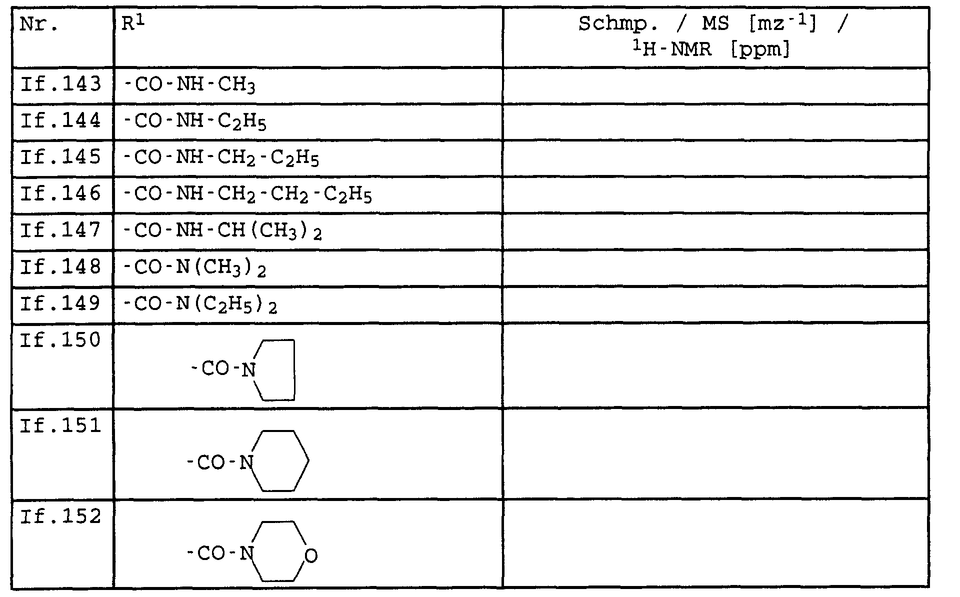

### Anwendungsbeispiele

Die herbizide Wirkung der substituierten 3-Phenylpyrazole der Formel I ließ sich durch Gewächshausversuche zeigen:

Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 3,0 kg aktive Substanz pro Hektar.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Echinochloa crus-galli | Hühnerhirse | Barnyardgrass |
| Galium aparine | Klettenlabkraut | Catchweed bedstraw |
| Ipomoea subspecies | Prunkwindearten | Morningglory |
| Setaria italica | Kolbenhirse | Foxtail millet |

Bei einer Aufwandmenge von 3,0 kg/ha a.S. ließen sich mit der Verbindung Nr. Ia.022 unerwünschte Pflanzen im Nachauflaufverfahren sehr gut bekämpfen.

## Patentansprüche

1. Substituierte 3-Phenylpyrazole der Formel I 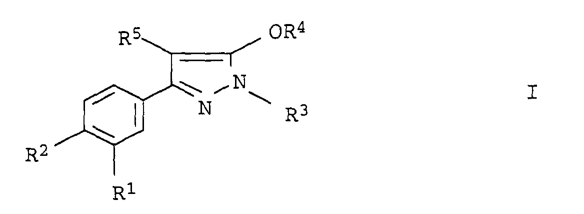 in der die Variablen folgende Bedeutung haben:
R¹ Wasserstoff, Nitro, C₁-C₈-Alkyl, C₁-C₈-Halogenalkyl, C₁-C₈-Alkyl-O-R⁶, C₁-C₈-Alkyl-O-CO-R⁶, C₁-C₈-Alkyl-S-R⁶, C₁-C₈-Alkyl-SO-R⁶, C₁-C₈-Alkyl-SO₂-R⁶, -SO₂-R⁶, -SO₂-O-R⁶, -SO₂-N(R⁷,R⁸), -N(R⁷,R⁸), -N(R⁷)-N(R⁸,R³²), -N=N-CO-R⁹, -N(R⁷)-N(R⁸)-CO-R⁹, -N(R¹⁰)-CO-R⁹, -N(R¹⁰)-SO₂-R¹¹, -N(SO₂-R¹¹)(SO₂-R¹²), -A-CO-O-R⁶, -A-CO-N(R⁷,R⁸), -A-CO-R²⁰, -A-CH(XR²¹, YR²²), -A-CO-N(R⁷)-C(R⁸, R¹⁸)-COOR⁶, -SO₂-N(R⁷)-C(R⁸,R¹⁸)-COOR⁶, -SO₂-N(R⁷)-C(R⁸,R¹⁸)-CO-N(R³²,R³³), 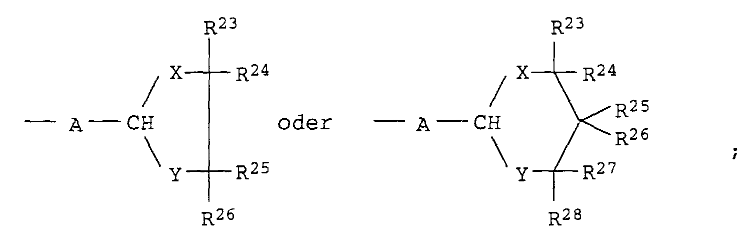
R² Cyano, Trifluormethyl oder Halogen;
R³ C₁-C₄-Alkyl oder C₁-C₄-Halogenalkyl;
R⁴ C₁-C₄-Alkyl oder C₁-C₄-Halogenalkyl;
R⁵ Halogen;
X,Y unabhängig voneinander Sauerstoff oder Schwefel;
A eine chemische Bindung, Methylen, Ethylen oder Vinylen;
R⁶ Wasserstoff, C₁-C₈-Alkyl, C₁-C₈-Halogenalkyl, C₃-C₇-Cycloalkyl, das seinerseits ein bis drei C₁-C₃-Alkylreste tragen kann, C₃-C₆-Alkenyl, C₅-C₇-Cycloalkenyl, das seinerseits ein bis drei C₁-C₃-Alkylreste tragen kann, C₃-C₆-Halogenalkenyl, Cyano-C₁-C₈-alkyl, C₃-C₆-Alkinyl, C₁-C₄-Alkoxy-C₁-C₄-alkyl, 2-Tetrahydrofuranyl-C₁-C₈-alkyl, 3-Oxetanyl, 3-Thiethanyl, Carboxyl-C₁-C₆-alkyl, (C₁-C₈-Alkoxy)carbonyl-C₁-C₆-alkyl, C₁-C₄-Alkoxy-(C₁-C₄-alkoxy)carbonyl-C₁-C₆-alkyl, Cyclopropylmethyl, (1-Methylthiocycloprop-1-yl)methyl, C₃-C₉-(α-Alkylalkyliden)iminooxy-C₁-C₆-alkyl, (C₁-C₄-Alkyl)carbonyl, C₁-C₄-Alkyl, das durch -C(R¹⁹)=N-O-(C₁-C₄-Alkyl), -C(R¹⁹)=N-O-(C₁-C₄-Halogenalkyl), -C(R¹⁹)=N-O-(C₃-C₆-Alkenyl), -C(R¹⁹)=N-O-(C₃-C₆-Halogenalkenyl) oder -C(R¹⁹)=N-O-(C₁-C₄-Alkyl)-R³⁴ substituiert ist, Phenyl, Phenyl-C₁-C₆-alkyl, Phenyl-C₂-C₆-alkenyl, Phenyl-C₃-C₆-alkinyl oder Phenoxy-C₁-C₆-alkyl, wobei der Phenylring jeweils unsubstituiert sein oder seinerseits ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl, Heteroaryl-C₁-C₆-alkyl, Heteroaryl-C₃-C₆-alkenyl, Heteroaryl-C₃-C₆-alkinyl oder Heteroaryloxy-C₁-C₆-alkyl, wobei der Heteroaromat jeweils ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ringglied einen Rest tragen kann, jeweils ausgewählt aus der Gruppe bestehend aus Hydroxyl, Halogen, C₁-C₄-Halogenalkyl, C₁-C₄-Alkoxy, C₁-C₄-Alkylthio und C₁-C₄-Alkyl;
R⁷, R⁸, R³², R³³ unabhängig voneinander
Wasserstoff, C₁-C₈-Alkyl, C₁-C₈-Halogenalkyl, C₂-C₈-Alkenyl, C₂-C₈-Alkinyl, C₁-C₄-Alkoxy-C₁-C₄-alkyl, C₁-C₄-Alkylthio-C₁-C₄-alkyl, Cyano-C₁-C₈-alkyl, Carboxyl-C₁-C₄-alkyl, (C₁-C₄-Alkoxy)carbonyl-C₁-C₄-alkyl, C₁-C₄-Alkylsulfonyl-C₁-C₄-alkyl, C₃-C₈-Cycloalkyl, C₁-C₆-Alkoxy, (C₃-C₆-Cycloalkoxy)carbonyl-C₁-C₄-alkyl, C₁-C₄-Alkoxy-(C₁-C₄-alkoxy)carbonyl-C₁-C₄-alkyl, (C₁-C₄-Alkyl)carbonyl, (C₁-C₄-Halogenalkyl)carbonyl, Tetrahydrofuran-2-on-3-yl, Phenyl, Phenyl-C₁-C₄-alkyl, wobei der Phenylring jeweils unsubstituiert sein oder ein bis drei Reste tragen kann, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl und C₂-C₆-Alkenyl, 5- oder 6-gliedriges Heteroaryl oder Heteroaryl-C₁-C₄-alkyl, wobei der Heteroaromat ein bis drei Heteroatome enthält, ausgewählt aus einer Gruppe bestehend aus ein oder zwei Stickstoffatomen und einem Sauerstoff- oder Schwefelatom, und wobei der Heteroaromat gewünschtenfalls noch an jedem substituierbaren Ring-Atom einen Rest tragen kann, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
oder
R⁷ und R⁸ zusammen eine Tetramethylen-, Pentamethylen- oder Ethylenoxyethylenkette, die gewünschtenfalls ein bis drei C₁-C₄-Alkylreste und/oder einen Rest -COOR⁶ tragen kann;
R⁹ Wasserstoff, C₁-C₆-Alkyl, C₁-C₆-Halogenalkyl, C₁-C₄-Alkoxy-C₁-C₄-alkyl, C₃-C₇-Cycloalkyl, das seinerseits ein bis drei Reste tragen kann, ausgewählt aus der Gruppe bestehend aus Halogen, C₁-C₄-Alkyl, C₁-C₄-Alkoxy und C₁-C₄-Alkylthio, Phenyl oder Phenyl-C₁-C₆-alkyl, wobei der Phenylring jeweils unsubstituiert sein oder ein bis drei Reste tragen kann, ausgewählt aus der Gruppe bestehend aus Halogen, Nitro, C₁-C₄-Alkyl, C₁-C₄-Alkoxy, C₁-C₄-Alkylthio und C₁-C₄-Halogenalkyl;
R¹⁰ Wasserstoff, C₁-C₄-Alkyl, C₃-C₆-Alkenyl, C₃-C₆-Alkinyl, C₁-C₄-Alkoxy-C₁-C₄-alkyl oder das Äquivalent eines landwirtschaftlich brauchbaren Kations;
R¹¹, R¹² unabhängig voneinander
C₁-C₄-Alkyl, C₁-C₄-Halogenalkyl, Phenyl, das unsubstituiert sein oder ein bis drei Substituenten tragen kann, jeweils ausgewählt aus der Gruppe bestehend aus Halogen, Nitro, C₁-C₄-Alkyl, C₁-C₄-Alkoxy, C₁-C₄-Alkylthio und C₁-C₄-Halogenalkyl,
oder 5- oder 6-gliedriges Heteroaryl mit ein bis drei Heteroatomen, ausgewählt aus der Gruppe bestehend aus 2 Stickstoffatomen und einem Sauerstoff- oder Schwefelatom, wobei der Heteroaromat unsubstituiert sein oder an jedem substituierbaren Ringglied gewünschtenfalls einen Substituenten tragen kann, jeweils ausgewählt aus der Gruppe bestehend aus Hydroxyl, Halogen, C₁-C₄-Alkyl, C₁-C₄-Halogenalkyl, C₁-C₄-Alkoxy und C₁-C₄-Alkylthio;
R¹⁸ Wasserstoff oder C₁-C₄-Alkyl;
R¹⁹ Wasserstoff, C₁-C₄-Alkyl, Phenyl oder Benzyl;
R²⁰ Wasserstoff, Cyano, Halogen, C₁-C₄-Alkyl, C₁-C₄-Alkylthio, C₂-C₄-Alkenyl, C₁-C₄-Halogenalkyl, C₁-C₄-Alkoxy-C₁-C₄-alkyl, Di-(C₁-C₄-alkoxy)-C₁-C₄-alkyl, C₁-C₄-Alkylthio-C₁-C₄-alkyl, (1,3-Dioxolan-2-yl)-C₁-C₄-alkyl oder (1,3-Dioxan-2-yl)-C₁-C₄-alkyl;
R²¹, R²² unabhängig voneinander C₁-C₈-Alkyl, C₁-C₈-Halogenalkyl oder C₁-C₄-Alkoxy-C₁-C₄-alkyl;
R²³, R²⁴, R²⁵, R²⁶, R²⁷, R²⁸ unabhängig voneinander Wasserstoff, Cyano, C₁-C₈-Alkyl, C₁-C₈-Halogenalkyl, C₁-C₄-Alkoxy-C₁-C₄-alkyl, C₁-C₈-Alkoxy, C₁-C₄-Alkoxy-C₁-C₄-alkoxy, -CO-O-R⁶, -CO-N(R⁷, R⁸), -CO-R²⁰, -S-R⁶, -SO₂-R⁶, -O-CO-R⁹ oder C₃-C₇-Cycloalkyl, das seinerseits ein bis drei Reste tragen kann, ausgewählt aus der Gruppe bestehend aus Halogen, C₁-C₄-Alkyl, C₁-C₄-Alkoxy und C₁-C₄-Alkylthio;
R³⁴ Phenyl oder 5- oder 6-gliedriges Heteroaryl mit ein bis drei Heteroatomen, ausgewählt aus der Gruppe bestehend aus 2 Stickstoffatomen und einem Sauerstoff- oder Schwefelatom, wobei jeder Phenyl- oder Heteroarylring unsubstituiert sein oder an jedem substituierbaren Ringglied gewünschtenfalls einen Substituenten tragen kann, jeweils ausgewählt aus der Gruppe bestehend aus Hydroxy, Nitro, Cyano, Halogen, C₁-C₄-Alkyl, C₁-C₄-Halogenalkyl, C₁-C₄-Alkoxy und C₁-C₄-Alkylthio,
sowie die landwirtschaftlich brauchbaren Salze von 1.

2. Verwendung der substituierten 3-Phenylpyrazole I gemäß Anspruch 1 als Herbizide.

3. Herbizides Mittel, enthaltend flüssige und/oder feste Trägerstoffe und gewünschtenfalls mindestens ein Adjuvans sowie eine herbizid wirksame Menge mindestens eines substituierten 3-Phenylpyrazols der Formel I oder ein Salz von I, gemäß Anspruch 1.

4. Verfahren zur Herstellung von herbizid wirksamen Mitteln, **dadurch gekennzeichnet, daß** man eine herbizid wirksame Menge mindestens eines substituierten 3-Phenylpyrazols der Formel I oder ein Salz von I, gemäß Anspruch 1, mit flüssigen und/oder festen Trägerstoffen und gewünschtenfalls mindestens einem Adjuvans mischt.

5. Verfahren zur Bekämpfung von unerwünschtem Pflanzenwuchs, **dadurch gekennzeichnet, daß** man eine herbizid wirksame Menge mindestens eines substituierten 3-Phenylpyrazols der Formel I oder ein Salz von I, gemäß Anspruch 1, auf Pflanzen, deren Lebensraum oder auf Saatgut einwirken läßt.

## Claims

1. A substituted 3-phenylpyrazole of the formula I 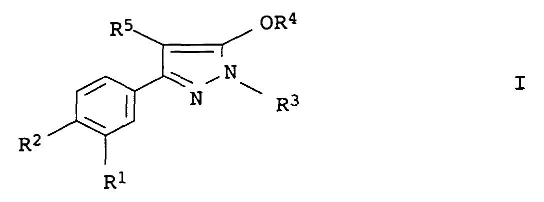 in which
R¹ is hydrogen, nitro, C₁-C₈-alkyl, C₁-C₈-haloalkyl, C₁-C₈-alkyl-O-R⁶, C₁-C₈-alkyl-O-CO-R⁶, C₁-C₈-alkyl-S-R⁶, C₁-C₈-alkyl-SO-R⁶, C₁-C₈-alkyl-SO₂-R⁶, -SO₂-R⁶, -SO₂-O-R⁶, -SO₂-N(R⁷,R⁸), -N(R⁷,R⁸), -N(R⁷)-N(R⁸,R³²), -N=N-CO-R⁹, -N(R⁷)-N(R⁸)-CO-R⁹, -N(R¹⁰)-CO-R⁹, -N(R¹⁰)-SO₂-R¹¹, -N(SO₂-R¹¹)(SO₂-R¹²), -A-CO-O-R⁶, -A-CO-N(R⁷,R⁸), -A-CO-R²⁰, -A-CH(XR²¹, YR²²), -A-CO-N(R⁷)-C(R⁸,R¹⁸)-COOR⁶, -SO₂-N(R⁷)-C(R⁸,R¹⁸)-COOR⁶, -SO₂-N(R⁷)-C(R⁸,R¹⁸)-CO-N(R³²,R³³), 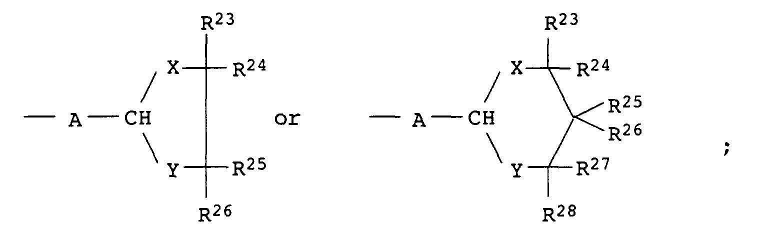
R² is cyano, trifluoromethyl or halogen;
R³ is C₁-C₄-alkyl or C₁-C₄-haloalkyl;
R⁴ is C₁-C₄-alkyl or C₁-C₄-haloalkyl;
R⁵ is halogen;
X and Y independently of one another are oxygen or sulfur;
A is a chemical bond, methylene, ethylene or vinylene;
R⁶ is hydrogen, C₁-C₈-alkyl, C₁-C₈-haloalkyl, C₃-C₇-cycloalkyl which may in turn carry from one to three C₁-C₃-alkyl radicals, C₃-C₆-alkenyl, C₅-C₇-cycloalkenyl which may in turn carry from one to three C₁-C₃-alkyl radicals, C₃-C₆-haloalkenyl, cyano-C₁-C₈-alkyl, C₃-C₆-alkynyl, C₁-C₄-alkoxy-C₁-C₄-alkyl, 2-tetrahydrofuryl-C₁-C₈-alkyl, 3-oxetanyl, 3-thietanyl, carboxyl-C₁-C₆-alkyl, (C₁-C₈-alkoxy)carbonyl-C₁-C₆-alkyl, C₁-C₄-alkoxy-(C₁-C₄-alkoxy)carbonyl-C₁-C₆-alkyl, cyclopropylmethyl, (1-methylthiocycloprop-1-yl)methyl, C₃-C₉-(α-alkylalkylidene)iminooxy-C₁-C₆-alkyl, (C₁-C₄-alkyl)carbonyl, C₁-C₄-alkyl which is substituted by -C(R¹⁹)=N-O-(C₁-C₄-alkyl), -C(R¹⁹)=N-O-(C₁-C₄-haloalkyl), -C(R¹⁹)=N-O-(C₃-C₆-alkenyl), -C(R¹⁹)=N-O-(C₃-C₆-haloalkenyl) or -C(R¹⁹)=N-O-(C₁-C₄-alkyl)-R³⁴, phenyl, phenyl-C₁-C₆-alkyl, phenyl-C₂-C₆-alkenyl, phenyl-C₃-C₆-alkynyl or phenoxy-C₁-C₆-alkyl, the phenyl ring being able in each case to be unsubstituted or in turn to carry from one to three radicals selected from the group consisting of halogen, nitro, cyano, C₁-C₄-alkyl, C₁-C₄-alkoxy, C₁-C₄-alkylthio, C₁-C₄-haloalkyl and C₂-C₆-alkenyl, 5- or 6-membered heteroaryl, heteroaryl-C₁-C₆-alkyl, heteroaryl-C₃-C₆-alkenyl, heteroaryl-C₃-C₆-alkynyl or heteroaryloxy-C₁-C₆-alkyl, the heteroaromatic radical containing in each case from one to three heteroatoms selected from a group consisting of one or two nitrogen atoms and one oxygen or sulfur atom, and the heteroaromatic radical being able, if desired, to carry on each substitutable ring member a radical selected in each case from the group consisting of hydroxyl, halogen, C₁-C₄-haloalkyl, C₁-C₄-alkoxy, C₁-C₄-alkylthio and C₁-C₄-alkyl;
R⁷, R⁸, R³², R³³ independently of one another are hydrogen, C₁-C₈-alkyl, C₁-C₈-haloalkyl, C₂-C₈-alkenyl, C₂-C₈-alkynyl, C₁-C₄-alkoxy-C₁-C₄-alkyl, C₁-C₄-alkylthio-C₁-C₄-alkyl, cyano-C₁-C₈-alkyl, carboxyl-C₁-C₄-alkyl, (C₁-C₄-alkoxy)carbonyl-C₁-C₄-alkyl, C₁-C₄-alkylsulfonyl-C₁-C₄-alkyl, C₃-C₈-cycloalkyl, C₁-C₆-alkoxy, (C₃-C₆-cycloalkoxy)carbonyl-C₁-C₄-alkyl, C₁-C₄-alkoxy-(C₁-C₄-alkoxy)carbonyl-C₁-C₄-alkyl, (C₁-C₄-alkyl)carbonyl, (C₁-C₄-haloalkyl)- carbonyl, tetrahydrofuran-2-on-3-yl, phenyl, phenyl-C₁-C₄- alkyl, in which the phenyl ring may in each case be unsubstituted or may carry one to three radicals selected from the group consisting of halogen, nitro, cyano, C₁-C₄-alkyl, C₁-C₄-alkoxy, C₁-C₄-alkylthio, C₁-C₄-haloalkyl and C₂-C₆-alkenyl, 5- or 6-membered heteroaryl or heteroaryl-C₁-C₄-alkyl, the heteroaromatic radical containing from one to three heteroatoms selected from a group consisting of one or two nitrogen atoms and one oxygen or sulfur atom, and the heteroaromatic radical being able, if desired, to carry on each substitutable ring atom a radical selected from the group consisting of hydroxyl, halogen, C₁-C₄-alkyl, C₁-C₄-alkoxy, C₁-C₄-alkylthio and C₁-C₄-haloalkyl;
or
R⁷ and R⁸ are together a tetramethylene, pentamethylene or ethyleneoxyethylene chain which may if desired carry from one to three C₁-C₄-alkyl radicals and/or a radical -COOR⁶;
R⁹ is hydrogen, C₁-C₆-alkyl, C₁-C₆-haloalkyl, C₁-C₄-alkoxy-C₁-C₄-alkyl, C₃-C₇-cycloalkyl which may in turn carry from one to three radicals selected from the group consisting of halogen, C₁-C₄-alkyl, C₁-C₄-alkoxy and C₁-C₄-alkylthio, phenyl or phenyl-C₁-C₆-alkyl, in which the phenyl ring may in each case be unsubstituted or may carry from one to three radicals selected from the group consisting of halogen, nitro, C₁-C₄-alkyl, C₁-C₄-alkoxy, C₁-C₄-alkylthio and C₁-C₄-haloalkyl;
R¹⁰ is hydrogen, C₁-C₄-alkyl, C₃-C₆-alkenyl, C₃-C₆-alkynyl, C₁-C₄-alkoxy-C₁-C₄-alkyl or the equivalent of an agriculturally usable cation;
R¹¹ and R¹² independently of one another
are C₁-C₄-alkyl, C₁-C₄-haloalkyl, phenyl which may be unsubstituted or may carry from one to three substituents in each case selected from the group consisting of halogen, nitro, C₁-C₄-alkyl, C₁-C₄-alkoxy, C₁-C₄-alkylthio and C₁-C₄-haloalkyl,
or 5- or 6-membered heteroaryl containing from one to three heteroatoms selected from the group consisting of 2 nitrogen atoms and one oxygen or sulfur atom, the heteroaromatic radical being able to be unsubstituted or to carry, if desired, on each substitutable ring member a substituent in each case selected from the group consisting of hydroxyl, halogen, C₁-C₄-alkyl, C₁-C₄-haloalkyl, C₁-C₄-alkoxy and C₁-C₄-alkylthio;
R¹⁸ is hydrogen or C₁-C₄-alkyl;
R¹⁹ is hydrogen, C₁-C₄-alkyl, phenyl or benzyl;
R²⁰ is hydrogen, cyano, halogen, C₁-C₄-alkyl, C₁-C₄-alkylthio, C₂-C₄-alkenyl, C₁-C₄-haloalkyl, C₁-C₄-alkoxy-C₁-C₄-alkyl, di-(C₁-C₄-alkoxy)-C₁-C₄-alkyl, C₁-C₄-alkylthio-C₁-C₄-alkyl, (1,3-dioxolan-2-yl)-C₁-C₄-alkyl or (1,3-dioxan-2-yl)-C₁-C₄-alkyl;
R²¹ and R²² independently of one another are C₁-C₈-alkyl, C₁-C₈-haloalkyl or C₁-C₄-alkoxy-C₁-C₄-alkyl;
R²³, R²⁴, R²⁵, R²⁶, R²⁷ and R²⁸ independently of one another are hydrogen, cyano, C₁-C₈-alkyl, C₁-C₈-haloalkyl, C₁-C₄-alkoxy-C₁-C₄-alkyl, C₁-C₈-alkoxy, C₁-C₄-alkoxy-C₁-C₄-alkoxy, -CO-O-R⁶, -CO-N(R⁷, R⁸), -CO-R²⁰, -S-R⁶, -SO₂-R⁶, -O-CO-R⁹ or C₃-C₇-cycloalkyl which may in turn carry from one to three radicals selected from the group consisting of halogen, C₁-C₄-alkyl, C₁-C₄-alkoxy and C₁-C₄-alkylthio;
R³⁴ is phenyl or 5- or 6-membered heteroaryl containing from one to three heteroatoms selected from the group consisting of 2 nitrogen atoms and one oxygen or sulfur atom, each phenyl or heteroaryl ring being able to be unsubstituted or, if desired, to carry on each substitutable ring member a substituent in each case selected from the group consisting of hydroxyl, nitro, cyano, halogen, C₁-C₄-alkyl, C₁-C₄-haloalkyl, C₁-C₄-alkoxy and C₁-C₄-alkylthio,
or an agriculturally usable salt of I.

2. The use of a substituted 3-phenylpyrazole I as claimed in claim 1 as a herbicide.

3. A herbicidal composition comprising liquid and/or solid carriers and if desired at least one adjuvant and a herbicidally active quantity of at least one substituted 3-phenylpyrazole of the formula I or a salt of I, as claimed in claim 1.

4. A method of producing herbicidally active compositions, which comprises mixing a herbicidally active quantity of at least one substituted 3-phenylpyrazole of the formula I or a salt of I, as claimed in claim 1, with liquid and/or solid carriers and if desired at least one adjuvant.

5. A method of controlling unwanted plant growth, which comprises allowing a herbicidally active quantity of at least one substituted 3-phenylpyrazole of the formula I or a salt of I, as claimed in claim 1, to act on plants, their habitat or on seeds.

## Revendications

1. 3-phénylpyrazoles substitués de formule I 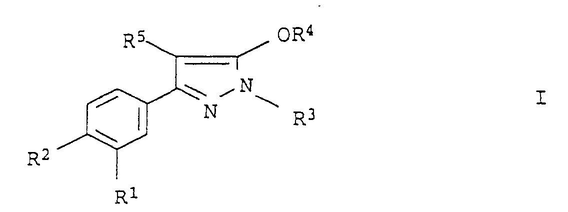 dans laquelle les variables ont la signification suivante:
R¹ hydrogène, nitro, alkyle en C₁-C₈, halogénoalkyle en C₁-C₈, alkyl(C₁-C₈)-O-R⁶, alkyl(C₁-C₈)-O-CO-R⁶, alkyl(C₁-C₈)-S-R⁶, alkyl(C₁-C₈)-SO-R⁶, alkyl(C₁-C₈)-SO₂-R⁶, -SO₂-R⁶, -SO₂-O-R⁶, -SO₂-N(R⁷,R⁸), -N(R⁷,R⁸), -N(R⁷)-N(R⁸,R³²), -N=N-CO-R⁹, -N(R⁷)-N(R⁸-CO-R⁹, -N(R¹⁰)-CO-R⁹, -N(R¹⁰)-SO₂-R¹¹, -N(SO₂-R¹¹)(SO₂-R¹²), -A-CO-O-R⁶, -A-CO-N(R⁷,R⁸), -A-CO-R²⁰, -A-CH(XR²¹,YR²²), -A-CO-N(R⁷)-C(R⁸,R¹⁸)-COOR⁶, -SO₂-N(R⁷)-C(R⁸,R¹⁸)-COOR⁶, -SO₂-N(N⁷)-C(R⁸,R¹⁸)-CO-N(R³²,R³³), 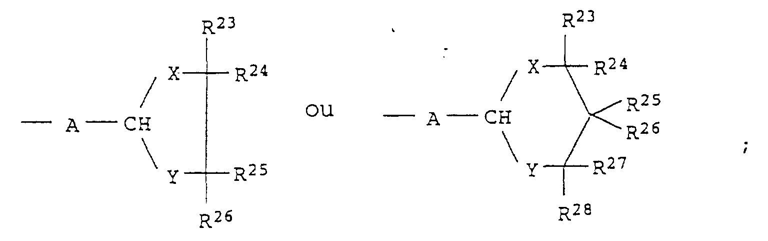
R² cyano, trifluorométhyle ou halogéno;
R³ alkyle en C₁-C₄ ou halogénoalkyle en C₁-C₄;
R⁴ alkyle en C₁-C₄ ou halogénoalkyle en C₁-C₄;
R⁵ halogéno;
X,Y indépendamment l'un de l'autre oxygène ou soufre;
A une liaison chimique, méthylène, éthylène ou vinylène;
R⁶ hydrogène, alkyle en C₁-C₈, halogénoalkyle en C₁-C₈, cycloalkyle en C₃-C₇ - qui à son tour peut porter un à trois restes alkyle en C₁-C₃ -, alcényle en C₃-C₆, cycloalcényle en C₅-C₇ - qui à son tour peut porter un à trois restes alkyle en C₁-C₃ -, halogénoalcényle en C₃-C₆, cyano-alkyle(C₁-C₈), alcynyle en C₃-C₆, alcoxy(C₁-C₄)-alkyle(C₁-C₄), 2-tétrahydrofurannyl-alkyle(C₁-C₈), 3-oxétannyle, 3-thiétannyle, carboxyl-alkyle(C₁-C₆), (alcoxy en C₁-C₈)carbonyl-alkyle(C₁-C₆), alcoxy(C₁-C₄)-(alcoxy en C₁-C₄)carbonyl-alkyle(C₁-C₆), cyclopropylméthyle, (1-méthylthiocycloprop-1-yl)méthyle, (α-alkylalkylidène en C₃-C₉)iminooxy-alkyle(C₁-C₆), (alkyl en C₁-C₄)-carbonyle, alkyle en C₁-C₄ - qui est substitué par un groupe -C(R¹⁹)=N-O-(alkyle en C₁-C₄), -C(R¹⁹)=N-O-(halogénoalkyle en C₁-C₄), -C(R¹⁹)=N-O-(alcényle en C₃-C₆), -C(R¹⁹)=N-O-(halogénoalcényle en C₃-C₆) ou -C(R¹⁹)=N-O-(alkyle en C₁-C₄)-R³⁴ -, phényle, phénylalkyle(C₁-C₆), phényl-alcényle(C₂-C₆), phénylalcynyle(C₃-C₆) ou phénoxy-alkyle(C₁-C₆), tandis que le cycle phényle dans chaque cas est non-substitué ou peut porter à son tour un à trois restes, choisis dans le groupe consistant en halogéno, nitro, cyano, alkyle en C₁-C₄, alcoxy en C₁-C₄, alkylthio en C₁-C₄, halogénoalkyle en C₁-C₄ et alcényle en C₂-C₆, hétéroaryle à 5 ou 6 chaînons, hétéroaryl-alkyle(C₁-C₆), hétéroaryl-alcényle(C₃-C₆), hétéroaryl-alcynyle-(C₃-C₆) ou hétéroaryloxy-alkyle(C₁-C₆), tandis que le groupe hétéroaromatique contient dans chaque cas un à trois hétéroatomes, choisis dans le groupe consistant en un ou deux atomes d'azote et un atome d'oxygène ou de soufre, et tandis que le groupe hétéroaromatique peut encore porter en option sur chaque membre cyclique substituable un reste, à chaque fois choisi dans le groupe consistant en hydroxyle, halogéno, halogénoalkyle en C₁-C₄, alcoxy en C₁-C₄, alkylthio en C₁-C₄ et alkyle en C₁-C₄;
R⁷, R⁸, R³², R³³, indépendamment l'un de l'autre, hydrogène, alkyle en C₁-C₈, halogénoalkyle en C₁-C₈, alcényle en C₂-C₈, alcynyle en C₂-C₈, alcoxy(C₁-C₄)-alkyle((C₁-C₄), alkyl(C₁-C₄) thio-alkyle(C₁-C₄), cyano-alkyle((C₁-C₈), carboxyl-alkyle(C₁-C₄), (alcoxy en C₁-C₄)carbonyl-alkyle(C₁-C₄), alkyl(C₁-C₄)-sulfonyl-alkyle(C₁-C₄), cycloalkyle en C₃-C₈, alcoxy en C₁-C₆, (cycloalcoxy en C₃-C₆)carbonyl-alkyle(C₁-C₄), alcoxy(C₁-C₄)-(alcoxy en C₁-C₄)carbonyl-alkyle en C₁-C₄, (alkyl en C₁-C₄)carbonyle, (halogénoalkyl en C₁-C₄)carbonyle, tétrahydrofuranne-2-one-3-yle, phényle, phényl-alkyle(C₁-C₄), tandis que le cycle phényle à chaque fois est non substitué ou peut porter un à trois restes, choisis dans le groupe constitué par halogéno, nitro, cyano, alkyle en C₁-C₄, alcoxy en C₁-C₄, alkyl(C₁-C₄)thio, halogénoalkyle en C₁-C₄ et alcényle en C₂-C₆, hétéroaryle à 5 ou 6 chaînons ou hétéroaryl-aryle(C₁-C₄), tandis que le groupe hétéroaromatique contient un à trois hétéroatomes, choisis dans un groupe consistant en un ou deux atomes d'azote et un atome d'oxygène ou de soufre et tandis que le groupe hétéroaromatique peut encore porter en option sur chaque atome de cycle substituable un este choisi dans le groupe consistant hydroxyle, halogéno, alkyle en C₁-C₄, alcoxy en C₁-C₄, alkyl(C₁-C₄)thio et halogénoalkyle en C₁-C₄;
ou
R⁷ et R⁸ ensemble peut former une chaîne tétraméthylène, pentaméthylène ou éthylène-oxyéthylène, qui peut en option porter un à trois restes alkyle en C₁-C₄ et/ou un reste -COOR⁶;
R⁹ hydrogène, alkyle en C₁-C₆, halogénoalkyle en C₁-C₆, alcoxy(C₁-C₄)-alkyle(C₁-C₄), cycloalkyle en C₃-C₇, qui à son tour peut porter un à trois restes choisis dans le groupe consistant en halogéno, alkyle en C₁-C₄, alcoxy en C₁-C₄ et alkyl(C₁-C₄)thio, phényle ou phényl-alkyle(C₁-C₆), tandis que le cycle phényle à chaque fois est non-substitué ou peut porter un à trois restes choisis dans le groupe des groupes halogéno, nitro, alkyle en C₁-C₄, alcoxy en C₁-C₄, alkyl(C₁-C₄)thio et halogénoalkyle en C₁-C₄;
R¹⁰ hydrogène, alkyle en C₁-C₄, alcényle en C₃-C₆, alcynyle en C₃-C₆, alcoxy(C₁-C₄)-alkyle(C₁-C₄) ou l'équivalent d'un cation utilisable en agriculture;
R¹¹, R¹² indépendamment l'un de l'autre alkyle en C₁-C₄, halogénoalkyle en C₁-C₄, phényle, qui peut être non-substitué ou peut porter un à trois substituants, à chaque fois choisis dans le groupe consistant en halogéno, nitro, alkyle en C₁-C₄, alcoxy en C₁-C₄, alkyl(C₁-C₄)thio et halogénoalkyle en C₁-C₄,
ou hétéroaryle à 5 ou 6 chaînons ayant un à trois hétéroatomes, choisis dans le groupe consistant en 2 atomes d'azote et un atome d'oxygène ou de soufre, tandis que le groupe hétéroaromatique peut être non-substitué ou peut porter en option sur chaque membre de cycle substituable un substituant choisi à chaque fois dans le groupe consistant en hydroxyle, halogéno, alkyle en C₁-C₄, halogénoalkyle en C₁-C₄, alcoxy en C₁-C₄ et alkyl(C₁-C₄)thio;
R¹⁸ hydrogène ou alkyle en C₁-C₄;
R¹⁹ hydrogène, alkyle en C₁-C₄, phényle ou benzyle;
R²⁰ hydrogène, cyano, halogéno, alkyle en C₁-C₄, alkyl(C₁-C₄)thio, alcényle en C₂-C₄, halogénoalkyle en C₁-C₄, alcoxy(C₁-C₄)-alkyle(C₁-C₄), di-(alcoxy en C₁-C₄)-alkyle(C₁-C₄), alkyl(C₁-C₄)thio-alkyle(C₁-C₄), (1,3-dioxalanne-2-yl)-alkyle(C₁-C₄) ou (1,3-dioxolanne-2-yl)-alkyle(C₁-C₄) ;
R²¹,R²² indépendamment l'un de l'autre, alkyle en C₁-C₈, halogénoalkyle en C₁-C₈ ou alcoxy(C₁-C₄)-alkyle(C₁-C₄) ;
R²³, R²⁴, R²⁵, R²⁶, R²⁷, R²⁸ indépendamment l'un de l'autre, hydrogène, cyano, alkyle en C₁-C₈, halogénoalkyle en C₁-C₈, alcoxy(C₁-C₄)-alkyle(C₁-C₄), alcoxy en C₁-C₈, alcoxy(C₁-C₄)-alcoxy(C₁-C₄), -CO-O-R⁶, -CO-N(R⁷,R⁸), -CO-R²⁰, -S-R⁶, -SO₂-R⁶, -O-CO-R⁹ ou cycloalkyle en C₃-C₇, qui à son tour peut porter un à trois restes, choisis dans le groupe consistant en halogéno, alkyle en C₁-C₄, alcoxy en C₁-C₄ et alkyl (C₁-C₄) thio;
R³⁴ phényle ou hétéroaryle à 5 ou 6 chaînons avec un à trois hétéroatomes, choisis dans le groupe consistant en 2 atomes d'azote et un atome d'oxygène ou de soufre, tandis que chaque cycle phényle ou hétéroaryle peut être non-substitué ou peut porter en option sur chaque membre de cycle substituable un substituant choisi dans le groupe consistant en hdyroxy, nitro, cyano, halogéno, alkyle en C₁-C₄, halogénoalkyle en C₁-C₄, alcoxy en C₁-C₄ et alkyl(C₁-C₄)thio,
ainsi que les sels de I utilisables en agriculture.

2. Utilisation des 3-phénylpyrazoles substitués I selon la revendication 1 comme herbicides.

3. Produit herbicide, contenant des substances support liquides et/ou solides et en option au moins un adjuvant, ainsi qu'une quantité à efficacité herbicide d'au moins un 3-phénylpyrazole substitué de formule I ou un sel de I, selon la revendication 1.

4. Procédé pour la préparation de produits à activité herbicide, **caractérisé par** le fait qu'on mélange une quantité à activité herbicide d'au moins un 3-phénylpyrazole substitué de formule I ou un sel de I, selon la revendication 1, avec des substances support liquides et/ou solides et en option au moins un adjuvant.

5. Procédé pour la lutte contre la croissante des plantes parasites, **caractérisé par** le fait qu'on fait agir une quantité à efficacité herbicide d'au moins un 3-phénylpyrazole substitué de formule I ou un sel de I, selon la revendication 1, sur des plantes, leur biotope ou sur des semences.
